# EUROPÄISCHE PATENTSCHRIFT

(11) **EP 2 307 390 B1**
(45) Veröffentlichungstag und Bekanntmachung des Hinweises auf die Patenterteilung: **22.01.2014**
(21) Anmeldenummer: 09768923.6
(22) Anmeldetag: 16.06.2009
(51) Int. Cl.: C07D 285/08, A01N 43/82

(54) **THIADIAZOLYLOXYPHENYLAMIDINE UND DEREN VERWENDUNG ALS FUNGIZIDE**
THIADIAZOLYLOXYPHENYLAMIDINES AND USE THEREOF AS FUNGICIDES
THIADIAZOLYLOXYPHÉNYLAMIDINES ET LEUR UTILISATION COMME FONGICIDES

(30) Priorität: 27.06.2008 EP 08159267
(43) Veröffentlichungstag der Anmeldung: 13.04.2011
(73) Patentinhaber: Bayer CropScience AG, 40789 Monheim (DE)
(72) Erfinder: CRISTAU, Pierre, 69009 Lyon (FR); GREUL, Jörg, Nico, 42799 Leichlingen (DE); HEINEMANN, Ulrich, 42799 Leichlingen (DE); KUNZ, Klaus, 40625 Düsseldorf (DE); ORT, Oswald, 51375 Leverkusen (DE); SEITZ, Thomas, 40764 Langenfeld (DE); VOERSTE, Arnd, 50674 Köln (DE); DAHMEN, Peter, 41470 Neuss (DE); WACHENDORFF-NEUMANN, Ulrike, 56566 Neuwied (DE); TIETJEN, Klaus-Günther, 40764 Langenfeld (DE); HADANO, Hiroyuki, Tochigi 329-0434 (JP)
(86) Internationale Anmeldenummer: PCT/EP2009/004307
(87) Internationale Veröffentlichungsnummer: WO 2009/156074

(56) Entgegenhaltungen:
- WO-A-00/46184
- WO-A-2004/041777
- WO-A-2005/007636
- WO-A-2007/031513
- WO-A-2008/110278
- DE-A1- 2 242 185
- DE-C- 960 281
- US-A- 3 917 630
- US-A- 5 405 853
- GÖRDELER ET AL: "Über 1.2.4-Thiodiazole, VIII. Synthese von 5-Chlor-1.2.4-Thiodiazolen aus Perchlormethylmercaptan und Amidinen" CHEM. BER., Bd. 90, 1957, Seiten 182-187, XP002502319
- KOHARA ET AL: "A Facile Synthesis of 3-?Substituted 5-?Oxo-?1,2,4-?thiadiazoles from Amidoximes" J. HETEROCYCL. CHEM., Bd. 37, Nr. 6, 2000, Seiten 1419-1424, XP002502320
- WÖRFFEL ET AL: "5-Sulfanilamido- und 3-Sulfanilamido-1,2,4-thiodiazole" ARCHIV DER PHARMAZIE, Bd. 295, 1962, Seiten 811-816, XP002502321
- GÖRDELER ET AL: "Darstellung und Eigenschaften des 1.2.4- und des 1.3.4-Thiodiazols" CHEM. BER., Bd. 89, 1956, Seiten 1534-1543, XP002502322

## Beschreibung

Die vorliegende Erfindung betrifft Thiadiazolyloxyphenylamidine der allgemeinen Formel (I), ein Verfahren zu deren Herstellung, die Verwendung der erfindungsgemäßen Amidine zum Bekämpfen von unerwünschten Mikroorganismen, sowie ein Mittel zu diesem Zweck, umfassend die erfindungsgemäßen Thiadiazolyloxyphenylamidine. Weiterhin betrifft die Erfindung ein Verfahren zum Bekämpfen unerwünschter Mikroorganismen durch Ausbringen der erfindungsgemäßen Verbindungen auf die Mikroorganismen und/oder in deren Lebensraum.

WO-A-00/046 184 offenbart die Verwendung von Amidinen als Fungizide.

WO-A-03/093 224 offenbart die Verwendung von Arylamidin-Derivaten als Fungizide.

WO-A-03/024 219 offenbart Fungizidzusammensetzungen umfassend wenigstens ein N2-Phenylamidin-Derivat in Kombination mit einem weiteren ausgewählten bekannten Wirkstoff.

WO-A-04/037 239 offenbart fungizide Medikamente auf der Basis von N2-Phenylamidin-Derivaten.

WO-A-07/031 513 offenbart Thiadiazolyl-substituierte Phenylamidine sowie deren Herstellung und Verwendung als Fungizide.

WO-A-2008 110 278 offenbart N² -Phenylamidine sowie deren Verwendung als Herbizide, insbesondere Thiadiazolyloxyphenylamidine.

Die Wirksamkeit der im Stand der Technik beschriebenen Amidine ist gut, lässt jedoch in manchen Fallen zu wünschen übrig.

Der vorliegenden Erfindung liegt daher die Aufgabe zugrunde, Amidine mit einer verbesserten fungiziden Wirksamkeit zur Verfügung zu stellen.

Die Aufgabe wurde überraschenderweise gelöst durch Thiadiazolyloxyphenylamidine der Formel (I) 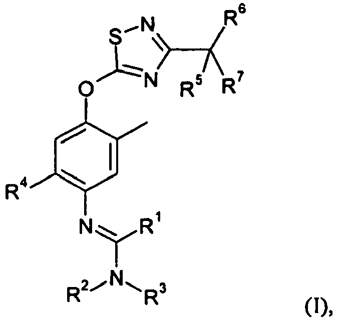 in welcher
- R¹: ausgewählt ist aus Wasserstoff; linearen, verzweigten C₁₋₁₂-Alkyl-, C₂₋₁₂-Alkenyl-, C₂₋₁₂-Alkinyl- oder cyclischen C₃₋₈-Alkyl-, C₄₋₈-Alkenyl-, C₄₋₈-Alkinyl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus-R', -X, -OR', -SR', -NR'₂, -SiR'₃, -COOR', -CN und -CONR₂' substituiert sein können, wobei R' Wasserstoff oder eine C₁₋₁₂-Alkyl-Gruppe ist; -SH; -SR", wobei R" eine C₁₋₁₂-Alkyl-Gruppe ist, die mit einer oder mehreren Gruppen, die ausgewählt sind aus -R', -X, -OR', -SR', -NR'₂, -SiR'₃, -COOR', -CN und-CONR₂', substituiert sein kann, worin R' die obige Bedeutung hat;
- R²: ausgewählt ist aus linearen, verzweigten C₁₋₁₂-Alkyl-, C₂₋₁₂-Alkenyl-, C₂₋₁₂-Alkinyl-, cyclischen C₃₋₈-Alkyl-, C₄₋₈-Alkenyl-, C₄₋₈-Alkinyl- oder C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus-R', -X, -OR', -SR', -NR'₂, -SiR'₃, -COOR', -CN und -CONR₂', substituiert sein können, wobei R' die obigen Bedeutungen hat;
- R³: ausgewählt ist aus -CN, -SH, -SR", -OR", -(C=O)-R", wobei R" die obigen Bedeutungen hat; linearen, verzweigten C₁₋₁₂-Alkyl-, C₂₋₁₂-Alkenyl-, C₂₋₁₂-Alkinyl-, cyclischen C₃₋₈-Alkyl-, C₄₋₈-Alkenyl-, C₄₋₈-Alkinyl- oder C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus - R', -X, -OR', -SR', -NR'₂, -SiR'₃, -COOR', -CN und -CONR₂', substituiert sein können, wobei R' die obigen Bedeutungen hat;
oder in der
R² und R³,
R² und R¹ oder
- R¹ und R³: gemeinsam mit den Atomen, an die sie gebunden sind oder mit weiteren Atomen, ausgewählt aus N, O, P und S, einen vier- bis siebengliedrigen Ring bilden können, der mit R'-, OR'-, SR'-, NR'₂-, SiR'₃-Gruppen substituiert sein kann, wobei R' die obigen Bedeutungen hat;
- R⁴: ausgewählt ist aus der Gruppe bestehend aus Halogen (X-) und -CN;
- R⁵, R⁶ und R⁷: unabhängig voneinander ausgewählt sind aus Wasserstoff, linearen, verzweigten C₁₋₁₂-Alkyl-, C₂₋₁₂-Alkenyl-, C₂₋₁₂-Alkinyl-, cyclischen C₃₋₈-Alkyl-, C₄₋₈-Alkenyl-, C₄₋₈-Alkinyl-Gruppen oder C₅₋₁₈-Aryl-, C₇₋₁₉-Aralkyl oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', Halogen- (-X), Alkoxy-(-OR'), Thioether- oder Mercapto- (-SR'), Amino- (-NR'₂), Silyl- (-SiR'₃), Carboxyl- (-COOR'), Cyan- (-CN) und Amid-Gruppen (-CONR₂'), substituiert sein können, wobei R' die obigen Bedeutungen hat;
oder
- R⁵ und R⁶, R⁶ und R⁷ oder R⁵ und R⁷: gemeinsam mit dem C-Atom, an das sie gebunden sind oder mit weiteren Atomen, ausgewählt aus N, O, P und S, einen drei- bis siebengliedrigen Ring bilden können, der mit X-, R'-, OR'-, SR'-, NR'₂-, SiR'₃- C₅₋₁₈-Aryl-, C₇₋₁₉-Ar-alkyl oder C₇₋₁₉-Alkaryl-Gruppen substituiert sein kann, wobei R' die obigen Bedeutungen hat und die Aryl-, Aralkyl oder Alkaryl-Guppen im Ring mit X-, R'-, OR'-, SR'-Gruppen substituiert sein können;
oder
- R⁵ und R⁶, R⁶ und R⁷ oder R⁵ und R⁷: gemeinsam mit dem C-Atom, an das sie gebunden sind, eine Doppelbindung bilden können, die mit -CN, -SH, -SR", -OR", -(C=O)-R", wobei R" die obigen Bedeutungen hat; linearen, verzweigten C₁₋₁₂-Alkyl-, C₂₋₁₂-Alkenyl-, C₂₁₂-Alkinyl-, cyclischen C₃₋₈-Alkyl-, C₄₋₈-Alkenyl-, C₄₋₈-Alkinyl- oder C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', -X, -OR', -SR', -NR'₂, -SiR'₃, -COOR', -CN und -CONR₂', substituiert sein können, wobei R' die obigen Bedeutungen hat;
und deren Salze, ausgenommen die Verbindungen, in welchen
R¹ gleich H, R² und R³ gleich Methyl, R⁴ gleich Cl und R⁵, R⁶, R⁷ gleich Methyl ist oder R¹ gleich H, R² gleich Methyl, R³ gleich Ethyl, R⁴ gleich Cl und R⁵, R⁶, R⁷ gleich Methyl ist oder R¹ gleich H, R² und R³ gleich (CH₂)₄, R⁴ gleich Cl und R⁵, R⁶, R⁷ gleich Methyl ist oder R¹ gleich H, R² und R³ gleich (CH₂)₅, R⁴ gleich Cl und R⁵, R⁶, R⁷ gleich Methyl ist.

### Allgemeine Definitionen

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen sind beispielsweise ausgewählt aus Trifluormethyl (CF₃), Difluormethyl (CHF₂), CF₃CH₂, ClCH₂, CF₃CCl₂.

Alkyl-Gruppen sind im Zusammenhang mit der vorliegenden Erfindung, soweit nicht abweichend definiert, lineare, verzweigte oder ringförmige Kohlenwasserstoff-Gruppen, die optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkyl-Gruppen optional durch weitere Gruppen substituiert sein, die ausgewählt sind aus -R', Halogen- (-X), Alkoxy- (-OR'), Thioether- oder Mercapto-(-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₁-C₁₂-Alkyl umfasst den größten hierin definierten Bereich für einen Alkyl-Gruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Alkenyl-Gruppen sind im Zusammenhang mit der vorliegenden Erfindung, soweit nicht abweichend definiert, lineare, verzweigte oder ringförmige Kohlenwasserstoff-Gruppen, die wenigstens eine einfache Unsättigung (Doppelbindung) enthalten und optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkenyl-Gruppen optional durch weitere Gruppen substituiert sein, die ausgewählt sind aus -R', Halogen- (-X), Alkoxy- (-OR'), Thioether- oder Mercapto- (-SR'), Amino-(-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₂-C₁₂-Alkenyl umfasst den größten hierin definierten Bereich für einen Alkenyl-Gruppe. Im Einzelnen umfasst diese Definition beispielsweise die Bedeutungen Vinyl; Allyl (2-Propenyl), Isopropenyl (1-Methylethenyl); But-1-enyl (Crotyl), But-2-enyl, But-3-enyl; Hex-1-enyl, Hex-2-enyl, Hex-3-enyl, Hex-4-enyl, Hex-5-enyl; Hept-1-enyl, Hept-2-enyl, Hept-3-enyl, Hept-4-enyl, Hept-5-enyl, Hept-6-enyl; Oct-1-enyl, Oct-2-enyl, Oct-3-enyl, Oct-4-enyl, Oct-5-enyl, Oct-6-enyl, Oct-7-enyl; Non-1-enyl, Non-2-enyl, Non-3-enyl, Non-4-enyl, Non-5-enyl, Non-6-enyl, Non-7-enyl, Non-8-enyl; Dec-1-enyl, Dec-2-enyl, Dec-3-enyl, Dec-4-enyl, Dec-5-enyl, Dec-6-enyl, Dec-7-enyl, Dec-8-enyl, Dec-9-enyl; Undec-1-enyl, Undec-2-enyl, Undec-3-enyl, Undec-4-enyl, Undec-5-enyl, Undec-6-enyl, Undec-7-enyl, Undec-8-enyl, Undec-9-enyl, Undec-10-enyl; Dodec-1-enyl, Dodec-2-enyl, Dodec-3-enyl, Dodec-4-enyl, Dodec-5-enyl, Dodec-6-enyl, Dodec-7-enyl, Dodec-8-enyl, Dodec-9-enyl, Dodec-10-enyl, Dodec-11-enyl.

Alkinyl-Gruppen sind im Zusammenhang mit der vorliegenden Erfindung, soweit nicht abweichend definiert, lineare, verzweigte oder ringförmige Kohlenwasserstoff-Gruppen, die wenigstens eine zweifache Unsättigung (Dreifachbindung) enthalten und optional eine, zwei oder mehrere einfache oder zweifache Unsättigungen oder ein, zwei oder mehrere Heteroatome, die ausgewählt sind aus O, N, P und S, aufweisen können. Außerdem können die erfindungsgemäßen Alkinyl-Gruppen optional durch weitere Gruppen substituiert sein, die ausgewählt sind aus -R', Halogen- (-X), Alkoxy- (-OR'), Thioether- oder Mercapto- (-SR'), Amino-(-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine lineare, verzweigte oder cyclische C₁₋₁₂-Alkyl-Gruppe ist, die ein oder mehrere Heteroatome, ausgewählt aus N, O, P und S, aufweisen kann.

Die Definition C₂-C₁₂-Alkinyl umfasst den größten hierin definierten Bereich für einen Alkinyl-Gruppe. Im Einzelnen umfasst diese Definition beispielsweise die Bedeutungen Ethinyl (Acetylenyl); Prop-1-inyl und Prop-2-inyl.

Die Definition C₃-C₈-Cycloalkyl umfasst monocyclische, gesättigte Kohlenwasserstoffgruppen mit 3 bis 8 Kohlenstoffringgliedern, wie Cyclopropyl, Cyclobutyl, Cyclopentyl, Cyclohexyl, Cycloheptyl und Cyclooctyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S aufweisen können und optional durch weitere Gruppen substituiert sein können, die ausgewählt sind aus -R', Halogen- (-X), Alkoxy-(-OR'), Thioether- oder Mercapto- (-SR'), Amino- (-NR'₂), Silyl- (-SiR'₃), Carboxyl-(-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₅₋₁₈-Aryl umfasst den größten hierin definierten Bereich für eine Aryl-Gruppe mit 5 bis 18 Gerüst-Atomen, wobei die C-Atome gegen Heteroatome ausgetauscht sein können. Im Einzelnen umfasst diese Definition beispielsweise die Bedeutungen Cyclopentadienyl, Phenyl, Cycloheptatrienyl, Cyclooctatetraenyl, Naphthyl und Anthracenyl; 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; 1-Pyrrolyl, 1-Pyrazolyl, 1,2,4-Triazol-1-yl, 1-Imidazolyl, 1,2,3-Triazol-1-yl, 1,3,4-Triazol-1-yl; 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Arylalkyl-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Aralkyl-Gruppe umfasst den größten hierin definierten Bereich für einen Arylalkyl-Gruppe mit insgesamt 7 bis 19 Atomen in Gerüst und Alkylenkette. Im Einzelnen umfasst diese Definition beispielsweise die Bedeutungen Benzyl- und Phenylethyl-.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Alkylaryl-Gruppe umfasst den größten hierin definierten Bereich für einen Alkylaryl-Gruppe mit insgesamt 7 bis 19 Atomen in Gerüst und Alkylenkette. Im Einzelnen umfasst diese Definition beispielsweise die Bedeutungen Tolyl-, 2,3-, 2,4-, 2,5-, 2,6-, 3,4- oder 3,5-Dimethylphenyl.

Die Alkyl-, Alkenyl-, Alkinyl-, Aryl,- Alkaryl- und Aralkylgruppen können zudem ein oder mehrere Heteroatome aufweisen, die - soweit nicht abweichend definiert - ausgewählt sind aus N, O, P und S. Die Heteroatome ersetzen dabei die bezifferten Kohlenstoffatome.

Die erfindungsgemäßen Verbindungen können gegebenenfalls als Mischungen verschiedener Stereoisomere, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

Die erfindungsgemäßen Amidine sind Verbindungen der Formel (I) 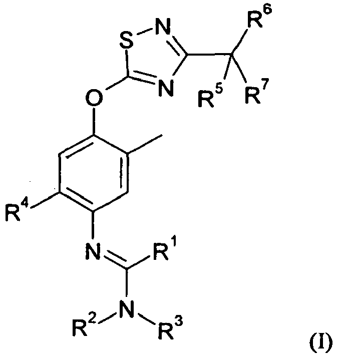 oder deren Salze, N-Oxide, Metallkomplexe und deren Stereoisomere.

In Formel (I) haben die Gruppen die im Folgenden definierten Bedeutungen. Die getroffenen Definitionen gelten für alle Zwischenprodukte gleichermaßen:
- R¹: ist ausgewählt aus Wasserstoff; linearen, verzweigten C₁₋₁₂-Alkyl-, C₂₋₁₂-Alkenyl-, C₂₋₁₂-Alkinyl- oder cyclischen C₃₋₈-Alkyl-, C₄₋₈-Alkenyl-, C₄₋₈-Alkinyl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus-R', -X, -OR', -SR', -NR'₂, -SiR'₃, -COOR', -CN und -CONR₂' substituiert sein können, wobei R' Wasserstoff oder eine C₁₋₁₂-Alkyl-Gruppe ist; -SH; -SR", wobei R" eine C₁₋₁₂-Alkyl-Gruppe ist, die mit einer oder mehreren Gruppen, die ausgewählt sind aus -R', -X, -OR', -SR', -NR'₂, -SiR'₃, -COOR', -CN und-CONR₂', substituiert sein kann, worin R' die obige Bedeutung hat;
- R²: ist ausgewählt aus linearen, verzweigten C₁₋₁₂-Alkyl-, C₂₋₁₂-Alkenyl-, C₂₋₁₂-Alkinyl, cyclischen C₃₋₈-Alkyl-, C₄₋₈-Alkenyl-, C₄₋₈-Alkinyl- oder C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus-R', -X, -OR', -SR', -NR'₂, -SiR'₃, -COOR', -CN und -CONR₂', substituiert sein können, wobei R' die obigen Bedeutungen hat;
- R³: ist ausgewählt aus -CN, -SH, -SR", -OR", -(C=O)-R", wobei R" die obigen Bedeutungen hat; linearen, verzweigten C₁₋₁₂-Alkyl-, C₂₋₁₂-Alkenyl-, C₂₋₁₂-Alkinyl-, cyclischen C₃₋₈-Alkyl-, C₄₋₈-Alkenyl-, C₄₋₈-Alkinyl- oder C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus-R', -X, -OR', -SR', -NR'₂, -SiR'₃, -COOR', -CN und -CONR₂', substituiert sein können, wobei R' die obigen Bedeutungen hat;
In einer alternativen erfindungsgemäßen Ausführungsform können R² und R³, R² und R¹ oder R¹ und R³ gemeinsam mit den Atomen, an die sie gebunden sind oder mit weiteren Atomen, ausgewählt aus N, O, P und S, einen vier- bis siebengliedrigen Ring bilden, der mit R'-, OR'-, SR', -NR'₂-, SiR'₃-Gruppen substituiert sein kann, wobei R' die obigen Bedeutungen hat;
- R⁴: ist ausgewählt aus der Gruppe bestehend aus Halogen (X-) und -CN;
- R⁵, R⁶ und R⁷: sind unabhängig voneinander ausgewählt aus Wasserstoff, linearen, verzweigten C₁₋₁₂-Alkyl-, C₂₋₁₂-Alkenyl-, C₂₋₁₂-Alkinyl-, cyclischen C₃₋₈-Alkyl-, C₄₋₈-Alkenyl-, C₄₋₈-Alkinyl-Gruppen oder C₅₋₁₈-Aryl-, C₇₋₁₉-Aralkyl oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', Halogen- (-X), Alkoxy-(-OR'), Thioether- oder Mercapto- (-SR'), Amino- (-NR'₂), Silyl- (-SiR'₃), Carboxyl- (-COOR'), Cyan- (-CN) und Amid-Gruppen (-CONR₂'), substituiert sein können, wobei R' die obigen Bedeutungen hat;

In einer alternativen Ausführungsform der Erfindung können R⁵ und R⁶, R⁶ und R⁷ oder R⁵ und R⁷ gemeinsam mit dem C-Atom, an das sie gebunden sind oder mit weiteren Atomen, ausgewählt aus N, O, P und S, einen drei- bis siebengliedrigen Ring bilden, der mit X-, R'-, OR'-, SR'-, NR'₂-, SiR'₃- C₅₋₁₈-Aryl-, C₇₋₁₉-Aralkyl oder C₇₋₁₉-Alkaryl-Gruppen substituiert sein kann, wobei R' die obigen Bedeutungen hat und die Aryl-, Aralkyl oder Alkaryl-Guppen wiederum im Ring mit X-, R'-, OR'-, SR'-Gruppen substituiert sein können;

In einer weiteren alternativen Ausführungsform der Erfindung können R⁵ und R⁶, R⁶ und R⁷ oder R⁵ und R⁷ gemeinsam mit dem C-Atom, an das sie gebunden sind, eine Doppelbindung bilden. Die Doppelbindung kann mit mit -CN, -SH, -SR", -OR", -(C=O)-R", wobei R" die obigen Bedeutungen hat; linearen, verzweigten C₁₋₁₂-Alkyl-, C₂₋₁₂-Alkenyl-, C₂₋₁₂-Alkinyl-, cyclischen C₃₋₈-Alkyl-, C₄₋₈-Alkenyl-, C₄₋₈-Alkinyl- oder C₅₋₁₈-Aryl-, C₇₋₁₉-Aralkyl- oder C₇₋₁₉-Alkaryl-Gruppen substituiert sein. Wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', -X, -OR', -SR', -NR'₂, -SiR'₃, -COOR', -CN und -CONR₂', substituiert sein können, wobei R' die obigen Bedeutungen hat;
und deren Salze.

Die jeweiligen Gruppen R⁵, R⁶ oder R⁷, die nicht an der Bildung des Rings oder der Doppelbindung beteiligt sind, entsprechen der zuvor getroffenen Definition.

In Formel (I) haben die Gruppen die im Folgenden definierten *bevorzugten* Bedeutungen. Die als *bevorzugt* getroffenen Definitionen gelten für alle Zwischenprodukte gleichermaßen:
- R¹: ist ausgewählt aus der Gruppe bestehend aus Wasserstoff, einer Mercapto-Gruppe (-SH) oder C₁₋₈-Alkyl-Gruppen;
- R²: ist ausgewählt aus linearen oder verzweigten C₁₋₈-Alkyl-Gruppen;
- R³: ist ausgewählt aus linearen, verzweigten und alicyclischen C₁₋₈-Alkyl-Gruppen;

In einer alternativen *bevorzugten* erfindungsgemäßen Ausführungsform können R² und R³ gemeinsam mit dem N-Atom, an das sie gebunden sind oder mit weiteren Atomen, die ausgewählt sind aus N und O, einen fünf- bis sechsgliedrigen Ring bilden, der mit einer oder mehreren C₁₋₁₂-Alkyl-Gruppen substituiert sein kann;
- R⁴: ist ausgewählt aus der Gruppe bestehend aus F, Cl, Br und I;
- R⁵ und R⁶: sind unabhängig voneinander ausgewählt aus Wasserstoff und linearen C₁₋₈-Alkyl-Gruppen;
- R⁷: ist ausgewählt aus der Gruppe bestehend aus Wasserstoff, linearen, verzweigten, alicyclischen oder heterocyclischen C₁₋₁₂-Alkyl-Gruppen, Halogenatomen, C₁₋₄-Haloalkyl-Gruppen, Phenyl- und Benzyl-Gruppen, wobei die Phenyl- oder Benzyl-Gruppen mit einem oder mehreren Atomen, ausgewählt aus -R', -X, -OR', -SR', -NR'₂, -SiR'₃, -COOR', -CN und-CONR₂' substituiert sein können;

In einer alternativen bevorzugten Ausführungsform der Erfindung bilden R⁵ und R⁶ gemeinsam mit dem C-Atom, an das sie gebunden sind oder mit weiteren Atomen, ausgewählt aus N, O, P und S, einen drei- vier- oder fünfgliedrigen Ring bilden können, der mit X-, R'- oder PhenylGruppen substituiert sein kann, wobei R' die obigen Bedeutungen hat. In dieser Ausführungsform ist der Rest R⁷ ausgewählt aus Wasserstoff und Halogenatomen, Phenyl-Phenoxy- und Benzyl-Gruppen, wobei die zuvor genannten Gruppen durch ein, zwei oder mehrere Halogenatome substitiert sein können.

In einer weiteren bevorzugten Ausführungsform bilden R⁵ und R⁶ gemeinsam mit dem C-Atom, an das sie gebunden sind, eine Doppelbindung. Die Doppelbindung kann mit Wasserstoff, linearen, verzweigten C₁₋₁₂-Alkyl-Gruppen oder cyclischen C₃₋₈-Alkyl-Gruppen, Halogenatomen, C₁₋₄-Haloalkyl-Gruppen, Phenyl- und Benzyl-Gruppen substituiert sein, wobei die zuvor genannten Phenyl- oder Benzyl-Gruppen wiederum mit einem oder mehrerern Atomen, ausgewählt aus -R', -X, -OR', -SR', -NR'₂, -SiR'₃, -COOR', -CN und -CONR₂' substituiert sein können. In dieser Ausführungsform ist der Rest R⁷ ausgewählt aus Wasserstoff und C₁₋₄-Alkyl.

In Formel (I) haben die Reste die im Folgenden definierten *besonders bevorzugten* Bedeutungen. Die als *besonders bevorzugt* getroffenen Definitionen gelten für alle Zwischenprodukte gleichermaßen:
- R¹: ist ausgewählt aus der Gruppe bestehend aus Wasserstoff, Mercapto und Methyl;
- R²: ist ausgewählt aus der Gruppe bestehend aus Methyl und Ethyl;
- R³: ist ausgewählt aus der Gruppe bestehend aus Methyl, Ethyl und Isopropyl;

In einer alternativen *besonders bevorzugten* erfindungsgemäßen Ausführungsform bilden R² und R³, gemeinsam mit dem N-Atom, an das sie gebunden sind, einen Piperidyl-, Pyrrolidyl-oder 2,6-Dimethylmorpholinyl-Rest;
- R⁴: ist ausgewählt aus der Gruppe bestehend aus Cl und Br;
- R⁵ und R⁶: sind unabhängig voneinander ausgewählt aus Wasserstoff, Methyl- und Ethyl-Gruppen;
- R⁷: ist ausgewählt aus der Gruppe bestehend aus Methyl-, Methoxy-, Ethoxy-, Trimethylsilyl- Triethylsilyl-, Phenyl-, Benzyl-, 4-Chlorbenzyl-, 4-Chlorphenyl-, 4-Bromphenyl- 2,4-Dichlorphenyl-, 2-Chlorphenoxy-, 3-Chlorphenoxy, 4-Chlorphenoxy und 3-Trifluormethyl-Gruppen.

In einer alternativen *besonders bevorzugten* erfindungsgemäßen Ausführungsform bilden R⁵ und R⁶ gemeinsam mit dem C-Atom, an dass sie gebunden sind, einen Cyclopropylring, der mit einem oder zwei Chlor- oder Bromatomen oder mit einer Phenyl- oder Halophenyl-Gruppe substituiert sein kann. In dieser Ausführungsform ist der Rest R⁷ ausgewählt aus Halogen, vorzugsweise Chlor und Phenyl- oder Benzyl-Gruppen, die mit Halogenatomen, vorzugsweise mit Chlor substituiert sein können.

In einer weitem *besonders bevorzugten* erfindungsgemäßen Ausführungsform bilden R⁵ und R⁶ gemeinsam mit dem C-Atom, an das sie gebunden sind, eine Doppelbindung, die mit einer 4-Fluorphenyl-Gruppe substituiert sein kann. In dieser Ausführungsform ist der Rest R⁷ ausgewählt aus Wasserstoff oder C₁₋₄-Alkyl. Anders ausgedrückt ist der Thiadiazolylrest in 3-Position mit einer 4-Fluorobenzyliden-Gruppe substituiert.

Die vorliegende Erfindung betrifft darüber hinaus auch die Salze, N-Oxide, Metallkomplexe der zuvor beschriebenen Verbindungen und deren Stereoisomere.

Je nach Art der oben definierten Substituenten weisen die Verbindungen der Formel (I) saure oder basische Eigenschaften auf und können mit anorganischen oder organischen Säuren oder mit Basen oder mit Metallionen Salze, gegebenenfalls auch innere Salze oder Addukte bilden.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Tragen die Verbindungen der Formel (I) Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkyl-Gruppen, Mono-, Diund Trialkanolamine von (C₁-C₄)-Alkanolen, Cholin sowie Chlorcholin.

Tragen die Verbindungen der Formel (I) Amino, Alkylamino oder andere, basische Eigenschaften induzierende Gruppen, so können diese Verbindungen mit Säuren zu Salzen umgesetzt werden.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄.

Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxalsäure, Alkylsulfonsäuren (Sulfonsäuren mit geradkettigen oder verzweigten Alkyl-Gruppen mit 1 bis 20 Kohlenstoffatomen), Arylsulfonsäuren oder -disulfonsäuren (aromatische Gruppen wie Phenyl und Naphthyl welche ein oder zwei Sulfonsäuregruppen tragen), Alkylphosphon-säuren (Phosphonsäuren mit geradkettigen oder verzweigten Alkyl-Gruppen mit 1 bis 20 Kohlenstoffatomen), Arylphosphonsäuren oder - diphosphonsäuren (aromatische Reste wie Phenyl und Naphthyl, welche ein oder zwei Phosphonsäure-Gruppen tragen), wobei die Alkyl- bzw. Aryl-Gruppen weitere Substituenten tragen können, z.B. p-Toluolsulfonsäure, Salicylsäure, p-Aminosalicylsäure, 2-Phenoxybenzoesäure, 2-Acetoxybenzoesäure etc.

Die so erhältlichen Salze weisen ebenfalls fungizide, insektizide oder herbizide Eigenschaften auf.

Im Zusammenhang mit der vorliegenden Erfindung besonders bevorzugte Amidine sind ausgewählt aus der Gruppe bestehend aus: N'-{4-[(3-tert-Butyl-1,2,4-thiadiazol-5-yl)oxy]-2-chlor-5-methylphenyl}-N-ethyl-N-methylimidoformamid (Beispiel 1); N'-{2-Brom-4-[(3-tert-butyl-1,2,4-thiadiazol-5-yl)oxy]-5-methylphenyl}-N-ethyl-N-methylimidoformamid (Beispiel 2); N'-(2-Chlor-4-{[3-(1-chlorcyclopropyl)-1,2,4-thiadiazol-5-yl]oxy}-5-methylphenyl)-N-ethyl-N-methylimidoformamid (Beispiel 3); N'-(2-Chlor-4-{[3-(1-chlorcyclopropyl)-1,2,4-thiadiazol-5-yl]oxy}-5-methylphenyl)-N-methyl-N-propan-2-ylimidoformamid (Beispiel 4); 2-Chlor-4-{[3-(1-chlorcyclopropyl)-1,2,4-thiadiazol-5-yl]oxy}-5-methyl-N-[(E)-piperidin-1-ylmethyliden]anilin (Beispiel 5); N'-[2-Chlor-4-({3-[1-(4-chlorphenyl)ethyl]-1,2,4-thiadiazol-5-yl}oxy)-5-methylphenyl]-N-ethyl-N-methylimidoformamid (Beispiel 6); 2-Chlor-4-({3-[1-(4-chlorphenyl)ethyl]-1,2,4-thiadiazol-5-yl}oxy)-5-methyl-N-[(E)-piperidin-1-ylmethyliden]anilin (Beispiel 7); N'-[2-Chlor-4-({3-[1-(4-chlorphenyl)ethyl]-1,2,4-thiadiazol-5-yl}oxy)-5-methylphenyl]-N-methyl-N-propan-2-ylimidoformamid (Beispiel 8); N'-[2-Chlor-4-({3-[2-(4-chlorphenyl)ethyl]-1,2,4-thiadiazol-5-yl}oxy)-5-methylphenyl]-N-ethyl-N-methylimidoformamid (Beispiel 9); 2-Chlor-4-({3-[2-(4-chlorphenyl)ethyl]-1,2,4-thiadiazol-5-yl}oxy)-5-methyl-N-[(Z)-piperidin-1-ylmethyliden]anilin (Beispiel 10); N'-[2-Chlor-4-({3-[2-(4-chlorphenyl)propan-2-yl]-1,2,4-thiadiazol-5-yl}oxy)-5-methylphenyl]-N-ethyl-N-methylimidoformamid (Beispiel 11); 2-Chlor-4-({3-[2-(4-chlorphenyl)propan-2-yl]-1,2,4-thiadiazol-5-yl}oxy)-5-methyl-N-[(Z)-piperidin-1-ylmethyliden]anilin (Beispiel 12); N'-[2-Chlor-4-({3-[2-(4-chlorphenyl)propan-2-yl]-1,2,4-thiadiazol-5-yl}oxy)-5-methylphenyl]-N-methyl-N-propan-2-ylimidoformamid (Beispiel 13); N'-{4-[(3-Benzyl-1,2,4-thiadiazol-5-yl)oxy]-2-chlor-5-methylphenyl}-N-ethyl-N-methylimidoformamid (Beispiel 14); N'-[2-Chlor-4-({3-[(E)-2-(4-fluorphenyl)ethenyl]-1,2,4-thiadiazol-5-yl}oxy)-5-methylphenyl]-N-ethyl-N-methylimidoformamid (Beispiel 15); 2-Chlor-4-({3-[(E)-2-(4-fluorphenyl)ethenyl]-1,2,4-thiadiazol-5-yl}oxy)-5-methyl-N-[(Z)-piperidin-1-ylmethyliden]anilin (Beispiel 16); N'-[2-Chlor-4-({3-[1-(4-chlorphenyl)cyclopropyl]-1,2,4-thiadiazol-5-yl}oxy)-5-methylphenyl]-N-ethyl-N-methylimidoformamid (Beispiel 17); N'-(2-Chlor-4-{[3-(4-chlorbenzyl)-1,2,4-thiadiazol-5-yl]oxy}-5-methylphenyl)-N-ethyl-N-methylimidoformamid (Beispiel 18); N'-(2-Chlor-4-{[3-(chlormethyl)-1,2,4-thiadiazol-5-yl]oxy}-5-methylphenyl)-N-ethyl-N-methylimidoformamid (Beispiel 19); N'-[2-Chlor-4-({3-[1-(4-chlorphenyl)cyclopropyl]-1,2,4-thiadiazol-5-yl}oxy)-5-methylphenyl]-N-methyl-N-propan-2-ylimidoformamid (Beispiel 20); 2-Chlor-4-({3-[1-(4-chlorphenyl)cyclopropyl]-1,2,4-thiadiazol-5-yl}oxy)-5-methyl-N-[(Z)-piperidin-1-ylmethyliden]anilin (Beispiel 21); N'-[2-Chlor-4-({3-[(4-chlorphenoxy)methyl]-1,2,4-thiadiazol-5-yl}oxy)-5-methylphenyl]-N-ethyl-N-methylimidoformamid (Beispiel 22); N'-[2-Chlor-4-({3-[(E)-2-(4-fluorphenyl)ethenyl]-1,2,4-thiadiazol-5-yl}oxy)-5-methylphenyl]-N-methyl-N-propan-2-ylimidoformamid (Beispiel 23); N'-{4-[(3-tert-Butyl-1,2,4-thiadiazol-5-yl)oxy]-2-chlor-5-methylphenyl}-N-methyl-N-propylimidoformamid (Beispiel 24); N'-{4-[(3-tert-Butyl-1,2,4-thiadiazol-5-yl)oxy]-2-chlor-5-methylphenyl}-N-methyl-N-prop-2-en-1-ylimidoformamid (Beispiel 25); N'-{4-[(3-tert-Butyl-1,2,4-thiadiazol-5-yl)oxy]-2-chlor-5-methylphenyl}-N-(cyclopropylmethyl)-N-methylimidoformamid (Beispiel 26); N'-{4-[(3-tert-Butyl-1,2,4-thiadiazol-5-yl)oxy]-2-chior-5-methylphenyl}-N-(3-methoxypropyl)-N-methylimidoformamid (Beispiel 27); N'-(2-Chlor-5-methyl-4-{[3-(2-methyl-1-phenylpropan-2-yl)-1,2,4-thiadiazol-5-yl]oxy}phenyl)-N-ethyl-N-methylimidoformamid (Beispiel 28); rel-N'-[2-Chlor-5-methyl-4-({3-[(1R,2R)-2-phenylcyclopropyl]-1,2,4-thiadiazol-5-yl}oxy)phenyl]-N-ethyl-N-methylimidoformamid (Beispiel 29); rel-2-Chlor-5-methyl-4-({3-[(1R,2R)-2-phenyl-cyclopropyl]-1,2,4-thiadiazol-5-yl}oxy)-N-[(E)-piperidin-1-ylmethyliden]anilin (Beispiel 30); rel-N'-[2-Chlor-5-methyl-4-({3-[(1R,2R)-2-phenylcyclopropyl]-1,2,4-thiadiazol-5-yl}oxy)phenyl]-N-methyl-N-propan-2-ylimidoformamid (Beispiel 31); N'-(2-Chlor-4-{[3-(2,2-dichlor-1-methylcyclopropyl)-1,2,4-thiadiazol-5-yl]oxy}-5-methylphenyl)-N-ethyl-N-methylimidoformamid (Beispiel 32); N'-(4-{[3-(4-Brombenzyl)-1,2,4-thiadiazol-5-yl]oxy}-2-chlor-5-methylphenyl)-N-ethyl-N-methylimidoformamid (Beispiel 33); N'-[2-Chlor-5-methyl-4-({3-[3-(trifluormethyl)benzyl]-1,2,4-thiadiazol-5-yl}oxy)phenyl]-N-ethyl-N-methylimidoformamid (Beispiel 34); N'-[2-Chlor-4-({3-[(3-chlorphenoxy)methyl]-1,2,4-thiadiazol-5-yl}oxy)-5-methylphenyl]-N-ethyl-N-methylimidoformamid (Beispiel 35); N'-[2-Chlor-4-({3-[(2-chlorphenoxy)methyl]-1,2,4-thiadiazol-5-yl}oxy)-5-methylphenyl]-N-ethyl-N-methylimidoformamid (Beispiel 36); N'-(2-Chlor-5-methyl-4-{[3-(phenoxymethyl)-1,2,4-thiadiazol-5-yl]oxy}phenyl)-N-ethyl-N-methylimidoformamid (Beispiel 37); N'-(2-Chlor-4-{[3-(2,4-dichlorbenzyl)-1,2,4-thiadiazol-5-yl]oxy}-5-methylphenyl)-N-ethyl-N-methylimidoformamid (Beispiel 38); N'-(2-Chlor-4-{[3-(1-chlor-2-methylpropan-2-yl)-1,2,4-thiadiazol-5-yl]oxy}-5-methylphenyl)-N-ethyl-N-methylimidoformamid (Beispiel 39); N'-(2-Chlor-4-{[3-(2,4-difluorbenzyl)-1,2,4-thiadiazol-5-yl]oxy}-5-methylphenyl)-N-ethyl-N-methylimidoformamid (Beispiel 40).

### Herstellung der erfindungsgemäßen Amidine

Die erfindungsgemäßen Amidine können durch das in den folgenden Schemata (Ia) und (Ib) dargestellte Verfahren erhalten werden:

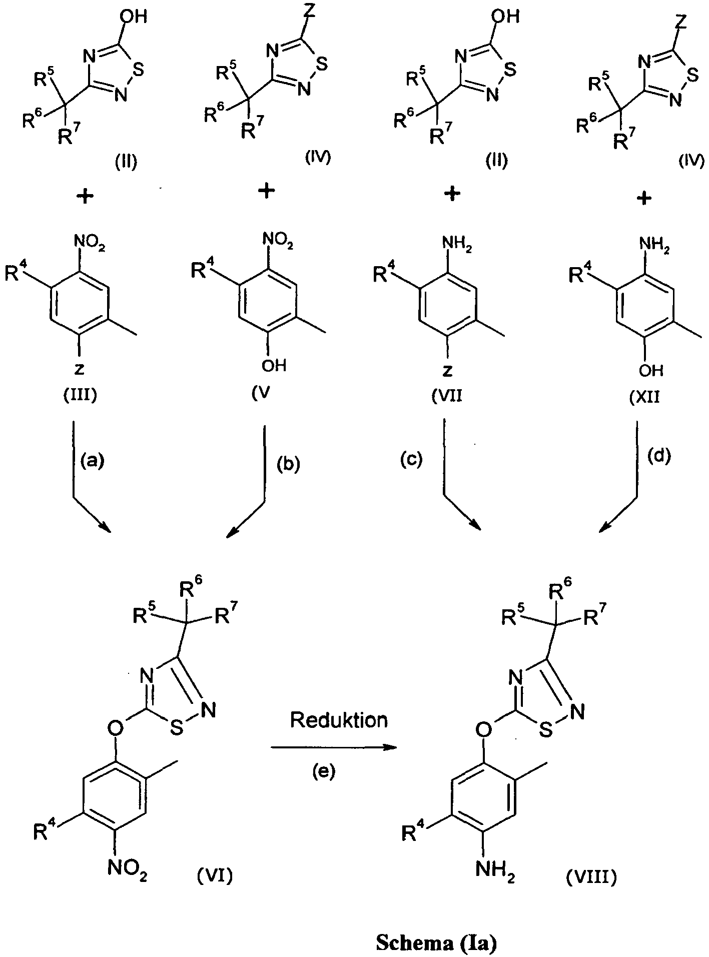

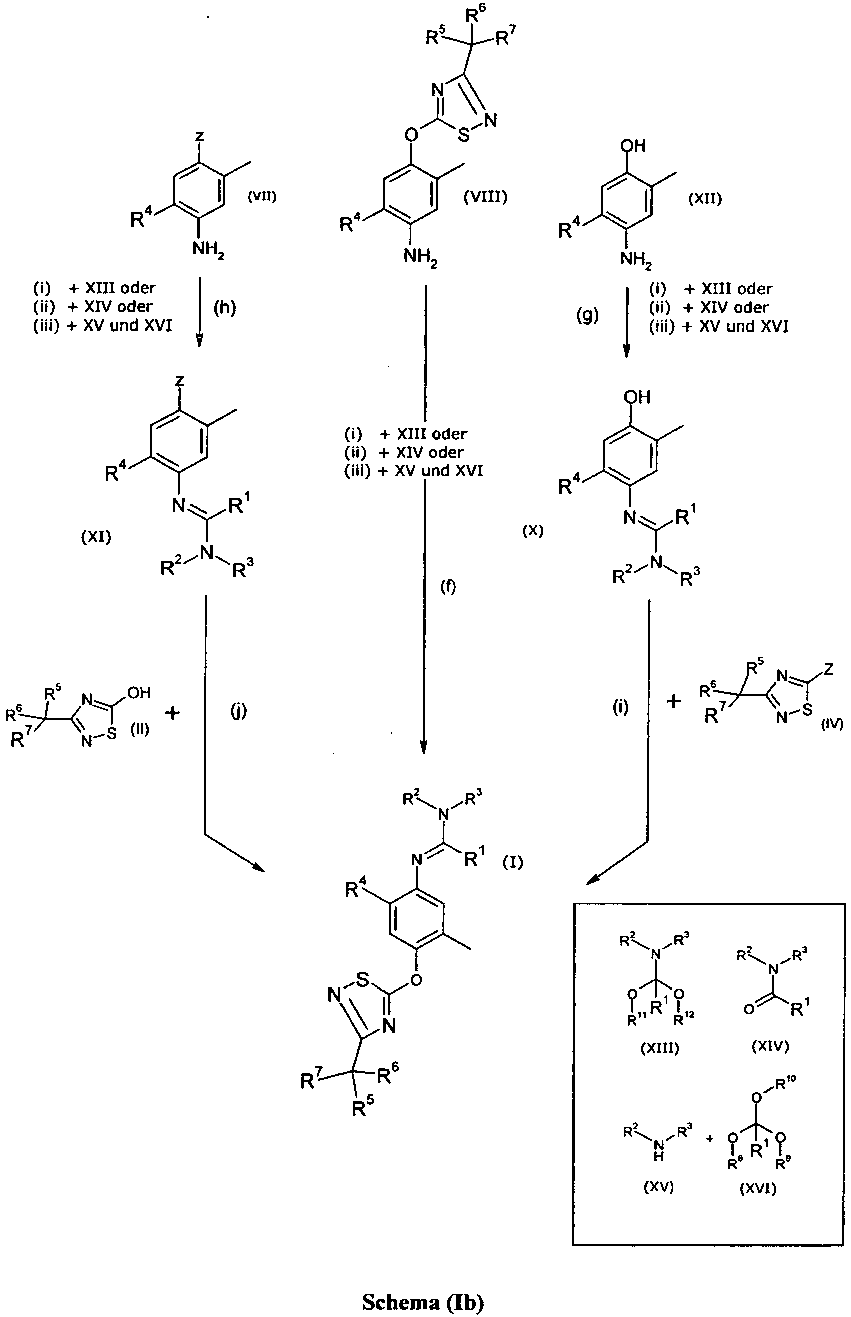

### Schritt (a)

In einer erfindungsgemäßen Ausführungsform werden Nitrobenzolderivate der Formel (III) mit Thiadiazolyl-Alkoholen der Formel (II) oder den daraus gebildeten Alkoholaten gemäß dem nachfolgenden Reaktionsschema zu Nitrophenylethern der Formel (VI) umgesetzt:

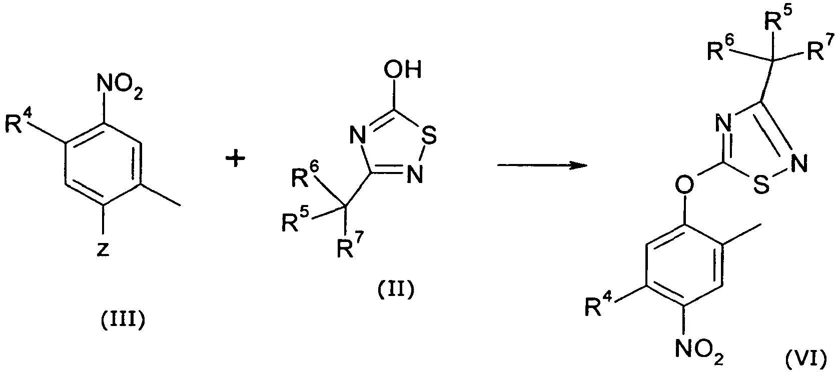

Als Austrittsgruppe Z sind alle Substituenten geeignet, die bei den vorherrschenden Reaktionsbedingungen eine ausreichende Nucleofugizität aufweisen. Beispielhaft seien Halogene, Triflat, Mesylat, Tosylat oder SO₂Me als geeignete Austrittsgruppen genannt.

Die Nitrobenzolderivate der Formel (III) sind gemäß Journal of the Chemical Society 1926, 2036 erhältlich.

Die Reaktion erfolgt vorzugsweise in Gegenwart einer Base.

Geeignete Basen sind organische und anorganische Basen, die üblicherweise in solchen Reaktionen verwendet werden. Vorzugsweise werden Basen verwendet, die beispielhaft ausgewählt sind aus der Gruppe bestehend aus Hydriden, Hydroxiden, Amiden, Alkoholaten, Acetaten, Fluoriden, Phosphaten, Carbonaten und Hydrogencarbonaten von Alkali- oder Erdalkalimetallen. Besonders bevorzugt sind dabei Natriumamid, Natriumhydrid, Lithiumdiisopropylamid, Natriummethanolat, Kalium-tert-butanolat, Natriumhydroxid, Kaliumhydroxid, Natriumacetat, Natriumphosphat, Kaliumphosphat, Kaliumfluorid, Caesiumfluorid, Natriumcarbonat, Kaliumcarbonat, Kaliumhydrogencarbonat, Natriumhydrogencarbonat und Caesiumcarbonat. Außerdem sind tertiäre Amine, wie z.B. Trimethylamin, Triethylamin, Tributylamin, N,N-Dimethylanilin, N,N-Dimethylbenzylamin, Pyridin, N-Methylpiperidin, N-Methylpyrolidon, N,N-Dimethylaminopyridin, Diazabicyclooctan (DABCO), Diazabicyclononen (DBN) und Diazabicycloundecen (DBU).

Gegebenenfalls kann ein Katalysator, der ausgewählt ist aus der Gruppe bestehend aus Palladium, Kupfer und deren Salzen oder Komplexen eingesetzt werden.

Die Reaktion des Nitrobenzolderivats mit dem Phenol kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln.

Bevorzugt werden aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlorkohlenstoff, Dichlorethan oder Trichlorethan; Ether, wie beispielsweise Diethylether, Diisopropylether, Methyl-tert-butylether (MTBE), Methyl-tert-amylether, Dioxan, Tetrahydrofuran, 1,2-Dimethoxyethan, 1,2-Diethoxyethan oder Anisol; Nitrile, wie beispielsweise Acetonitril, Propionitril, n- oder iso-Butyronitril oder Benzonitril; Amide, wie beispielsweise N,N-Dimethylformamid (DMF), N,N-Dimethylacetamid, N-Methylformanilid, N-Methylpyrrolidon (NMP) oder Hexamethylenphosphorsäuretriamid; oder Mischungen dieser mit Wasser sowie reines Wasser.

Die Reaktion kann im Vakuum, bei Normaldruck oder unter Überdruck und bei Temperaturen von -20 bis 200 °C durchgeführt werden, vorzugsweise erfolgt die Reaktion bei Normaldruck und Temperaturen von 50 bis 150 °C.

### Schritt (b)

In einer alternativen erfindungsgemäßen Ausführungsform werden Nitrophenolderivate der Formel (V) oder die daraus gebildeten Phenolate mit Thiadiazolyl-Derivaten der Formel (IV) gemäß dem nachfolgenden Reaktionsschema zu Nitrophenylethern der Formel (VI) umgesetzt:

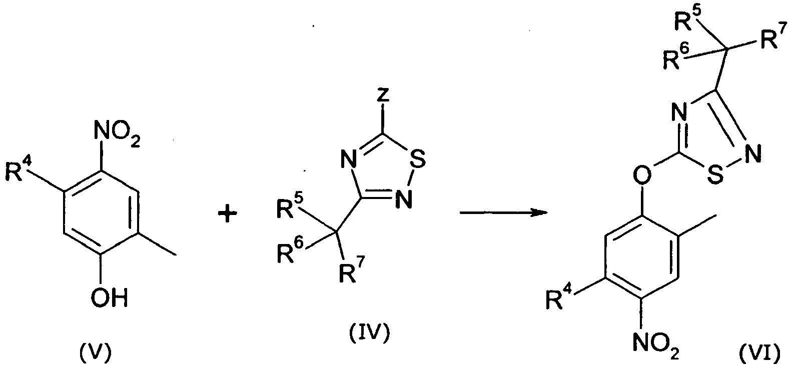

Die Nitrophenolderivate der Formel (V) sind gemäß Journal of the Chemical Society 1926, 2036 erhältlich.

Im Hinblick auf Reaktionsbedingungen, Lösungsmittel, Katalysatoren und geeignete Austrittsgruppen, sei auf Schritt (a) verwiesen.

### Schritt (c)

In einer weiteren alternativen erfindungsgemäßen Ausführungsform werden Aniline der Formel (VII) mit Thiadiazolyl-Alkoholen der Formel (II) oder den daraus gebildeten Alkoholaten gemäß dem nachfolgenden Reaktionsschema zu Aminophenylethern der Formel (VIII) umgesetzt:

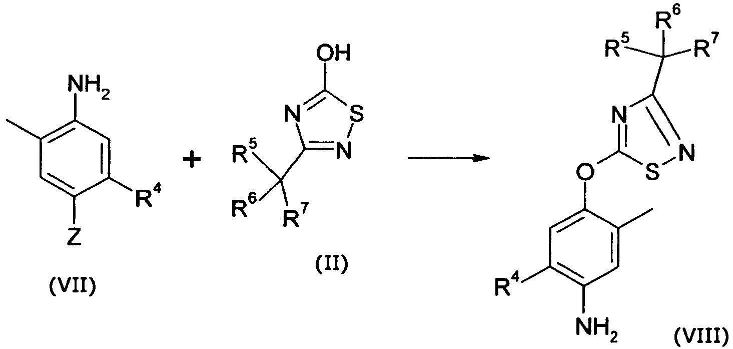

Im Hinblick auf Reaktionsbedingungen, Lösungsmittel, Katalysatoren und geeignete Austrittsgruppen, sei auf Schritt (a) verwiesen.

### Schritt (d)

In einer weiteren alternativen erfindungsgemäßen Ausführungsform werden Aminophenole der Formel (XII) mit Thiadiazolyl-Derivaten der Formel (IV) gemäß dem nachfolgenden Reaktionsschema zu Aminophenylethern der Formel (VIII) umgesetzt:

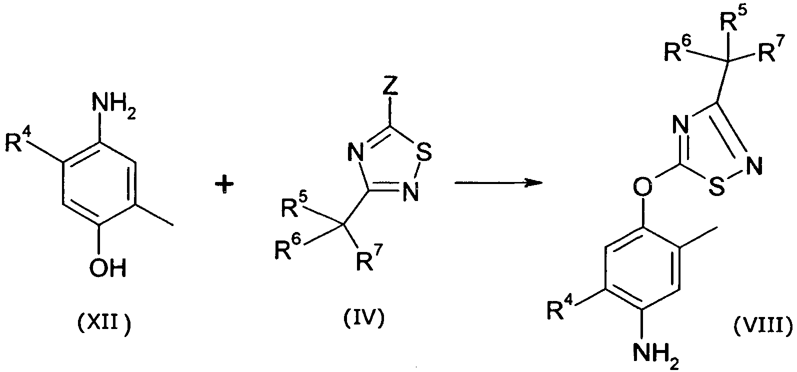

Im Hinblick auf Reaktionsbedingungen, Lösungsmittel, Katalysatoren und geeignete Austrittsgruppen, sei auf Schritte (a) und (c) verwiesen.

### Schritt (e)

Die in den Schritten (a) und (b) erhaltenen Nitrophenylether der Formel (VI) können gemäß dem nachfolgenden Reaktionsschema zu den Anilinethern der Formel (VIII) reduziert werden:

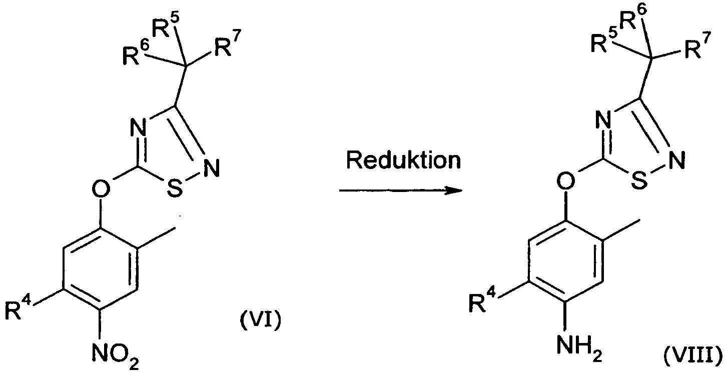

Die Reduktion gemäß Schritt (e) kann durch sämtliche im Stand der Technik zur Reduktion von Nitro-Gruppen beschriebenen Methoden erfolgen.

Vorzugsweise erfolgt die Reduktion mit Zinnchlorid in konzentrierter Salzsäure wie in WO-A-0 046 184 beschrieben. Alternativ kann die Reduktion aber auch mit Wasserstoffgas, ggf. in Gegenwart geeigneter Hydrierkatalysatoren, wie z.B. Raney-Nickel oder Pd/C, erfolgen. Die Reaktionsbedingungen sind im Stand der Technik vorbeschrieben und dem Fachmann geläufig.

Wird die Reduktion in der Flüssigphase durchgeführt, so sollte die Reaktion in einem gegenüber den vorherrschenden Reaktionsbedingungen inerten Lösungsmittel erfolgen. Ein solches ist beispielsweise Toluol.

### Schritt (f)

Die Umsetzung der Anilinethern der Formel (VIII) zu den erfindungsgemäßen Amidinen der Formel (I) gemäß Schritt (f) kann, wie zuvor in Schema (I) dargestellt nach unterschiedlichen alternativen Methoden unter Verwendung von
(i) Aminoacetalen der Formel (XIII) oder
(ii) Amiden der Formel (XIV) oder
(iii) Aminen der Formel (XV) in Gegenwart von Orthoestern der Formel (XVI)
   gemäß dem nachfolgenden Reaktionsschema erfolgen:

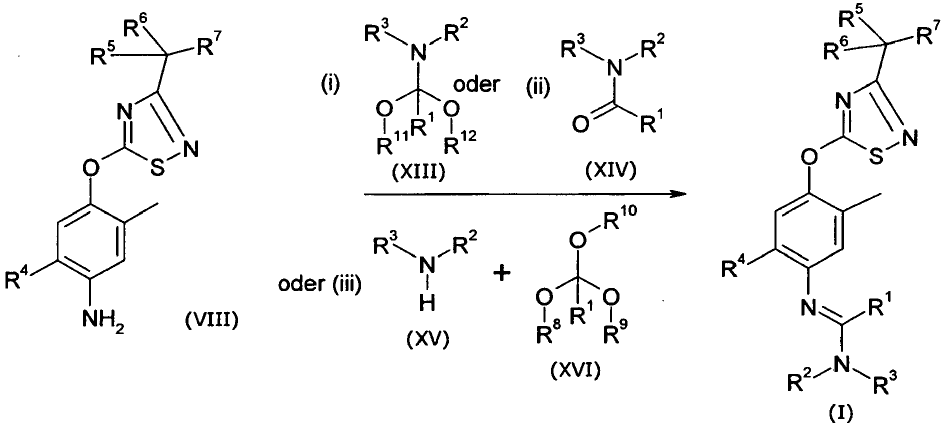

Die einzelnen alternativen Ausführungsformen (i) bis (iii) des erfindungsgemäßen Verfahrens sollen infolge kurz erläutert werden:
(i) Gemäß einer erfindungsgemäßen Ausführungsform, die im Schema (I) als Schritt (i) dargestellt ist, werden die Anilinether der Formel (VIII) mit Aminoacetalen der Formel (XIII), in der R¹, R² und R³ wie zuvor beschrieben definiert sind und R¹¹ und R¹² ausgewählt sind aus C₁₋₈-Alkyl-Gruppen, vorzugsweise aus C₂₋₆-Alkyl-Gruppen, besonders bevorzugt aus C₃₋₅-Alkyl-Gruppen und gemeinsam mit den O-Atomen, an die sie gebunden sind, einen fünf- oder sechsgliedrigen Ring bilden können, zu den erfindungsgemäßen Thiadiazolyloxyphenylamidinen der Formel (I) umgesetzt.
   Die Aminoacetale der Formel (XIII) sind aus den in JACS, 65, 1566 (1943) beschriebenen Formamiden durch Umsetzung mit Alkylierungsreagenzien, wie z.B. Dimethylsulfat, erhältlich.
   Die Reaktion gemäß Schritt (i) erfolgt vorzugsweise in Gegenwart einer Säure.
   Geeignete Säuren sind beispielsweise ausgewählt aus der Gruppe bestehend aus organischen und anorganischen Säuren, wobei p-Toluolsulfonsäure, Methansulfonsäure, Salzsäure (gasförmig, wässrig oder in organischer Lösung) oder Schwefelsäure.
(ii) In einer alternativen erfindungsgemäßen Ausführungsform, die im Schema (I) als Schritt (ii) dargestellt ist, werden die Anilinether der Formel (VIII) mit Amiden der Formel (XIV), in der die Gruppen R¹, R² und R³ wie zuvor definiert sind, zu den erfindungsgemäßen Thiadiazolyloxyphenylamidinen umgesetzt.
   Die Reaktion gemäß Schritt (ii) erfolgt ggf. in Gegenwart eines Halogenierungsmittels. Geeignete Halogenierungsmittel sind beispielsweise ausgewählt aus der Gruppe bestehend aus PCl₅, PCl₃, POCl₃ oder SOCl₂.
   Zudem kann die Reaktion alternativ in Gegenwart eines Kondensationsmittels erfolgen.
   Geeignete Kondensationsmittel sind solche, die üblicherweise zur Knüpfung von Amidbindungen verwendet werden, beispielhaft seien Säurehalogenidbildner wie z.B. Phosgen, Phosphortribromid, Phosphortrichlorid, Phosphorpentachlorid, Phosphortrichloridoxid oder Thionylchlorid; Anhydridbildner wie z.B. Chlorformiat, Methyl-chlorformiat, Isopropylehlorformiat, Isobutylchlorformiat oder Methansulfonylchlorid; Carbodiimine wie z.B. N,N'-Dicyclohexylcarbodiimin (DCC) oder andere übliche Kondensationsmittel wie z.B. Phosphorpentoxid, Polyphosphorsäure, N,N'-Carbodiimidazol, 2-Ethoxy-N-ethoxycarbonyl-1,2-dihydroquinolin (EEDQ), Triphenyl-phosphin/Tetrachlormethan oder Bromtripyrrolidinophosphoniumhexafluorophosphat genannt.
   Die Reaktion gemäß Schritt (ii) erfolgt vorzugsweise in einem Lösungsmittel, welches ausgewählt ist aus den üblichen, bei den vorherrschenden Reaktionsbedingungen inerten Lösungsmitteln. Bevorzugt werden aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlorkohlenstoff, Dichlorethan oder Trichlorethan; Ether, wie beispielsweise Diethylether, Diisopropylether, Methyl-tert-butylether (MTBE), Methyl-tert-amylether, Dioxan, Tetrahydrofuran, 1,2-Dimethoxyethan, 1,2-Diethoxyethan oder Anisol; Nitrile, wie beispielsweise Acetonitril, Propionitril, n- oder iso-Butyronitril oder Benzonitril; Amide, wie beispielsweise N,N-Dimethylformamid (DMF), N,N-Dimethylacetamid, N-Methylformanilid, N-Methylpyrrolidon (NMP) oder Hexamethylenphosphorsäuretriamid; Ester, wie beispielsweise Methyl- oder Ethylacetat; Sulfoxide, wie beispielsweise Dimethylsulfoxid (DMSO); Sulfone, wie beispielsweise Sulfolan; Alkohole, wie beispielsweise Methanol, Ethanol, n- oder iso-Propanol, n-, iso-, sec-oder tert-Butanol, Ethandiol, Propan-1,2-diol, Ethoxiethanol, Methoxyethanol, Diethylenglycolmonomethylether, Diethylenglycolmonoethylether oder Mischungen dieser verwendet.
(iii) Gemäß einer weiteren alternativen erfindungsgemäßen Ausführungsform, die in Schema (I) als Schritt (iii) dargestellt ist, werden die Anilinether der Formel (VIII) mit Aminen der Formel (XV), in der die Gruppen R² und R³ wie zuvor definiert sind in Gegenwart von Orthoestern der Formel (XVI), in der R¹ Wasserstoff ist und R⁸ bis R¹⁰ unabhängig voneinander ausgewählt sind aus C₁₋₈-Alkyl-Gruppen, vorzugsweise aus C₂₋₆-Alkyl-Gruppen, besonders bevorzugt aus C₃₋₅-Alkyl-Gruppen und gemeinsam mit den O-Atomen, an die sie gebunden sind, einen fünf- oder sechsgliedrigen Ring bilden können, zu den erfindungsgemäßen Thiadiazolyloxyphenylamidinen umgesetzt.
   Die Reaktion gemäß Schritt (iii) erfolgt vorzugsweise in einem Lösungsmittel, welches ausgewählt ist aus den üblichen, bei den vorherrschenden Reaktionsbedingungen inerten Lösungsmitteln. Bevorzugt werden aliphatische, alicyclische oder aromatische Kohlenwasserstoffe, wie beispielsweise Petrolether, Hexan, Heptan, Cyclohexan, Methyl-cyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlorkohlenstoff, Dichlorethan oder Trichlorethan; Ether, wie beispielsweise Diethylether, Diisopropylether, Methyl-tert-butylether (MTBE), Methyl-tert-amylether, Dioxan, Tetrahydrofuran, 1,2-Dimethoxyethan, 1,2-Diethoxyethan oder Anisol; Nitrile, wie beispielsweise Acetonitril, Propionitril, n- oder iso-Butyronitril oder Benzonitril; Amide, wie beispielsweise N,N-Dimethylformamid (DMF), N,N-Dimethylacetamid, N-Methylformanilid, N-Methylpyrrolidon (NMP) oder Hexamethylenphosphorsäuretriamid; Ester, wie beispielsweise Methyl- oder Ethylacetat; Sulfoxide, wie beispielsweise Dimethylsulfoxid (DMSO); Sulfone, wie beispielsweise Sulfolan; Alkohole, wie beispielsweise Methanol, Ethanol, n- oder iso-Propanol, n-, iso-, sec- oder tert-Butanol, Ethandiol, Propan-1,2-diol, Ethoxiethanol, Methoxyethanol, Diethylenglycolmonomethylether, Diethylenglycolmonoethylether; oder Mischungen dieser mit Wasser sowie reines Wasser verwendet.

### Schritt (g)

In einer alternativen erfindungsgemäßen Ausführungsform können bereits die Aminophenole der Formel (XII)
(i) mit Aminoacetalen der Formel (XIII) oder
(ii) mit Amiden der Formel (XIV) oder
(iii) mit Aminen der Formel (XV) in Gegenwart von Orthoestern der Formel (XVI)
   gemäß dem nachfolgenden Reaktionsschema zu Amidinen der Formel (X) umgesetzt werden:

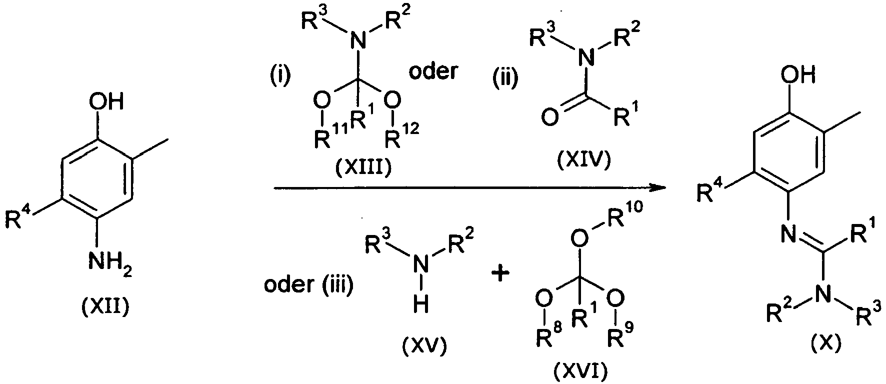

Hinsichtlich der Reaktionsbedingungen, Lösungsmittel und Katalysatoren, sei auf Schritt (f) verwiesen.

Die weitere Umsetzung der Amidine der Formel (X) zu den erfindungsgemäßen Zielmolekülen der Formel (I) kann beispielsweise wie in Schritt (j) beschrieben erfolgen.

### Schritt (h)

In einer alternativen erfindungsgemäßen Ausführungsform können die Aminophenylderivate der Formel (VII)
(i) mit Aminoacetalen der Formel (XIII) oder
(ii) mit Amiden der Formel (XIV) oder
(iii) mit Aminen der Formel (XV) in Gegenwart von Orthoestern der Formel (XVI)
   gemäß dem nachfolgenden Reaktionsschema zu Amidinen der Formel (XI) umgesetzt werden:

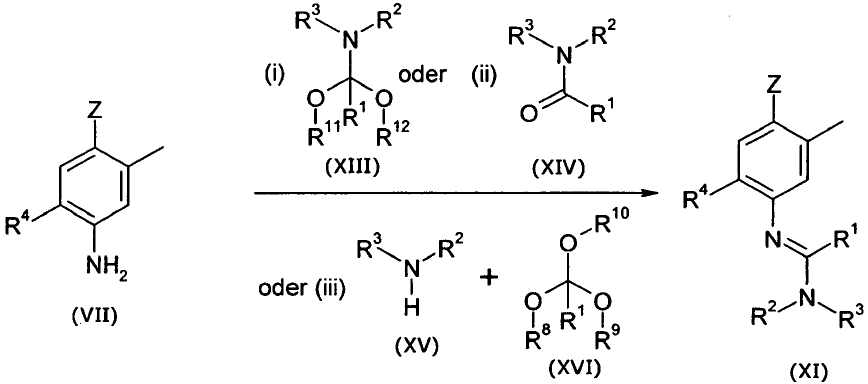

Hinsichtlich der Reaktionsbedingungen, Lösungsmittel und Katalysatoren, sei auf Schritt (f) verwiesen.

Die weitere Umsetzung der Amidine der Formel (XI) zu den erfindungsgemäßen Zielmolekülen der Formel (I) kann beispielsweise wie in Schritt (i) beschrieben erfolgen.

### Schritt (i)

Gemäß einer weiteren erfindungsgemäßen Ausführungsform können die aus Schritt (h) erhältlichen Amidine der Formel (XI) mit Thiadiazolyl-Alkoholen der Formel (II) oder den daraus gebildeten Alkoholaten zu den erfindungsgemäßen Zielmolekülen der Formel (I) gemäß dem nachfolgenden Reaktionsschema umgesetzt werden:

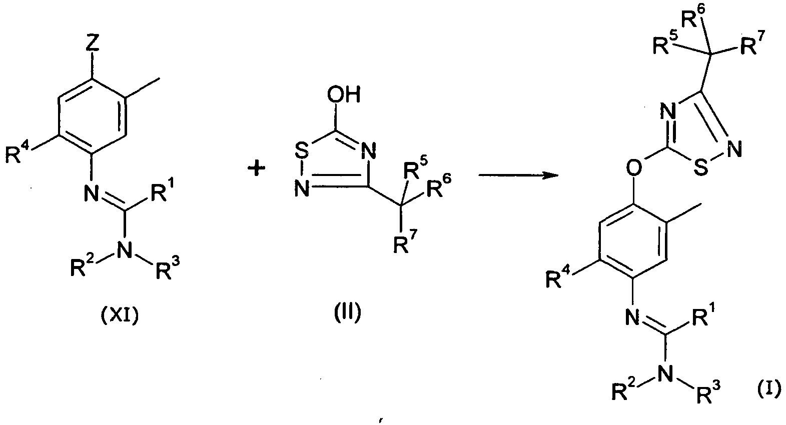

Hinsichtlich der Reaktionsbedingungen, Lösungsmittel und Katalysatoren sei auf Schritt (f) verwiesen.

### Schritt (j)

Gemäß einer weiteren erfindungsgemäßen Ausführungsform können die aus Schritt (g) erhältlichen Amidine der Formel (X) mit Thiadiazolyl-Derivaten der Formel (IV) zu den erfindungsgemäßen Zielmolekülen der Formel (I) gemäß dem nachfolgenden Reaktionsschema umgesetzt werden:

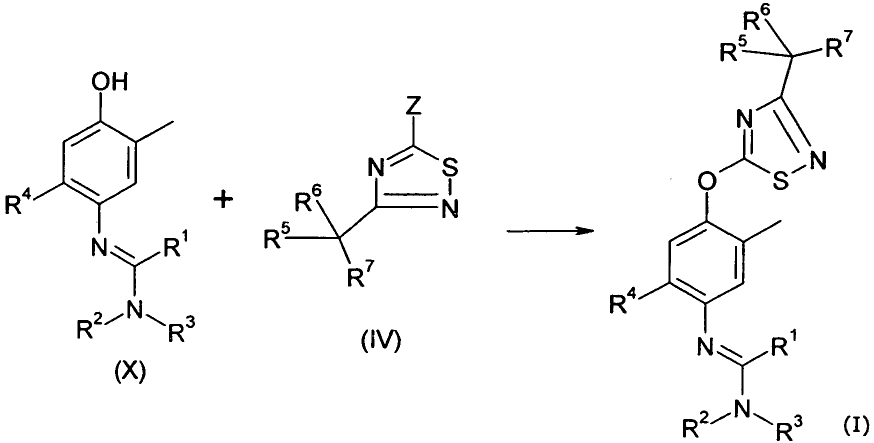

Hinsichtlich der Reaktionsbedingungen, Lösungsmittel und Katalysatoren, sei auf Schritt (f) und auf Tabellen I und II verwiesen.

Im Zusammenhang mit den erfindungsgemäßen Verfahren zur Herstellung der Amidine der Formel (I) sind die folgenden Kombinationen von Reaktionsschritten als Vorteilhaft anzusehen: Schritte (a), (e) und (f); Schritte (b), (e) und (f); Schritte (c) und (f); Schritte (d) und (f); Schritte (h) und (i) und/oder Schritte (g) und (j).

Die Herstellung der erfindungsgemäßen Thiadiazolyloxyphenylamidine erfolgt ggf. ohne zwischenzeitliche Isolierung der Zwischenprodukte.

Die abschließende Reinigung der Thiadiazolyloxyphenylamidine kann ggf. durch übliche Reinigungsverfahren erfolgen. Vorzugsweise erfolgt die Reinigung durch Kristallisation.

Die in Schritten (b), (d) und (j) des zuvor beschriebenen Verfahrens verwendeten Thiadiazolyl-Derivate der Formel IVa worin Z ein Chloratom ist, können beispielsweise gemäß dem im nachfolgenden Schema oder den in DE-A-960281 oder in Chemische Berichte, 90, 182-7; 1957 beschriebenen Verfahren erhalten werden:

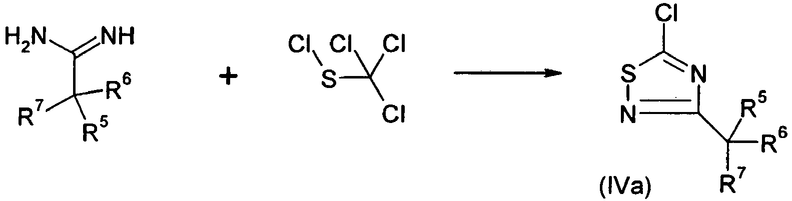

Die Chloride der Formel (VIa) können durch saure Hydrolyse in die Alkohole der Formel (II) überführt werden.

Die in Schritten (b), (d) und (j) des zuvor beschriebenen Verfahrens verwendeten Thiadiazolyl-Derivate der Formel IVb, worin Z eine Tosyl-Gruppe ist, können beispielsweise gemäß dem im nachfolgenden Schema beschriebenen Verfahren erhalten werden:

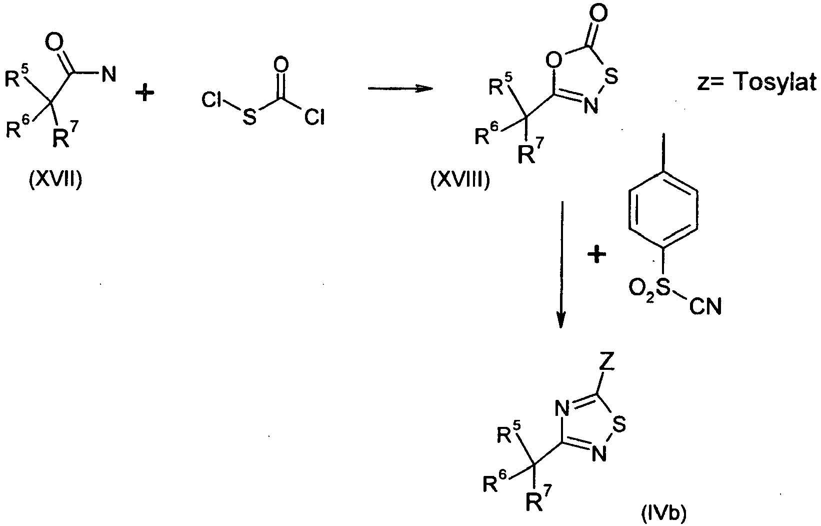

Die verwendeten Carbonsäureamide der allgemeinen Formel (XVII) können beispielsweise nach der Vorschrift in Houben-Weyl VIII, S.655 ff. hergestellt werden.

### Bekämpfung von unerwünschten Mikroorganismen

Die erfindungsgemäßen Amidine weisen eine starke mikrobizide Wirkung auf und können zur Bekämpfung von unerwünschten Mikroorganismen, wie Fungi (Pilze) und Bakterien, im Pflanzenschutz und im Materialschutz eingesetzt werden.

### Pflanzenschutz

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen. Beispielhaft aber nicht begrenzend seien einige Erreger von pilzlichen und bakteriellen Erkrankungen, die unter die oben aufgezählten Oberbegriffe fallen, genannt:
Erkrankungen, hervorgerufen durch Erreger des Echten Mehltaus wie z.B.
Blumeria-Arten, wie beispielsweise Blumeria graminis;
Podosphaera-Arten, wie beispielsweise Podosphaera leucotricha;
Sphaerotheca-Arten, wie beispielsweise Sphaerotheca fuliginea;
Uncinula-Arten, wie beispielsweise Uncinula necator;
Erkrankungen, hervorgerufen durch Erreger von Rostkrankheiten wie z.B.
Gymnosporangium-Arten, wie beispielsweise Gymnosporangium sabinae
Hemileia-Arten, wie beispielsweise Hemileia vastatrix;
Phakopsora-Arten, wie beispielsweise Phakopsora pachyrhizi und Phakopsora meibomiae;
Puccinia-Arten, wie beispielsweise Puccinia recondita;
Uromyces-Arten, wie beispielsweise Uromyces appendiculatus;
Erkrankungen, hervorgerufen durch Erreger der Gruppe der Oomyceten wie z.B.
Bremia-Arten, wie beispielsweise Bremia lactucae;
Peronospora-Arten, wie beispielsweise Peronospora pisi oder P. brassicae;
Phytophthora-Arten, wie beispielsweise Phytophthora infestans;
Plasmopara-Arten, wie beispielsweise Plasmopara viticola;
Pseudoperonospora-Arten, wie beispielsweise Pseudoperonospora humuli oder Pseudoperonospora cubensis;
Pythium-Arten, wie beispielsweise Pythium ultimum;
Blattfleckenkrankheiten und Blattwelken, hervorgerufen durch z.B.
Alternaria-Arten, wie beispielsweise Alternaria solani;
Cercospora-Arten, wie beispielsweise Cercospora beticola;
Cladosporium-Arten, wie beispielsweise Cladosporium cucumerinum;
Cochliobolus-Arten, wie beispielsweise Cochliobolus sativus
(Konidienform: Drechslera, Syn: Helminthosporium);
Colletotrichum-Arten, wie beispielsweise Colletotrichum lindemuthanium;
Cycloconium-Arten, wie beispielsweise Cycloconium oleaginum;
Diaporthe-Arten, wie beispielsweise Diaporthe citri;
Elsinoe-Arten, wie beispielsweise Elsinoe fawcettii;
Gloeosporium-Arten, wie beispielsweise Gloeosporium laeticolor;
Glomerella-Arten, wie beispielsweise Glomerella cingulata;
Guignardia-Arten, wie beispielsweise Guignardia bidwelli;
Leptosphaeria-Arten, wie beispielsweise Leptosphaeria maculans;
Magnaporthe-Arten, wie beispielsweise Magnaporthe grisea;
Mycosphaerella-Arten, wie beispielsweise Mycosphaerella graminicola und Mycosphaerella fijiensis;
Phaeosphaeria-Arten, wie beispielsweise Phaeosphaeria nodorum;
Pyrenophora-Arten, wie beispielsweise Pyrenophora teres;
Ramularia-Arten, wie beispielsweise Ramularia collo-cygni;
Rhynchosporium-Arten, wie beispielsweise Rhynchosporium secalis;
Septoria-Arten, wie beispielsweise Septoria apii;
Typhula-Arten, wie beispielsweise Typhula incarnata;
Venturia-Arten, wie beispielsweise Venturia inaequalis;
Wurzel- und Stengelkrankheiten, hervorgerufen durch z.B.
Corticium-Arten, wie beispielsweise Corticium graminearum;
Fusarium-Arten, wie beispielsweise Fusarium oxysporum;
Gaeumannomyces-Arten, wie beispielsweise Gaeumannomyces graminis;
Rhizoctonia-Arten, wie beispielsweise Rhizoctonia solani;
Tapesia-Arten, wie beispielsweise Tapesia acuformis;
Thielaviopsis-Arten, wie beispielsweise Thielaviopsis basicola;
Ähren- und Rispenerkrankungen (inklusive Maiskolben), hervorgerufen durch z.B.
Alternaria-Arten, wie beispielsweise Alternaria spp.;
Aspergillus-Arten, wie beispielsweise Aspergillus flavus;
Cladosporium-Arten, wie beispielsweise Cladosporium cladosporioides;
Claviceps-Arten, wie beispielsweise Claviceps purpurea;
Fusarium-Arten, wie beispielsweise Fusarium culmorum;
Gibberella-Arten, wie beispielsweise Gibberella zeae;
Monographella-Arten, wie beispielsweise Monographella nivalis;
Erkrankungen, hervorgerufen durch Brandpilze wie z.B.
Sphacelotheca-Arten, wie beispielsweise Sphacelotheca reiliana;
Tilletia-Arten, wie beispielsweise Tilletia caries;
Urocystis-Arten, wie beispielsweise Urocystis occulta;
Ustilago-Arten, wie beispielsweise Ustilago nuda;
Fruchtfäule hervorgerufen durch z.B.
Aspergillus-Arten, wie beispielsweise Aspergillus flavus;
Botrytis-Arten, wie beispielsweise Botrytis cinerea;
Penicillium-Arten, wie beispielsweise Penicillium expansum und Penicillium purpurogenum;
Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B.
Alternaria-Arten, wie beispielsweise Alternaria brassicicola
Aphanomyces-Arten, wie beispielsweise Aphanomyces euteiches
Ascochyta-Arten, wie beispielsweise Ascochyta lentis
Aspergillus-Arten, wie beispielsweise Aspergillus flavus
Cladosporium-Arten, wie beispielsweise Cladosporium herbarum
Cochliobolus-Arten, wie beispielsweise Cochliobolus sativus
(Konidienform: Drechslera, Bipolaris Syn: Helminthosporium);
Colletotrichum-Arten, wie beispielsweise Colletotrichum coccodes;
Fusarium-Arten, wie beispielsweise Fusarium culmorum;
Gibberella-Arten, wie beispielsweise Gibberella zeae;
Macrophomina-Arten, wie beispielsweise Macrophomina phaseolina
Monographella-Arten, wie beispielsweise Monographella nivalis;
Penicillium-Arten, wie beispielsweise Penicillium expansum
Phoma-Arten, wie beispielsweise Phoma lingam
Phomopsis-Arten, wie beispielsweise Phomopsis sojae;
Phytophthora Arten, wie beispielsweise Phytophthora cactorum;
Pyrenophora-Arten, wie beispielsweise Pyrenophora graminea
Pyricularia-Arten, wie beispielsweise Pyricularia oryzae;
Pythium-Arten, wie beispielsweise Pythium ultimum;
Rhizoctonia-Arten, wie beispielsweise Rhizoctonia solani;
Rhizopus-Arten, wie beispielsweise Rhizopus oryzae
Sclerotium-Arten, wie beispielsweise Sclerotium rolfsii;
Septoria-Arten, wie beispielsweise Septoria nodorum;
Typhula-Arten, wie beispielsweise Typhula incarnata;
Verticillium-Arten, wie beispielsweise Verticillium dahliae
Krebserkrankungen, Gallen und Hexenbesen, hervorgerufen durch z.B.
Nectria-Arten, wie beispielsweise Nectria galligena;
Welkeerkrankungen hervorgerufen durch z.B.
Monilinia-Arten, wie beispielsweise Monilinia laxa;
Deformationen von Blättern, Blüten und Früchten, hervorgerufen durch z.B.
Taphrina-Arten, wie beispielsweise Taphrina deformans;
Degenerationserkrankungen holziger pflanzen, hervorgerufen durch z.B.
Esca-Arten, wie beispielsweise Phaeomoniella chlamydospora und Phaeoacremonium aleophilum und Fomitiporia mediterranea;
Blüten- und Samenerkrankungen, hervorgerufen durch z.B.
Botrytis-Arten, wie beispielsweise Botrytis cinerea;
Erkrankungen von Pflanzenknollen, hervorgerufen durch z.B.
Rhizoctonia-Arten, wie beispielsweise Rhizoctonia solani;
Helminthosporium-Arten, wie beispielsweise Helminthosporium solani;
Erkrankungen, hervorgerufen durch bakterielle Erreger wie z.B.
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;

Bevorzugt können die folgenden Krankheiten von Soja-Bohnen bekämpft werden:
Pilzkrankheiten an Blättern, Stängeln, Schoten und Samen verursacht durch z.B.
Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis'sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi), Scab (Sphaceloma glycines), Stemphylium Leaf Blight (Stemphylium botryosum), Target Spot (Corynespora cassiicola)
Pilzkrankheiten an Wurzeln und der Stängelbasis verursacht durch z.B.
Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraums nach der Behandlung gegen den Befall durch die genannten Schadeneger zu schützen. Der Zeitraum, innerhalb dessen Schutz herbeigeführt wird, erstreckt sich im Allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie z.B. gegen Puccinia-Arten und von Krankheiten im Wein-, Obst- und Gemüseanbau, wie z.B. gegen Botrytis-, Venturia- oder Alternaria-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs-und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispiel-haft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

### Mycotoxine

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkomalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

### Materialschutz

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

### Formulierungen

Die vorliegende Erfindung betrifft ein Mittel zum Bekämpfen unerwünschter Mikroorganismen, umfassend wenigstens eines der erfindungsgemäßen Thiadiazolyloxyphenylamidine.

Die erfindungsgemäßen Thiadiazolyloxyphenylamidine können dazu in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/ oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die zuvor beschriebenen Formulierungen können in einem erfindungsgemäßen Verfahren zum Bekämpfen unerwünschter Mikroorganismen verwendet werden, bei dem die erfindungsgemäßen Thiadiazolyloxyphenylamidine auf die Mikroorganismen und/oder in deren Lebensraum ausgebracht werden.

### Saatgutbehandlung

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt wird, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von phytopathogenen Pilzen, indem das Saatgut mit einem erfindungsgemäßen Mittel behandelt wird.

Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen.

Weiterhin bezieht sich die Erfindung auf Saatgut, welches zum Schutz vor phytopathogenen Pilzen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Mischungen insbesondere auch bei transgenem Saatgut eingesetzt werden können.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffkombinationen können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe oder Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate. Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, be-sonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt der Wirkstoffe in den Formulierungen und nach dem Saatgut. Die Aufwandmengen an Wirkstoffkombination liegen im Allgemeinen zwischen 0,001 und 50 g pro Kilogramm Saatgut, vorzugsweise zwischen 0,01 und 15 g pro Kilogramm Saatgut.

### Mischung mit bekannten Fungiziden, Bakteriziden, Akariziden, Nematiziden oder Insektiziden

Die erfindungsgemäßen Amidine können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Thiadiazolyloxyphenylamidine können daher in nicht-medizinischen Anwendungen eingesetzt werden.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Es kann auch das Saatgut der Pflanzen behandelt werden.

Beim Einsatz der erfindungsgemäßen Thiadiazolyloxyphenylamidine als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im Allgemeinen zwischen 0,1 und 10.000 g/ha, vorzugsweise zwischen 1 und 5.000 g/ha.

### GMOs

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Mitochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Streßfaktoren resistent sind. Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-StreßBedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosate-acetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmem kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil_Crickmore/Bt/ beschrieben sind, zusammengestellt wurden, oder insektizide Teile davon, z.B. Proteine der Cry-Proteinklassen Cry1Ab, CrylAc, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein CrylA.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Emteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alteman produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

### Herstellungsbeispiele

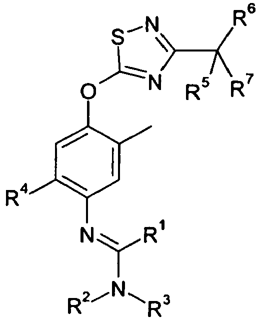

| **Nr** | **R¹** | **R²** | **R³** | **R⁴** | **R⁵** | **R⁶** | **R⁷** |
|---|---|---|---|---|---|---|---|
| 1 | H | Ethyl | CH₃ | Chlor | CH₃ | CH₃ | CH₃ |
| 2 | H | Ethyl | CH₃ | Brom | CH₃ | CH₃ | CH₃ |
| 3 | H | Ethyl | CH₃ | Chlor | 1-Chlorcyclopropyl | | |
| 4 | H | Propan- 2-yl | CH₃ | Chlor | 1-Chlorcyclopropyl | | |
| 5 | H | -(CH₂)₅- | | Chlor | 1-Chlorcyclopropyl | | |
| 6 | H | Ethyl | CH₃ | Chlor | CH₃ | H | 4-Chlorphenyl |
| 7 | H | -(CH₂)₅- | | Chlor | CH₃ | H | 4-Chlorphenyl |
| 8 | H | Propan- 2-yl | CH₃ | Chlor | CH₃ | H | 4-Chlorphenyl |
| 9 | H | Ethyl | CH₃ | Chlor | H | H | 4-Chlorbenzyl |
| 10 | H | -(CH₂)₅- | | Chlor | H | H | 4-Chlorbenzyl |
| 11 | H | Ethyl | CH₃ | Chlor | CH₃ | CH₃ | 4-Chlorphenyl |
| 12 | H | -(CH₂)₅- | | Chlor | CH₃ | CH₃ | 4-Chlorphenyl |
| 13 | H | Propan- 2-yl | CH₃ | Chlor | CH₃ | CH₃ | 4-Chlorphenyl |
| 14 | H | Ethyl | CH₃ | Chlor | H | H | Phenyl |
| 15 | H | Ethyl | CH₃ | Chlor | (E)-2-(4-Fluorphenyl)ethenyl | | |
| 16 | H | -(CH₂)₅- | | Chlor | (E)-2-(4-Fluorphenyl)ethenyl | | |
| 17 | H | Ethyl | CH₃ | Chlor | 1-(4-Chlorphenyl)cyclopropyl | | |
| 18 | H | Ethyl | CH₃ | Chlor | H | H | 4-Chlorphenyl |
| 19 | H | Ethyl | CH₃ | Chlor | H | H | Chlor |
| 20 | H | Propan- 2-yl | CH₃ | Chlor | 1-(4-Chlorphenyl)cyclopropyl | | |
| 21 | H | -(CH₂)₅- | | Chlor | 1-(4-Chlorphenyl)cyclopropyl | | |
| 22 | H | Ethyl | CH₃ | Chlor | H | H | 4-Chlorphenoxy |
| 23 | H | Propan- 2-yl | CH₃ | Chlor | (E)-2-(4-Fluorphenyl)ethenyl | | |
| 24 | H | Propyl | CH₃ | Chlor | CH₃ | CH₃ | CH₃ |
| 25 | H | Prop-2-en-1-yl | CH₃ | Chlor | CH₃ | CH₃ | CH₃ |
| 26 | H | Cyclopropylmethyl | CH₃ | Chlor | CH₃ | CH₃ | CH₃ |
| 27 | H | 3-Methoxy propyl | CH₃ | Chlor | CH₃ | CH₃ | CH₃ |
| 28 | H | Ethyl | CH₃ | Chlor | CH₃ | CH₃ | Benzyl |
| 29 | H | Ethyl | CH₃ | Chlor | (1S,2S)-2-Phenylcyclopropyl | | |
| 30 | H | -(CH₂)₅- | | Chlor | (1S,2S)-2-Phenylcyclopropyl | | |
| 31 | H | Propan-2-yl | CH₃ | Chlor | (1S,2S)-2-Phenylcyclopropyl | | |
| 32 | H | Ethyl | CH₃ | Chlor | 2,2-Dichlor-1-methylcyclopropyl | | |
| 33 | H | Ethyl | CH₃ | Chlor | H | H | 4-Bromphenyl |
| 34 | H | Ethyl | CH₃ | Chlor | H | H | 3-(Trifluormethyl)ph enyl |
| 35 | H | Ethyl | CH₃ | Chlor | H | H | 3-Chlorphenoxy |
| 36 | H | Ethyl | CH₃ | Chlor | H | H | 2-Chlorphenoxy |
| 37 | H | Ethyl | CH₃ | Chlor | H | H | Phenoxy |
| 38 | H | Ethyl | CH₃ | Chlor | H | H | 2,4-Dichlorphenyl |
| 39 | H | Ethyl | CH₃ | Chlor | CH₃ | CH₃ | Chlormethyl |
| 40 | H | Ethyl | CH₃ | Chlor | H | H | 2,4-Difluorphenyl |
| 41 | H | -(CH₂)₅- | | Chlor | H | H | Phenyl |
| 42 | H | Ethyl | CH₃ | Chlor | H | H | 4-[(Trifluormethyl)-sulfanyl]phenyl |
| 43 | H | Ethyl | CH₃ | Chlor | 1,2,3,4-Tetrahydronaphthalen-2-yl | | |
| 44 | H | Propan-2-yl | CH₃ | Chlor | 1,2,3,4-Tetrahydronaphthalen-2-yl | | |
| 45 | H | -(CH₂)₅- | | Chlor | 1,2,3,4-Tetrahydronaphthalen-2-yl | | |
| 46 | H | Ethyl | CH₃ | Chlor | Phenyl | | |
| 47 | H | Ethyl | CH3 | Chlor | 3,4-Difluorphenyl | | |
| 48 | H | Ethyl | CH3 | Chlor | 3-(Trifluormethoxy)phenyl | | |

| **Bsp. Nr.** | **¹H-NMR** |
|---|---|
| 1 | 7,72(b,1H); 7,45(s,1H); 6,98(s,1H); 3,44(q,2H); 3,02(s,3H); 2,12(s,3H); 1,31(s,9H); 1,16(t,3H) |
| 2 | 7,71(b,1H); 7,60(s,1H); 6,98(s,1H); 3,30(q,2H); 2,98(s,3H); 2,12(s,3H); 1,31(s,9H); 1,17(t,3H) |
| 3 | 7,74(s,1H); 7,47(s,1H); 6,99(s,1H); 3,46(m,2H); 2,98(s,3H); 2,14(s,3H); 1,62(m,2H); 1,52(m,2H); 1,16(t,3H) |
| 4* | 7,71(s,1H); 7,32(s,1H); 6,91(s,1H); 3,76(sp,1H); 2,91(s,3H); 2,16(s,3H); 1,65(m,2H); 1,50(m,2H); 1,24(d,6H) |
| 5* | 7,58(s,1H); 7,33(s,1H); 6,92(s,1H); 3,62(m,2H); 3,34(m,2H); 2,16(s,3H); 1,66(m,6H); 1,64(m,2H); 1,50(m,2H) |
| 6 | 7,73(s,1H); 7,43(s,1H); 7,30-7,37(m,4H); 6,97(s,1H); 4,31(q,1H); 3,40(m,2H); 2,97(s,3H); 2,10(s,3H); 1,58(d,3H); 1,15(t,3H) |
| 7 | 7,71(s,1H); 7,44(s,1H); 7,30-7,36(m,4H); 6,98(s,1H); 4,31(q,1H); 3,48(m,4H); 2,10(s,3H); 1,52-1,67(m+d,9H) |
| 8 | 7,78(s,1H); 7,43(s,1H); 7,30-7,36(m,4H); 6,97(s,1H); 4,31(q,1H); 3,85(m,1H); 2,88(s,3H); 2,10(s,3H); 1,58(d,3H); 1,20(d,6H) |
| 9 | 7,73(s,1H); 7,42(s,1H); 7,21-7,30(m,4H); 6,98(s,1H); 3,44(m,2H); 2,91-3,17(m,7H); 2,11 (s,3H); 1,16(t,3H) |
| 10 | 7,71(s,1H); 7,42(s,1H); 7,21-7,32(m,4H); 6,99(s,1H); 3,49(m,4H); 3,02-3,17(m,4H); 2,11(s,3H); 1,53-1,68(m,6H) |
| 11 | 7,73(s,1H); 7,43(s,1H); 7,26-7,34(m,4H); 6,97(s,1H); 3,40(m,2H); 2,97(s,3H); 2,10(s,3H); 1,69(s,6H); 1,15(t,3H) |
| 12 | 7,70(s,1H); 7,43(s,1H); 7,26-7,34(m,4H); 6,98(s,1H); 3,48(m,4H); 2,10(s,3H); 1,68(s,6H); 1,51-1,66(m,6H) |
| 13 | 7,78(s,1H); 7,43(s,1H); 7,25-7,35(m,4H); 6,97(s,1H); 3,85(m,1H); 2,88(s,3H); 2,10(s,3H); 1,69(s,6H); 1,20(d,6H) |
| 14 | 7,73(s,1H); 7,44(s,1H); 7,29(m,4H); 7,22(m,1H); 6,97(s,1H); 4,02(s,2H); 3,39(m,2H); 2,97(s,3H); 2,11(s,3H); 1,15(t,3H) |
| 15 | 7,71-7,77(m+s,3H); 7,60(d,1H); 7,39(s,1H); 7,18-7,23(m,2H); 7,05-7,09(m,2H); 3,41(m,2H); 2,98(s,3H); 2,21(s,3H); 1,16(t,3H) |
| 16 | 7,72-7,78(m+s,3H); 7,60(d,1H); 7,41(s,1H); 7,18-7,23(m,2H); 7,12(s,1H); 7,07(d,1H); 3,50(m,4H); 2,21(s,3H); 1,56-1,69(m,6H) |
| 17 | 7,72(s,1H); 7,44(s,1H); 7,33-7,39(m,4H); 6,97(s,1H); 3,39(m,2H); 2,97(s,3H); 2,12(s,3H); 1,56(m,2H); 1,31(m,2H); 1,15(t,3H) |
| 18 | 7,72(s,1H); 7,44(s,1H); 7,30-7,36(m,4H); 6,97(s,1H); 4,09(s,2H); 3,40(m,2H); 2,97(s,3H); 2,11(s,3H); 1,15(t,3H) |
| 19 | 7,74-1(b,1H); 7,48(s,1H); 7,00(s,1H); 4,71(s,2H); 2,98(s,3H); 2,14(s,3H); 1,16(t,3H) |
| 20 | 7,78(s,1H); 7,43(s,1H); 7,33-7,41(m,4H); 6,97(s,1H); 3,85(m,1H); 2,88(s,3H); 2,12(s,3H); 1,55(m,2H); 1,31(m,2H); 1,20(d,6H) |
| 21 | 7,70(s,1H); 7,43(s,1H); 7,33-7,40(m,4H); 6,98(s,1H); 3,48(m,4H); 2,12(s,3H); 1,51-1,67(m,8H); 1,31(m,2H) |
| 22 | 7,73(b,1H); 7,47(s,1H); 7,31 + 7,03(AA'BB'; 4H); 6,99(s,1H); 5,15(s,2H); 2,98(s,3H); 2,13(s,3H); 1,16(t,3H) |
| 23 | 7,70-7,82(m+s,3H); 7,60(d,1H); 7,50(s,1H); 7,18-7,23(m,2H); 7,07(s,1H); 7,02(s,1H); 3,85(m,1H); 2,90(s,3H); 2,17(s,3H); 1,19(d,6H) |
| 24 | 7,75(b,1H); 7,46(s,1H); 6,98(s,1H); 3,39(t,2H); 3,04(s,3H); 2,13(s,3H); 1,62(m,2H); 1,27(s,9H); 0,87(t,3H) |
| 25 | 7,78(b,1H); 7,46(s,1H); 7,00(s,1H); 5,87(m,1H); 5,22(m,2H); 3,98(m,2H); 2,95(s,3H); 2,14(s,3H); 1,29(s,9H) |
| 26 | 7,76(b,1H); 7,46(s,1H); 6,98(s,1H); 3,32(d,2H); 3,06(s,3H); 2,13(s,3H); 0,81(m,1H); 0,50(m,2H); 0,30(m,2H) |
| 27 | 7,71(b,1H); 7,46(s,1H); 6,97(s,1H); 3,34(m,2H); 3,24(s,3H); 2,12(s,3H); 1,88(m,2H); 1,62(m,2H); 1,31(s,9H) |
| 28 | 7,75(s,1H); 7,45(s,1H); 7,14-7,20(m,3H); 7,01(s,1H); 6,90(m,2H); 3,42(m,2H); 2,98(s,2H); 2,95(s,3H); 2,16(s,3H); 1,29(s,6H); 1,16(t,3H) |
| 29 | 7,74(s,1H); 7,46(s,1H); 7,16-7,30(m,5H); 6,99(s,1H); 3,40(m,2H); 2,97(s,3H); 2,50(m,1H); 2,40(m,1H); 2,15(s,3H); 1,62(m,1H); 1,48(m,1H); 1,16(t,3H) |
| 30 | 7,71(s,1H); 7,46(s,1H); 7,16-7,29(m,5H); 7,00(s,1H); 3,48(m,4H); 2,50(m,1H); 2,39(m,1H); 2,15(s,3H); 1,46-1,68(m,8H) |
| 31 | 7,79(s,1H); 7,45(s,1H); 7,16-7,30(m,5H); 6,99(s,1H); 3,85(m,1H); 2,89(s,3H); 2,50(m,1H); 2,39(m,1H); 2,15(s,3H); 1,62(m,1H); 1,48(m,1H); 1,21(d,6H) |
| 32 | 7,74(s,1H); 7,46(s,1H); 7,00(s,1H); 3,40(m,2H); 2,98(s,3H); 2,48(d,1H); 2,12(s,3H); 1,86(d,1H); 1,70(s,3H); 1,16(t,3H) |
| 33 | 7,73(s,1H); 7,48(d,2H); 7,44(s,1H); 7,25(d,2H); 6,98(s,1H); 4,07(s,2H); 3,40(m,2H); 2,97(s,3H); 2,11(s,3H); 1,16(t,3H) |
| 34 | 7,72(s,1H); 7,65(s,1H); 7,58(m,3H); 7,44(s,1H); 6,97(s,1H); 4,22(s,2H); 3,40(m,2H); 2,97(s,3H); 2,11(s,3H); 1,16(t,3H) |
| 35 | 7,73(b,1H); 7,47(s,1H); 7,32-6,97(m,5H); 5,18(s,2H); 2,98(s,3H); 2,13(s,3H); 1,16(t,3H) |
| 36 | 7,73(b,1H); 7,47(s,1H); 7,42-6,96(m,5H); 5,24(s,2H); 2,98(s,3H); 2,13(s,3H); 1,16(t,3H) |
| 37 | 7,73(b,1H); 7,60-6,98(m,6H); 5,13(s,2H); 2,98(s,3H); 2,13(s,3H); 1,16(t,3H) |
| 38 | 7,73(s,1H); 7,56(d,1H); 7,44(s,1H); 7,40(d,1H); 7,37(dd,1H); 6,97(s,1H); 4,21(s,2H); 3,40(m,2H); 2,97(s,3H); 2,12(s,3H); 1,16(t,3H) |
| 39 | 7,74(s,1H); 7,46(s,1H); 6,99(s,1H); 3,85(s,2H); 3,41(m,2H); 2,98(s,3H); 2,13(s,3H); 1,38(s,6H); 1,16(t,3H) |
| 40 | 7,72(s,1H); 7,45(s,1H); 7,41(m,1H); 7,14(m,1H); 7,02(m,1H); 6,97(s,1H); 4,10(s,2H); 3,40(m,2H); 2,97(t,3H); 2,12(s,3H); 1,15(t,3H) |
| 41 | 7,73(s,1H); 7,64(d,2H); 7,45(d,2H); 7,44(s,1H); 6,98(s,1H); 4,18(s,2H); 3,40(m,2H); 2,97(s,3H); 2,11(s,3H); 1,16(t,3H) |
| 42 | 7,70(s,1H); 7,44(s,1H); 7,29(m,4H); 7,22(m,1H); 6,98(s,1H); 4,08(s,2H); 3,50(m,4H); 2,11(s,3H); 1,64(m,2H); 1,54(m,4H) |
| 43 | 7,74(bs, 1H); 7.45(s, 1H); 7.07-7.16(m, 4H); 6,98(s, 1H); 3,41(m, 2H), 3,00-3,22 (m, 3H); 2,97(s, 3H); 2,84(m, 2H); 2,24(m, 1H); 2.14(s, 3H); 1,90(m, 1H); 1,15(t, 3H) |
| 44 | 7,79(bs, 1H); 7.45(s, 1H); 7.07-7.12(m, 4H); 6,99(s, 1H); 3,80(m, 1H), 3,03-3,22 (m, 3H); 2,88(s, 3H); 2,84(m, 2H); 2,24(m, 1H); 2.14(s, 3H); 1,90(m, 1H); 1,20(d, 6H) |
| 45 | 7,71(s, 1H); 7.46(s, 1H); 7.07-7.11 (m, 4H); 6,99(s, 1H); 3,48(m, 4H), 3,04-3,23 (m, 3H); 2,84(m, 2H); 2,24(m, 1H); 2.14(s, 3H); 1,90(m, 1H); 1,52-1,66(m, 6H) |

Die chemischen NMR-Verschiebungen δ in ppm wurden bei 400 MHZ falls nicht anders angegeben im Lösungsmittel DMSOd₆ mit Tetramethylsilan als internem Standard gemessen.
*: Aufnahme im Lösungsmittel CD₃CN

Folgende Abbkürzungen beschreiben die Signalaufspaltung:
s = Singulett,d = Dublett,t = Triplett,q = Quadruplett,m = Multiplett

### Synthesebeispiele:

### Beispiel 3: N'-(2-Chlor-4-{[3-(1-chlorcyclopropyl)-1,2,4-thiadiazol-5-yl]oxy}-5-methyl-phenyl)-N-ethyl-N-methylimidoformamid

0.63 g (1.86 mmol) 2-Chlor-4-{[3-(1-chlorcyclopropyl)-1,2,4-thiadiazol-5-yl]oxy}-5-methylanilin werden in 5 ml Toluol gelöst und mit 0.50 g (3.71 mmol) N-Ethyl-N-methylformamiddimethylacetal versetzt. Die Reaktionsmischung wird 12h refluxiert, anschliessend abgekühlt, im Vakuum vom Lösungsmittel befreit und säulenchromatographisch aufgereinigt. Man erhält 0.62 g Produkt (82.0 % Reinheit, 71.3 % Ausbeute; log P (pH2.3) =1.62).

### Synthese der Ausgangsverbindungen:

### 2-Chlor-4-{[3-(1-chlorcyclopropyl)-1,2,4-thiadiazol-5-yl]oxy}-5-methylanilin

3.00 g (19.04 mmol) 2-Chlor-4-hydroxy-5-methylanilin werden in 50 ml Acetonitril vorgelegt, bei Raumtemperatur mit 3.16 g (22.84 mmol) Kaliumcarbonat versetzt und und 30 min bei Raumtemperatur gerührt. Anschliessend werden 6.26 g (19.04 mmol) 3-(1-Chlorcyclopropyl)-5-[(4-methylphenyl]sulfonyl]-1,2,4-thiadiazol zugesetzt und die Reaktionsmischung 12 h bei 50°C gerührt. Nach Abkühlen wird einrotiert, der Rückstand in Dichlormethan aufgenommen, zweimal mit Wasser extrahiert, über Na2SO4 getrocknet und im Vakuum vom Lösungsmittel befreit. Der Rückstand wird säulenchromatographisch aufgereinigt. Man erhält 3.18 g Produkt (93.3 % Reinheit, 49.4 % Ausbeute, log P (pH 2.3) =3.50).

### 3-(1-Chlorcyclopropyl)-5-{(4-methylphenyl)sulfonyl}-1,2,4-thiadiazol

66.2 g (372 mmol) 5-(1-Chlorcyclopropyl)-1,3,4-oxathiazol-2-on und 70.9 g (391 mmol) Tosylcyanid werden in 125 ml 1,2-Dichlorbenzol 1h bei 160°C gerührt. Anschliessend wird das Reaktionsgemisch abgekühlt, mit Pentan versetzt, abfiltriert und im Vakuum getrocknet. Man erhält 100.0 g Produkt (99.9% Reinheit, 85.2% Ausbeute, log P (pH 2.3) =3.67).

### 5-(3-Chlorbenzyl)-1,3,4-oxathiazol-2-on

89.1 g (680 mmol) Chlorcarbonylsulfenylchlorid und 71.3 g (400 mmol) (1-Chlorcyclopropyl)-carbonsäureamid werden in 150 ml Toluol 3h bei 80°C gerührt. Anschliessend wir das Lösungsmittel im Vakuum entfernt, mit Ligroin versetzt und der Niederschlag abfiltriert und im Vakuum gerocknet. Man erhält 66.2 g Produkt (97.9% Reinheit, 91.2% Ausbeute).

### Beispiel 10: 2-Chlor-4-({3-[2-(4-chlorphenyl)ethyl]-1,2,4-thiadiazol-5-yl}oxy)-5-methyl-N-[(Z)-piperidin-1-ylmethyliden]anilin

0.30 g (0.79 mmol) 2-Chlor-4-({3-[2-(4-chlorphenyl)ethyl]-1,2,4-thiadiazol-5-yl}oxy)-5-methylanilin werden in 3 ml Toluol und 0.75 ml Methanol gelöst und mit 0.37 g (2.37 mmol) 1-(Dimethoxylmethyl)piperidin versetzt. Die Reaktionsmischung wird 18h refluxiert, anschliessend abgekühlt, im Vakuum vom Lösungsmittel befreit und säulenchromatographisch aufgereinigt. Man erhält 0.13 g Produkt (84.2 % Reinheit, 29.3% Ausbeute; log P (pH2.3) =2.64).

### Synthese der Ausgangsverbindungen:

### 2-Chlor-4-({3-[2-(4-chlorphenyl)ethyl]-1,2,4-thiadiazol-5-yl}oxy)-5-methylanilin

1.45 g (9.24 mmol) 2-Chlor-4-hydroxy-5-methylanilin werden in 18 ml Acetonitril vorgelegt, bei Raumtemperatur mit 1.53 g (11.08 mmol) Kaliumcarbonat versetzt und und 30 min bei Raumtemperatur gerührt. Anschliessend werden 3.50 g (9.24 mmol) 3-[2-(4-Chlorphenyl)ethyl]-5-[(4-methylphenyl]sulfonyl]-1,2,4-thiadiazol zugesetzt und die Reaktionsmischung 12 h bei 50°C gerührt. Nach Abkühlen wird einrotiert, der Rückstand in Dichlormethan aufgenommen, zweimal mit Wasser extrahiert, über Na2S04 getrocknet und im Vakuum vom Lösungsmittel befreit. Man erhält 3.11 g Produkt (94.3% Reinheit, 83.4 % Ausbeute, log P (pH 2.3) =4.50).

### 3-[2-(4-Chlorphenyl)ethyl]-5-[(4-methylphenyl]sulfonyl]-1,2,4-thiadiazol

10.90 g (45.1 mmol) 5-[2-(4-Chlorphenyl)ethyl]-1,3,4-oxathiazol-2-on und 8.58 g (47.4 mmol) Tosylcyanid werden in 11 ml 1,2-Dichlorbenzol 1h bei 160°C gerührt. Anschliessend wird das Reaktionsgemisch abgekühlt, mit 10 ml Pentan versetzt und der Niederschlag abfiltriert und im Vakuum getrocknet. Man erhält 11.06 g Produkt (99.9 % Reinheit, 64.7% Ausbeute; log P (pH2.3) =4.62).

### 5-[2-(4-Chlorphenyl)ethyl]-1,3,4-oxathiazol-2-on

11.60 g (88.5 mmol) Chlorcarbonylsulfenylchlorid und 13.54 g (73.8 mmol) 3-(4-Chlorphenyl)-propionsäureamid werden in 25 ml Toluol 3h bei 70°C gerührt. Anschliessend wird abgekühlt, im Vakuum vom Lösungsmittel befreit und säulenchromatographisch aufgereinigt. Man erhält 15.6 g Produkt (99.9% Reinheit, 87.6 % Ausbeute, log P (pH2.3) =3.36).

### Biologische Beispiele

### Beispiel 1: Alternaria-Test (Tomate) / protektiv

| | |
|---|---|
| Lösungsmittel : | 24,5 Gewichtsteile Aceton |
| | 24,5 Gewichtsteile Dimethylacetamid |
| Emulgator : | 1 Gewichtsteil Alkyl-Aryl-Polyglykolether(Polyoxyethylen(16)tri-stearylphenylether) |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Alternaria solani*** inokuliert. Die Pflanzen werden dann in einer Inkubationskabine bei ca. 20°C und 100% relativer Luftfeuchtigkeit aufgestellt.

3 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen **1, 2, 11, 12, 13, 14, 15, 17, 24, 28, 32, 33, 39, 40, 43, 44, 47 und 48** bei einer Konzentration an Wirkstoff von 100ppm einen Wirkungsgrad von 70% oder mehr.

### Beispiel 2: Uromyces - Test (Bohne) / protektiv

| | |
|---|---|
| Lösungsmittel : | 24,5 Gewichtsteile Aceton |
| | 24,5 Gewichtsteile Dimethylacetamid |
| Emulgator : | 1 Gewichtsteil Alkyl-Aryl-Polyglykolether(Polyoxyethylen(16)tri-stearylphenylether) |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension des Bohnenrosterregers ***Uromyces appendiculatus*** inokuliert und verbleiben dann 1 Tag bei ca. 20°C und 100% relativer Luftfeuchtigkeit in einer Inkubations-kabine.

Die Pflanzen werden dann im Gewächshaus bei ca. 21°C und einer relativen Luftfeuchtigkeit von ca. 90% aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen **1, 2, 6, 8, 14, 15, 18, 17, 32, 38, 39, 43, 44 und 47** bei einer Konzentration an Wirkstoff von 100ppm einen Wirkungsgrad von 70% oder mehr.

### Beispiel 3: Sphaerotheca-Test (Gurke) / protektiv

| | |
|---|---|
| Lösungsmittel: | 49 Gewichtsteile N, N - Dimethylformamid |
| Emulgator: | 1 Gewichtsteil Alkylarylpolyglykolether (Polyoxyethylen(16)tri-stearylphenylether) |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von ***Sphaerotheca fuliginea*** inokuliert. Anschließend werden die Pflanzen in einem Gewächshaus bei 70 % relativer Luftfeuchtigkeit und einer Temperatur von 23□C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die erfindungsgemäßen Verbindungen **1, 2, 3, 4, 5, 6, 7, 8, 9, 11, 12, 13, 14, 15, 16, 17, 18, 20, 21, 22, 23, 24, 25, 26, 28, 29, 30, 31, 32, 33, 34, 35, 36, 37, 38, 39, 40, 41, 42, 43, 44, 45, 47 und 48** bei einer Konzentration an Wirkstoff von 500ppm einen Wirkungsgrad von 70% oder mehr.

### Beispiel 4: Puccinia triticina -Test (Weizen) / protektiv

| | |
|---|---|
| Lösungsmittel: | 50 Gewichtsteile N,N-Dimethylacetamid |
| Emulgator: | 1 Gewichtsteil Alkylarylpolyglykolether(Polyoxyethylen(16)tri-stearylphenylether) |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit Sporen mit einer Sporensuspension von ***Puccinia triticina*** besprüht. Die Pflanzen verbleiben 48 Stunden bei 20°C und 100 % relativer Luftfeuchtigkeit in einer Inkubationskabine. Die Pflanzen werden in einem Gewächshaus bei einer Temperatur von ca. 20°C und einer relativen Luftfeuchtigkeit von ca. 80 % aufgestellt.

8 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen Verbindungen **1, 2, 3, 4, 5, 6, 9, 11, 13, 14, 15, 17, 18, 20, 24, 25, 26, 28, 29, 31, 32, 33, 34, 35, 38, 39, 40, 47 und 48** bei einer Konzentration an Wirkstoff von 500 ppm einen Wirkungsgrad von 70% oder mehr.

### Beispiel 5: Puccinia triticina -Test (Weizen) / kurativ

| | |
|---|---|
| Lösungsmittel: | 50 Gewichtsteile N,N-Dimethylacetamid |
| Emulgator: | 1 Gewichtsteil Alkylarylpolyglykolether (Polyoxyethylen(16)tri-stearylphenylether) |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer Sporensuspension von ***Puccinia triticina*** besprüht. Die Pflanzen verbleiben 48 Stunden bei 20°C und 100 % relativer Luftfeuchtigkeit in einer Inkubationskabine und werden dann mit der Wirkstoffzubereitung in der angegebenen Aufwandmenge besprüht. Die Pflanzen werden in einem Gewächshaus bei einer Temperatur von ca. 20°C und einer relativen Luftfeuchtigkeit von ca. 80 % aufgestellt.

8 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen Verbindungen **1, 2, 3, 4, 5, 6, 9, 11, 13, 14, 15, 17, 18, 19, 20, 24, 25, 26, 29, 31, 32, 33, 34, 35, 36, 38, 39, 40, 47 und 48** bei einer Konzentration an Wirkstoff von 500 ppm einen Wirkungsgrad von 70% oder mehr.

### Beispiel 6: Phakopsora test (Soja) / protectiv

| | |
|---|---|
| Lösungsmittel: | 28,5 Gewichtsteile Aceton |
| Emulgator: | 1,5 Gewichtsteile Polyoxyethylenalkylphenylether(Polyoxyethylen(16)-tristearylphenylether) |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Einen Tag nach dem Besprühen werden die Pflanzen mit einer wässrigen Sporensuspension von *Phakopsora pachyrhizi* besprüht. Die Pflanzen verbleiben bei 20°C und 80 % relativer Luftfeuchtigkeit im Gewächshaus.

11 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

In diesem Test zeigen die erfindungsgemäßen Verbindungen **1, 2, 14, 32 und 47** bei einer Konzentration an Wirkstoff von 100 ppm einen Wirkungsgrad von 80% oder mehr.

### Beispiel 6: Phaedon-Test (PHAECO Spritzbehandlung)

| | |
|---|---|
| Lösungsmittel: | 78,0 GewichtsteileAceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (Brassica pekinensis) werden mit einer Wirkstoffzubereitung der gewünschten Konzentration gespritzt und nach dem Abtrocknen mit Larven des Meerrettichblattkäfers (Phaedon cochleariae) besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von ≥ 80 % bei einer Aufwandmenge von 500 g/ha :
Bsp Nr: **3 und 8.**

## Patentansprüche

1. Thiadiazolyloxyphenylamidine der Formel (I) 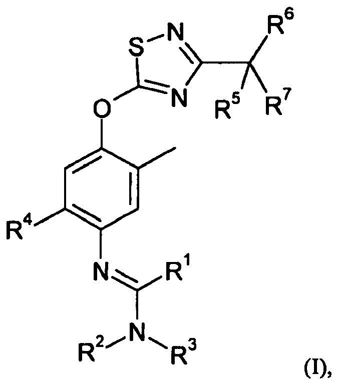 in welcher
R¹ ausgewählt ist aus Wasserstoff; linearen, verzweigten C₁₋₁₂-Alkyl-, C₂₋₁₂-Alkenyl-, C₂₋₁₂-Alkinyl- oder cyclischen C₃₋₈-Alkyl-, C₄₋₈-Alkenyl-, C₄₋₈-Alkinyl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus-R', -X, -OR', -SR', -NR'₂, -SiR'₃, -COOR', -CN und -CONR₂' substituiert sein können, wobei R' Wasserstoff oder eine C₁₋₁₂-Alkyl-Gruppe ist; -SH; -SR", wobei R" eine C₁₋₁₂-Alkyl-Gruppe ist, die mit einer oder mehreren Gruppen, die ausgewählt sind aus-R', -X, -OR', -SR', -NR'₂, -SiR'₃, -COOR', -CN und-CONR₂', substituiert sein kann, worin R' die obige Bedeutung hat;
R² ausgewählt ist aus linearen, verzweigten C₁₋₁₂-Alkyl-, C₂₋₁₂-Alkenyl-, C₂₋₁₂-Alkinyl-, cyclischen C₃₋₈-Alkyl-, C₄₋₈-Alkenyl-, C₄₋₈-Alkinyl- oder C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', -X, -OR', -SR', -NR'₂, -SiR'₃, -COOR', -CN und -CONR₂', substituiert sein können, wobei R' die obigen Bedeutungen hat;
R³ ausgewählt ist aus -CN, -SH, -SR", -OR", -(C=O)-R", wobei R" die obigen Bedeutungen hat; linearen, verzweigten C₁₋₁₂-Alkyl-, C₂₋₁₂-Alkenyl-, C₂₋₁₂-Alkinyl-, cyclischen C₃₋₈-Alkyl-, C₄₋₈-Alkenyl-, C₄₋₈-Alkinyl- oder C₅₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', -X, -OR', -SR', -NR'₂, -SiR'₃, -COOR', -CN und -CONR₂', substituiert sein können, wobei R' die obigen Bedeutungen hat;
oder in der
R² und R³,
R² und R¹ oder
R¹ und R³ gemeinsam mit den Atomen, an die sie gebunden sind oder mit weiteren Atomen, ausgewählt aus N, O, P und S, einen vier- bis siebengliedrigen Ring bilden können, der mit R'-, OR'-, SR'-, NR'₂-, SiR'₃-Gruppen substituiert sein kann, wobei R' die obigen Bedeutungen hat;
R⁴ ausgewählt ist aus der Gruppe bestehend aus Halogen (X-) und -CN;
R⁵, R⁶ und R⁷ unabhängig voneinander ausgewählt sind aus Wasserstoff, linearen, verzweigten C₁₋₁₂-Alkyl-, C₂₋₁₂-Alkenyl-, C₂₋₁₂-Alkinyl-, cyclischen C₃₋₈-Alkyl-, C₄₋₈-Alkenyl-, C₄₋₈-Alkinyl-Gruppen oder C₅₋₁₈-Aryl-, C₇₋₁₉-Aralkyl oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', Halogen- (-X), Alkoxy- (-OR'), Thioether- oder Mercapto- (-SR'), Amino-(-NR'₂), Silyl- (-SiR'₃), Carboxyl- (-COOR'), Cyan- (-CN) und Amid-Gruppen (-CONR₂'), substituiert sein können, wobei R' die obigen Bedeutungen hat;
oder
R⁵ und R⁶, R⁶ und R⁷ oder R⁵ und R⁷ gemeinsam mit dem C-Atom, an das sie gebunden sind oder mit weiteren Atomen, ausgewählt aus N, O, P und S, einen drei- bis siebengliedrigen Ring bilden können, der mit X-, R'-, OR'-, SR'-, NR'₂-, SiR'₃- C₅₋₁₈-Aryl-, C₇₋₁₉ Aralkyl oder C₇₋₁₉-Alkaryl-Gruppen substituiert sein kann, wobei R' die obigen Bedeutungen hat und die Aryl-, Aralkyl oder Alkaryl-Guppen imRing mit X-, R'-, OR'-, SR'-Gruppen substituiert sein können;
oder
R⁵ und R⁶, R⁶ und R⁷ oder R⁵ und R⁷ gemeinsam mit dem C-Atom, an das sie gebunden sind, eine Doppelbindung bilden können, die mit -CN, -SH, -SR", -OR", -(C=O)-R", wobei R" die obigen Bedeutungen hat; linearen, verzweigten C₁₋₁₂-Alkyl-, C₂₋₁₂-Alkenyl-, C₂₋₁₂-Alkinyl-, cyclischen C₃₋₈-Alkyl-, C₄₋₈-Alkenyl-, C₄₋₈-Alkinyl- oder C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', -X, -OR', -SR', -NR'₂, -SiR'₃, -COOR', -CN und -CONR₂', substituiert sein können, wobei R' die obigen Bedeutungen hat;
und deren Salze, ausgenommen die Verbindungen, in welchen R¹ gleich H, R² und R³ gleich Methyl, R⁴ gleich Cl und R⁵, R⁶, R⁷ gleich Methyl ist oder
R¹ gleich H, R² gleich Methyl, R³ gleich Ethyl, R⁴ gleich Cl und R⁵, R⁶, R⁷ gleich Methyl ist oder R¹ gleich H, R² und R³ gleich (CH₂)₄, R⁴ gleich Cl und R⁵, R⁶, R⁷ gleich Methyl ist oder
R¹ gleich H, R² und R³ gleich (CH₂)₅, R⁴ gleich Cl und R¹, R⁶, R⁷ gleich Methyl ist.

2. Thiadiazolyloxyphenylamidine der Formel (I), gemäß Anspruch 1, wobei
R¹ ausgewählt ist aus der Gruppe bestehend aus Wasserstoff, einer Mercapto-Gruppe (-SH) oder C₁₋₈-Alkyl-Gruppen;
R² ausgewählt ist aus linearen oder verzweigten C₁₋₈-Alkyl-Gruppen;
R³ ausgewählt ist aus linearen, verzweigten und alicyclischen C₁₋₈-Alkyl. Gruppen; oder in welcher
R² und R³ gemeinsam mit dem N-Atom, an das sie gebunden sind oder mit weiteren Atomen, die ausgewählt sind aus N und O, einen fünf- bis sechsgliedrigen Ring bilden können, der mit einer oder mehreren C₁₋₁₂-Alkyl-Gruppen substituiert sein kann;
R⁴ ausgewählt ist aus der Gruppe bestehend aus F, Cl, Br und I;
R⁵ und R⁶ unabhängig voneinander ausgewählt sind aus Wasserstoff, linearen C₁₋₈-Alkyl-Gruppen;
R⁷ ausgewählt ist aus der Gruppe bestehend aus Wasserstoff, linearen, verzweigten, alicyclischen oder heterocyclischen C₁₋₁₂-Alkyl-Gruppen, Halogenatomen, C₁₋₄-Haloalkyl-Gruppen, Phenyl- und Benzyl-Gruppen, wobei die Phenyl- oder Benzyl-Gruppen mit einem oder mehrerern Atomen, ausgewählt aus -R', -X, -OR', -SR', -NR'₂, -SiR'₃, -COOR', -CN und -CONR₂' substituiert sein können;
oder
R⁵ und R⁶ gemeinsam mit dem C-Atom, an das sie gebunden sind oder mit weiteren Atomen, ausgewählt aus N, O, P und S, einen drei- vier- oder fünfgliedrigen Ring bilden können, der mit X-, R'- oder Phenyl- Gruppen substituiert sein kann, wobei R' die obigen Bedeutungen hat und
R⁷ ausgewählt ist aus Wasserstoff und Halogenatomen, Phenyl- Phenoxy- und Benzyl-Gruppen, wobei die zuvor genannten Gruppen durch ein, zwei oder mehrere Halogenatome substitiert sein können;
oder
R⁵ und R⁶ gemeinsam mit dem C-Atom, an das sie gebunden sind, eine Doppelbindung bilden können, die mit Wasserstoff, linearen, verzweigten C₁₋₁₂-Alkyl-Gruppen oder cyclischen C₃₋₈-Alkyl-Gruppen, Halogenatomen, C₁₋₄-Haloalkyl-Gruppen, Phenyl- und Benzyl-Gruppen substituiert sein kann, wobei die zuvor genannten Phenyl- oder Benzyl-Gruppen mit einem oder mehreren Atomen, ausgewählt aus -R', -X, -OR', -SR', -NR'₂, -SiR'₃, -COOR', -CN und -CONR₂' substituiert sein können;
R⁷ Wasserstoff oder C₁₋₄-Alkyl ist;
und deren Salze.

3. Thiadiazolyloxyphenylamidine der Formel (I), gemäß einem der Ansprüche 1 oder 2, in welcher
R¹ ausgewählt ist aus der Gruppe bestehend aus Wasserstoff, Mercapto und Methyl;
R² ausgewählt ist aus der Gruppe bestehend aus Methyl und Ethyl;
R³ ausgewählt ist aus der Gruppe bestehend aus Methyl, Ethyl und Isopropyl;
oder wobei
R² und R³ gemeinsam mit dem N-Atom, an das sie gebunden sind, einen Piperidyl-, Pyrrolidyl- oder 2,6-Dimethylmorpholinyl-Rest bilden;
R⁴ ausgewählt ist aus der Gruppe bestehend aus Cl und Br;
R⁵ und R⁶ unabhängig voneinander ausgewählt sind aus Wasserstoff, Methyl- und Ethyl-Gruppen oder gemeinsam mit dem C-Atom, an dass sie gebunden sind, einen Cyclopropylring bilden können, der mit einem oder zwei Chlor- oder Bromatomen oder mit einer Phenyl- oder Halophenyl-Gruppe substituiert sein kann und
R⁷ ausgewählt ist aus der Gruppe bestehend aus Methyl-, Methoxy-, Ethoxy-, Trimethylsilyl- Triethylsilyl-, Phenyl-, Benzyl-, 4-Chlorbenzyl-, 4-Chlorphenyl-, 4-Bromphenyl- 2,4-Dichlorphenyl-, 2-Chlorphenoxy-, 3-Chlorphenoxy, 4-Chlorphenoxy und 3-Trifluormethyl-Gruppen
oder
R⁵ und R⁶ gemeinsam mit dem C-Atom, an das sie gebunden sind, eine Doppelbindung bilden können, die mit einer 4-Fluorphenyl-Gruppe substituiert ist und
R⁷ Wasserstoff oder C₁₋₄-Alkyl ist;
und deren Salze.

4. Thiadiazolyloxyphenylamidine der Formel (I), gemäß einem der Ansprüche 1 bis 3, in welcher R⁷ eine Phenyl-oder Benzyl-Gruppe ist, die jeweils in 4-Position mit Chlor oder Brom substituiert ist.

5. Thiadiazolyloxyphenylamidin gemäß einem der Ansprüche 1 bis 4 ausgewählt aus der Gruppe bestehend aus N'-{4-[(3-tert-Butyl-1,2,4-thiadiazol-5-yl)oxy]-2-chlor-5-methylphenyl}-N-ethyl-N-methylimidoformamid (Beispiel 1); N'-{2-Brom-4-[(3-tert-butyl-1,2,4-thiadiazol-5-yl)oxy]-5-methylphenyl}-N-ethyl-N-methylimidoformamid (Beispiel 2); N'-(2-Chlor-4-{[3-(1-chlorcyclopropyl)-1,2,4-thiadiazol-5-yl]oxy}-5-methylphenyl)-N-ethyl-N-methylimidoformamid (Beispiel 3); N'-(2-Chlor-4-{[3-(1-chlorcyclopropyl)-1,2,4-thiadiazol-5-yl]oxy}-5-methylphenyl)-N-methyl-N-propan-2-ylimidoformamid (Beispiel 4); 2-Chlor-4-{[3-(1-chlorcyclopropyl)-1,2,4-thiadiazol-5-yl]oxy}-5-methyl-N-[(E)-piperidin-1-ylmethyliden]anilin (Beispiel 5); N'-[2-Chlor-4-({3-[1-(4-chlorphenyl)ethyl]-1,2,4-thiadiazol-5-yl}oxy)-5-methylphenyl]-N-ethyl-N-methylimidoformamid (Beispiel 6); 2-Chlor-4-({3-[1-(4-chlorphenyl)ethyl]-1,2,4-thiadiazol-5-yl}oxy)-5-methyl-N-[(E)-piperidin-1-ylmethyliden]anilin (Beispiel 7); N'-[2-Chlor-4-({3-[1-(4-chlorphenyl)ethyl]-1,2,4-thiadiazol-5-yl}oxy)-5-methylphenyl]-N-methyl-N-propan-2-ylimidoformamid (Beispiel 8); N'-[2-Chlor-4-({3-[2-(4-chlorphenyl)ethyl]-1,2,4-thiadiazol-5-yl}oxy)-5-methylphenyl]-N-ethyl-N-methylimidoformamid (Beispiel 9); 2-Chlor-4-({3-[2-(4-chlorphenyl)ethyl]-1,2,4-thiadiazol-5-yl}oxy)-5-methyl-N-[(Z)-piperidin-1-ylmethyliden]anilin (Beispiel 10); N'-[2-Chlor-4-({3-[2-(4-chlorphenyl)propan-2-yl]-1,2,4-thiadiazol-5-yl}oxy)-S-methylphenyl]-N-ethyl-N-methylimidoformamid (Beispiel 11); 2-Chlor-4-({3-[2-(4-chlorphenyl)propan-2-yl]-1,2,4-thiadiazol-5-yl)oxy)-5-methyl-N-[(Z)-piperidin-1-ylmethyliden]anilin (Beispiel 12); N'-[2-Chlor-4-({3-[2-(4-chlorphenyl)propan-2-yl]-1,2,4-thiadiazol-5-yl}oxy)-5-methylphenyl]-N-methyl-N-propan-2-ylimidoformamid (Beispiel 13); N'-{4-[(3-Benzyl-1,2,4-thiadiazol-5-yl)oxy]-2-chlor-5-methylphenyl}-N-ethyl-N-methylimidoformamid (Beispiel 14); N'-[2-Chlor-4-({3-[(E)-2-(4-fluorphenyl)ethenyl]-1,2,4-thiadiazol-5-yl}oxy)-5-methylphenyl]-N-ethyl-N-methylimidoformamid (Beispiel 15); 2-Chlor-4-({3-[(E)-2-(4-fluorphenyl)ethenyl]-1,2,4-thiadiazol-5-yl}oxy)-5-methyl-N-[(Z)-piperidin-1-ylmethyliden]anilin (Beispiel 16); N'-[2-Chlor-4-({3-[1-(4-chlorphenyl)cyclopropyl]-1,2,4-thiadiazol-5-yl}oxy)-5-methylphenyl]-N-ethyl-N-methylimidoformamid (Beispiel 17); N'-(2-Chlor-4-{[3-(4-chlorbenzyl)-1,2,4-thiadiazol-5-yl]oxy}-5-methylphenyl)-N-ethyl-N-methylimidoformamid (Beispiel 18); N'-(2-Chlor-4-{[3-(chlormethyl)-1,2,4-thiadiazol-5-yl]oxy}-5-methylphenyl)-N-ethyl-N-methylimidoformamid (Beispiel 19); N'-[2-Chlor-4-({3-[1-(4-chlorphenyl)cyclopropyl]-1,2,4-thiadiazol-5-yl}oxy)-5-methylphenyl]-N-methyl-N-propan-2-ylimidoformamid (Beispiel 20); 2-Chlor-4-({3-[1-(4-chlorphenyl)cyclopropyl]-1,2,4-thiadiazol-S-yl}oxy)-5-methyl-N-[(Z)-piperidin-1-ylmethyliden]anilin (Beispiel 21); N'-[2-Chlor-4-({3-[(4-chlorphenoxy)methyl]-1,2,4-thiadiazol-5-yl}oxy)-5-methylphenyl]-N-ethyl-N-methylimidoformamid (Beispiel 22); N'-(2-Chlor-4-({3-[(E)-2-(4-fluorphenyl)ethenyl]-1,2,4-thiadiazol-5-yl}oxy)-5-methylphenyl]-N-methyl-N-propan-2-ylimidoformamid (Beispiel 23); N'-{4-[(3-tert-Butyl-1,2,4-thiadiazol-5-yl)oxy]-2-chlor-5-methylphenyl}-N-methyl-N-propylimidoformamid (Beispiel 24); N'-{4-[(3-tert-Butyl-1,2,4-thiadiazol-5-yl)oxy]-2-chlor-5-methylphenyl}-N-methyl-N-prop-2-en-1-ylimidoformamid (Beispiel 25); N'-{4-[(3-tert-Butyl-1,2,4-thiadiazol-5-yl)oxy]-2-chlor-5-methylphenyl}-N-(cyclopropylmethyl)-N-methylimidoformamid (Beispiel 26); N'-{4-[(3-tert-Butyl-1,2,4-thiadiazol-5-yl)oxy]-2-chlor-5-methylphenyl}-N-(3-methoxypropyl)-N-methylimidoformamid (Beispiel 27); N'-(2-Chlor-5-methyl-4-{[3-(2-methyl-1-phenylpropan-2-yl)-1,2,4-thiadiazol-5-yl]oxy}phenyl)-N-ethyl-N-methylimidoformamid (Beispiel 28); rel-N'-[2-Chlor-5-methyl-4-({3-[(1R,2R)-2-phenylcyclopropyl]-1,2,4-thiadiazol-5-yl}oxy)phenyl]-N-ethyl-N-methylimidoformamid (Beispiel 29); rel-2-Chlor-5-methyl-4-({3-[(1R,2R)-2-phenylcyclopropyl]-1,2,4-thiadiazol-5-yl}oxy)-N-[(E)-piperidin-1-ylmethyliden]anilin (Beispiel 30); rel-N'-[2-Chlor-5-methyl-4-({3-[(1R,2R)-2-phenylcyclopropyl]-1,2,4-thiadiazol-5-yl}oxy)phenyl]-N-methyl-N-propan-2-ylimidoformamid (Beispiel 31); N'-(2-Chlor-4-{[3-(2,2-dichlor-1-methylcyclopropyl)-1,2,4-thiadiazol-5-yl]oxy}-5-methylphenyl)-N-ethyl-N-methylimidoformamid (Beispiel 32); N'-(4-{[3-(4-Brombenzyl)-1,2,4-thiadiazol-5-yl]oxy}-2-chlor-5-methylphenyl)-N-ethyl-N-methylimidoformamid (Beispiel 33); N'-[2-Chlor-5-methyl-4-({3-[3-(trifluormethyl)benzyl]-1,2,4-thiadiazol-5-yl}oxy)phenyl]-N-ethyl-N-methylimidoformamid (Beispiel 34); N'-[2-Chlor-4-({3-[(3-chlorphenoxy)methyl]-1,2,4-thiadiazol-5-yl}oxy)-5-methylphenyl]-N-ethyl-N-methylimidoformamid (Beispiel 35); N'-[2-Chlor-4-({3-[(2-chlorphenoxy)methyl]-1,2,4-thiadiazol-5-yl}oxy)-5-methylphenyl]-N-ethyl-N-methylimidoformamid (Beispiel 36); N'-(2-Chlor-5-methyl-4-{[3-(phenoxymethyl)-1,2,4-thiadiazol-5-yl]oxy}phenyl)-N-ethyl-N-methylimidoformamid (Beispiel 37); N'-(2-Chlor-4-{[3-(2,4-dichlorbenzyl)-1,2,4-thiadiazol-5-yl]oxy}-5-methylphenyl)-N-ethyl-N-methylimidoformamid (Beispiel 38); N'-(2-Chlor-4-{[3-(1-chlor-2-methylpropan-2-yl)-1,2,4-thiadiazol-5-yl]oxy}-5-methylphenyl)-N-ethyl-N-methylimidoformamid (Beispiel 39); N'-(2-Chlor-4-{[3-(2,4-difluorbenzyl)-1,2,4-thiadiazol-5-yl]oxy}-5-methylphenyl)-N-ethyl-N-methylimidoformamid (Beispiel 40).

6. Verfahren zum Herstellen der Thiadiazolyloxyphenylamidine gemäß einem der Ansprüche 1 bis 5 umfassend wenigstens einen der folgenden Schritte (a) bis (j):
(a) Umsetzung von Nitrobenzolderivaten der Formel (III) mit einem Thiadiazolyl-Alkohol der Formel (II) gemäß dem nachfolgenden Reaktionsschema: 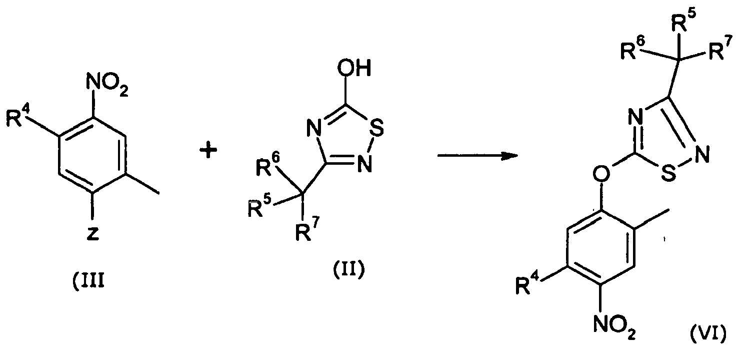
(b) Umsetzung von Nitrophenolderivaten der Formel (V) mit Thiadiazolyl-Derivaten der Formel (IV) gemäß dem nachfolgenden Reaktionsschema: 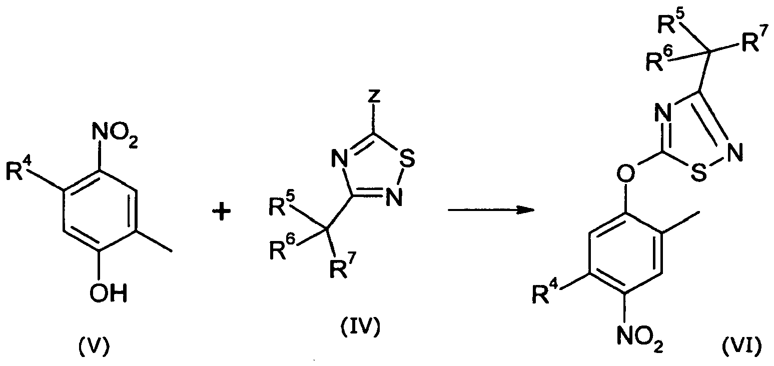
(c) Umsetzung von Anilinen der Formel (VII) mit einem Thiadiazolyl-Alkohol gemäß Formel (II) gemäß dem nachfolgenden Reaktionsschema: 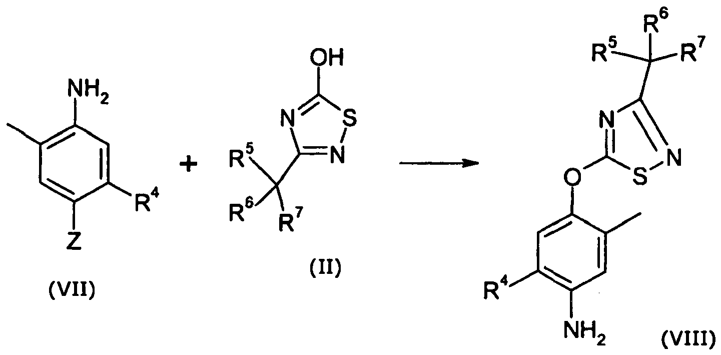
(d) Umsetzung von Aminophenolen der Formel (XII) mit Thiadiazolyl-Derivaten der Formel (IV) gemäß dem nachfolgenden Reaktionsschema: 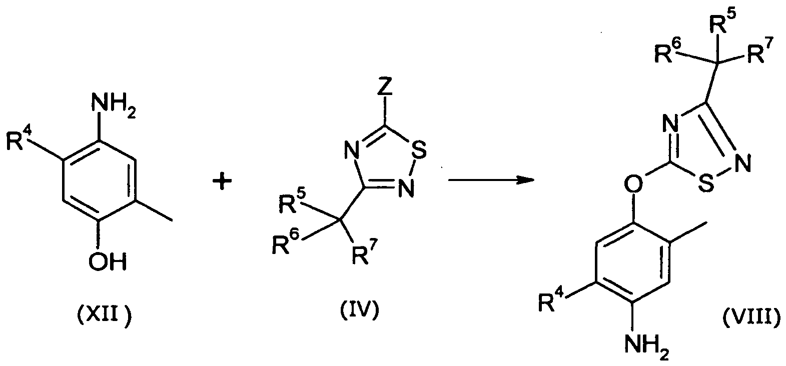
(e) Reduktion der Nitrophenoxyether der Formel (VI) zu Anilinethern der Formel (VIII) gemäß dem nachfolgenden Reaktionsschema: 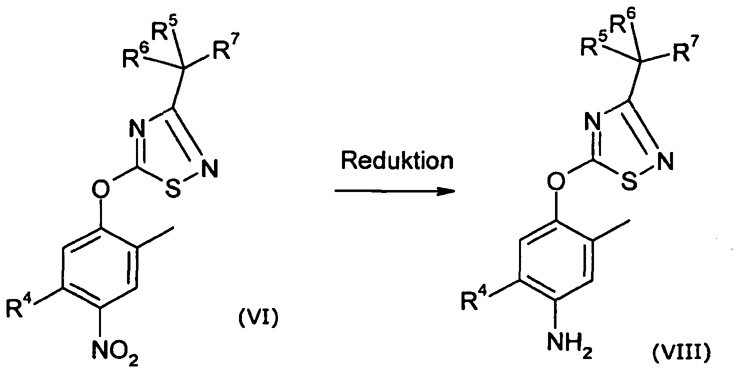
(f) Umsetzung der Anilinether der Formel (VIII) mit
(i) Aminoacetalen der Formel (XIII) oder
(ii) mit Amiden der Formel (XIV) oder
(iii) mit Aminen der Formel (XV) in Gegenwart von Orthoestern der Formel (XVI)
gemäß dem nachfolgenden Reaktionsschema: 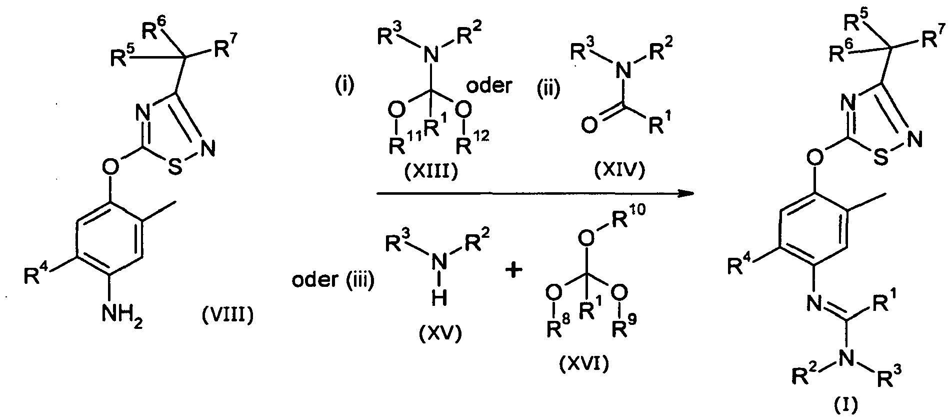
(g) Umsetzung der Aminophenole der Formel (XII) mit
(i) Aminoacetalen der Formel (XIII) oder
(ii) mit Amiden der Formel (XIV) oder
(iii) mit Aminen der Formel (XV) in Gegenwart von Orthoestern der Formel (XVI)
gemäß dem nachfolgenden Reaktionsschema: 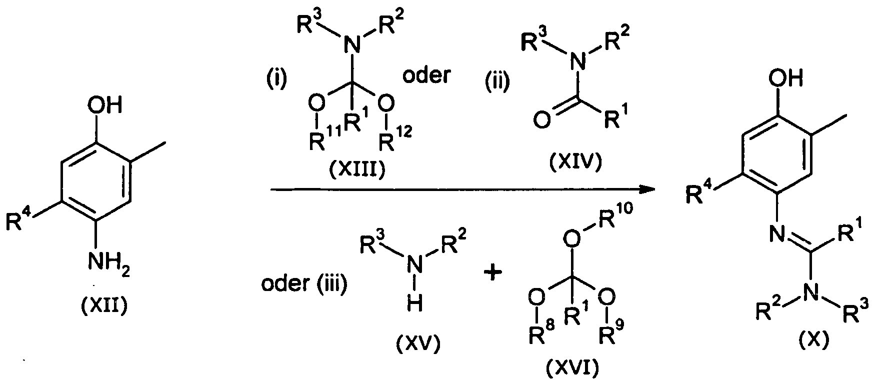
(h) Umsetzung der Aminophenole der Formel (VII) mit
(i) Aminoacetalen der Formel (XIII) oder
(ii) mit Amiden der Formel (XIV) oder
(iii) mit Aminen der Formel (XV) in Gegenwart von Orthoestern der Formel (XVI)
gemäß dem nachfolgenden Reaktionsschema: 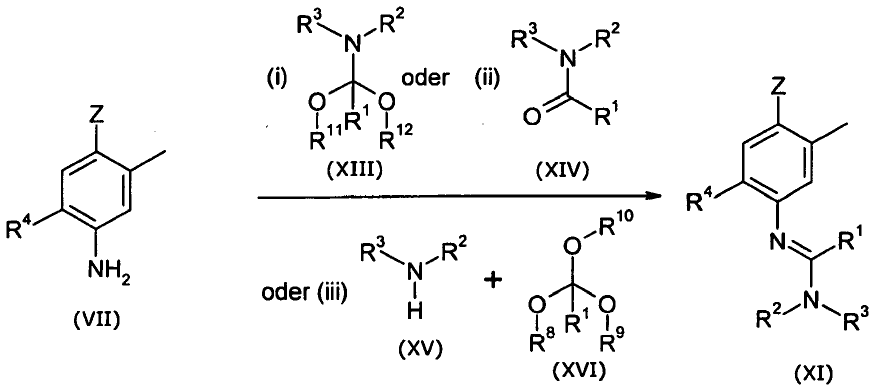
(i) Umsetzung von Amidinen der Formel (XI) mit einem Thiadiazolylalkohol der Formel (II) gemäß dem nachfolgenden Reaktionsschema: 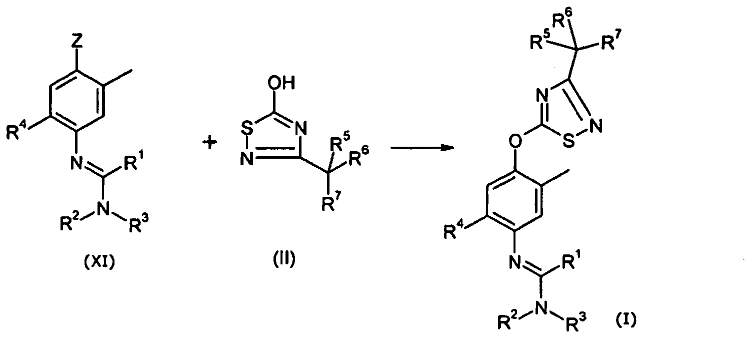
(j) Umsetzung von Amidinen der Formel (XI) mit Thiadiazolyl-Derivaten der Formel (IV) gemäß dem nachfolgenden Reaktionsschema: 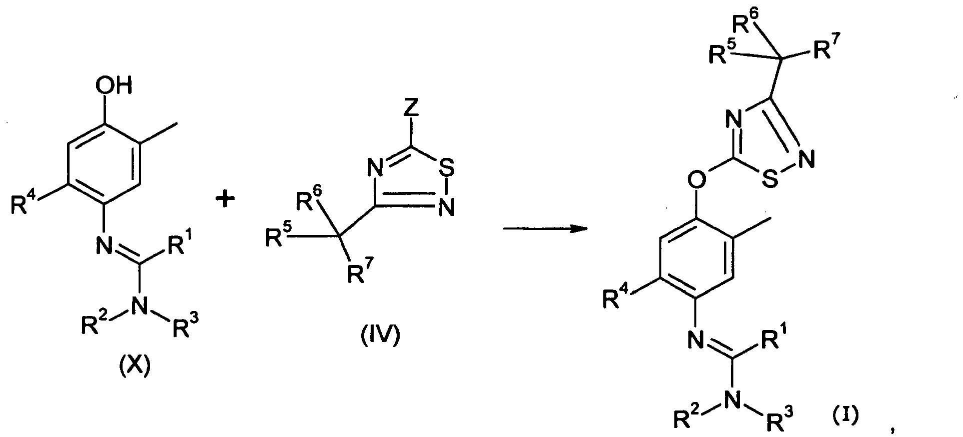 wobei in den obigen Schemata
Z eine Austrittsgruppe ist;
R¹ bis R⁷ die obigen Bedeutungen haben;
und
R⁸ bis R¹⁰ unabhängig voneinander ausgewählt sind aus der Gruppe bestehend aus Wasserstoff, C₁₋₁₂-Alkyl-, C₂₋₁₂-Alkenyl-, C₂₋₁₂-Alkinyl- oder C₅₋₁₈-Aryl-oder C₇₋₁₉-Arylalkyl-, C₇₋₁₉-Alkylaryl-Gruppen und jeweils R⁸ mit R⁹, R⁸ mit R¹⁰ oder R⁹ mit R¹⁰ gemeinsam mit den Atomen, an die sie gebunden sind und gegebenenfalls mit weiteren C-, N-, O- oder S-Atomen einen fünf-, sechs- oder siebengliedrigen Ring bilden können;
R¹¹ und R¹² unabhängig voneinander ausgewählt sind aus der Gruppe bestehend aus Wasserstoff, C₁₋₁₂-Alkyl-, C₂₋₁₂-Alkenyl- C₂₋₁₂-Alkinyl- oder C₅₋₁₈-Aryl- oder C₇₋₁₉-Arylalkyl-Gruppen und gemeinsam mit den Atomen, an die sie gebunden sind, einen fünf-, sechs- oder siebengliedrigen Ring bilden können.

7. Thiadiazolyl-Derivate der Formel (IV) 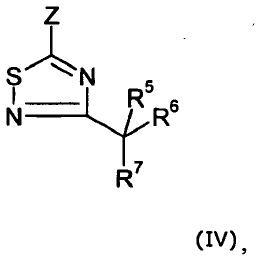 in der Z eine Austrittsgruppe, ausgewählt aus der Gruppe bestehend aus Triflat, Mesylat oder Tosylat ist und R⁵, R⁶, R⁷ die in Anspruch 1 angegebenen Bedeutungen haben.

8. Thiadiazolylaminophenylether der Formel (VIII) 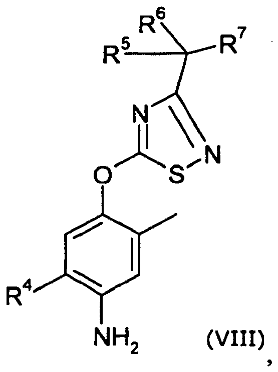 in der R⁴ bis R⁷ die in Anspruch 1 angegebenen Bedeutungen haben.

9. Thiadiazolylaminophenylether der Formel (VI) 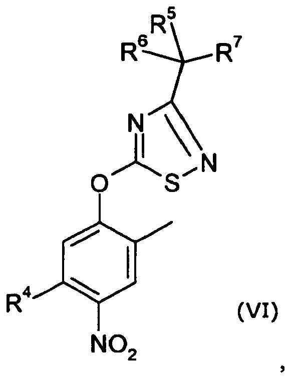 in der R⁴ bis R⁷ die in Anspruch 1 angegebenen Bedeutungen haben.

10. Mittel zum Bekämpfen unerwünschter Mikroorganismen im Pflanzenschutz und im Materialschutz, umfassend wenigstens ein Thiadiazolyloxyphenylamidin gemäß einem der Ansprüche 1 bis 5.

11. Verwendung eines Thiadiazolyloxyphenylamidins gemäß irgendeinem der Ansprüche 1 bis 5 zum Bekämpfen unerwünschter Mikroorganismen im Pflanzenschutz und im Materialschutz.

12. Verfahren zum Bekämpfen unerwünschter Mikroorganismen im Pflanzenschutz und im Materialschutz, **dadurch gekennzeichnet, dass** Thiadiazolyloxyphenylamidine gemäß einem der Ansprüche 1 bis 5 auf die Mikroorganismen und/oder in deren Lebensraum ausgebracht werden.

13. Saatgut, welches mit wenigstens einem Thiadiazolyloxyphenylamidin gemäß einem der Ansprüche 1 bis 5 behandelt ist.

## Claims

1. Thiadiazolyloxyphenylamidines of the formula (I) 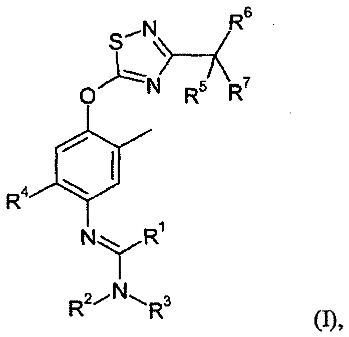 in which
R¹ is chosen from hydrogen; linear or branched C₁₋₁₂-alkyl, C₂₋₁₂-alkenyl or C₂₋₁₂-alkynyl or cyclic C₃₋₈-alkyl, C₄₋₈-alkenyl or C₄₋₈-alkynyl groups, it being possible, in the ring system of all abovementioned cyclic groups, for one or more carbon atoms to be replaced by heteroatoms chosen from nitrogen, oxygen, phosphorus and sulphur and it being possible for all abovementioned groups to be substituted with one or more groups chosen from -R', -X, -OR', -SR', -NR'₂, -SiR'3, -COOR', -CN and -CONR'₂, R' being hydrogen or a C₁₋₁₂-alkyl group; -SH; -SR", R" being a C₁₋₁₂-alkyl group which can be substituted with one or more groups chosen from -R', -X, -OR', -SR', -NR'₂, -SiR'₃, -COOR', -CN and -CONR'₂, R' having the above meanings;
R² is chosen from linear or branched C₁₋₁₂-alkyl, C₂₋₁₂-alkenyl or C₂₋₁₂-alkynyl, cyclic C₃₋₈-alkyl, C₄₋₈-alkenyl or C₄₋₈-alkynyl or C₅₋₁₈-aryl, C₇₋₁₉-aralkyl or C₇₋₁₉-alkaryl groups, it being possible, in the ring system of all abovementioned cyclic groups, for one or more carbon atoms to be replaced by heteroatoms chosen from nitrogen, oxygen, phosphorus and sulphur and it being possible for all abovementioned groups to be substituted with one or more groups chosen from -R', -X, -OR', -SR', -NR'₂, -SiR'₃, -COOR', -CN and -CONR'₂, R' having the above meanings;
R³ is chosen from -CN, -SH, -SR", -OR", -(C=O)-R", R" having the above meanings; linear or branched C₁₋₁₂-alkyl, C₂₋₁₂-alkenyl or C₂₋₁₂-alkynyl, cyclic C₃₋₈-alkyl, C₄₋₈-alkenyl or C₄₋₈-alkynyl or C₅₋₁₈-aryl, C₇₋₁₉-aralkyl or C₇₋₁₉-alkaryl groups, it being possible, in the ring system of all abovementioned cyclic groups, for one or more carbon atoms to be replaced by heteroatoms chosen from nitrogen, oxygen, phosphorus and sulphur and it being possible for all abovementioned groups to be substituted with one or more groups chosen from -R', -X, -OR', -SR', -NR'₂, -SiR'₃, -COOR', -CN and -CONR'₂, R' having the above meanings;
or in which
R² and R³,
R² and R¹ or
R¹ and R³ can form, together with the atoms to which they are bonded or with additional atoms chosen from nitrogen, oxygen, phosphorus and sulphur, a four- to seven-membered ring which can be substituted with R', OR', SR', NR'₂ or SiR'₃ groups, R' having the above meanings;
R⁴ is chosen from the group consisting of halogen (-X) and -CN;
R⁵, R⁶ and R⁷ are chosen, independently of one another, from hydrogen, linear or branched C₁₁₂-alkyl, C₂₋₁₂-alkenyl or C₂₋₁₂-alkynyl, cyclic C₃₋₈-alkyl, C₄₋₈-alkenyl or C₄₋₈-alkynyl or C₅₋₁₈-aryl, C₇₋₁₉-aralkyl or C₇₋₁₉-alkaryl groups, it being possible, in the ring system of all abovementioned cyclic groups, for one or more carbon atoms to be replaced by heteroatoms chosen from nitrogen, oxygen, phosphorus and sulphur and it being possible for all abovementioned groups to be substituted with one or more groups chosen from -R', halogen (-X), alkoxy (-OR'), thioether or mercapto (-SR'), amino (-NR'₂), silyl (-SiR'₃), carboxyl (-COOR'), cyano (-CN) and amide (-CONR'₂) groups, R' having the above meanings;
or
R⁵ and R⁶, R⁶ and R⁷ or R⁵ and R⁷ can form, together with the carbon atom to which they are bonded or with additional atoms chosen from nitrogen, oxygen, phosphorus and sulphur, a three- to seven-membered ring which can be substituted with X, R', OR', SR', NR'₂, SiR'₃, C₁₅₋₁₈-aryl, C₇₋₁₉-aralkyl or C₇₋₁₉-alkaryl groups, R' having the above meanings and it being possible for the aryl, aralkyl or alkaryl groups to be substituted in the ring with X, R', OR' or SR' groups; or
R⁵ and R⁶, R⁶ and R⁷ or R⁵ and R⁷ can form, together with the carbon atom to which they are bonded, a double bond which can be substituted with -CN, -SH, -SR", -OR", or -(C=O)-R", R" having the above meanings; or linear or branched C₁₋₁₂-alkyl, C₂₋₁₂-alkenyl or C₂₋₁₂-alkynyl, cyclic C₃₋₈-alkyl, C₄₋₈-alkenyl or C₄₋₈-alkynyl or C₅₋₁₈-aryl, C₇₋₁₉-aralkyl or C₇₋₁₉-alkaryl groups, it being possible, in the ring system of all abovementioned cyclic groups, for one or more carbon atoms to be replaced by heteroatoms chosen from nitrogen, oxygen, phosphorus and sulphur and it being possible for all abovementioned groups to be substituted with one or more groups chosen from -R', -X, -OR', -SR', -NR'₂, -SiR'₃, -COOR', -CN and -CONR'₂, R' having the above meanings;
and the salts thereof, with the exception of the compounds in which
R¹ is H, R² and R³ are methyl, R⁴ is Cl and R⁵, R⁶, R⁷ are methyl or R¹ is H, R² is methyl, R³ is ethyl, R⁴ is Cl and R⁵, R⁶, R⁷ are methyl or R¹ is H, R² and R³ are (CH₂)₄, R⁴ is Cl and R⁵, R⁶, R⁷ are methyl or R¹ is H, R² and R³ are (CH₂)₅, R⁴ is Cl and R⁵, R⁶, R⁷ are methyl.

2. Thiadiazolyloxyphenylamidines of the formula (I) according to Claim 1, in which
R¹ is chosen from the group consisting of hydrogen, a mercapto group (-SH) or C₁₋₈-alkyl groups;
R² is chosen from linear or branched C₁₋₈-alkyl groups;
R³ is chosen from linear, branched and alicyclic C₁₋₈-alkyl groups; or in which
R² and R³ can form, together with the nitrogen atom to which they are bonded or with additional atoms chosen from nitrogen and oxygen, a five- to six-membered ring which can be substituted with one or more C₁₋₁₂-alkyl groups;
R⁴ is chosen from the group consisting of F, Cl, Br and I;
R⁵ and R⁶ are chosen, independently of one another, from hydrogen and linear C₁₋₈-alkyl groups;
R⁷ is chosen from the group consisting of hydrogen, linear, branched, alicyclic or heterocyclic C₁₋₁₂-alkyl groups, halogen atoms, C₁₋₄-haloalkyl groups, phenyl groups and benzyl groups, it being possible for the phenyl or benzyl groups to be substituted with one or more groups chosen from -R', -X, -OR', -SR', -NR'₂, -SiR'₃, -COOR', -CN and -CONR'₂;
or
R⁵ and R⁶ can form, together with the carbon atom to which they are bonded or with additional atoms chosen from nitrogen, oxygen, phosphorus and sulphur, a three-, four- or five-membered ring which can be substituted with X, R' or phenyl groups, R' having the above meanings, and
R⁷ is chosen from hydrogen, halogen atoms, phenyl groups, phenoxy groups and benzyl groups, it being possible for the abovementioned groups to be substituted by one, two or more halogen atoms;
or
R⁵ and R⁶ can form, together with the carbon atom to which they are bonded, a double bond which can be substituted with hydrogen, linear or branched C₁₋₁₂-alkyl groups or cyclic C₃₋₈-alkyl groups, halogen atoms, C₁₋₄-haloalkyl groups, phenyl groups and benzyl groups, it being possible for the abovementioned phenyl or benzyl groups to be substituted with one or more groups chosen from -R', -X, -OR', -SR', -NR'₂, -SiR'₃, -COOR', -CN and -CONR'₂;
R⁷ is hydrogen or C₁₋₄-alkyl;
and the salts thereof.

3. Thiadiazolyloxyphenylamidines of the formula (I) according to either of Claims 1 or 2, in which
R¹ is chosen from the group consisting of hydrogen, mercapto and methyl;
R² is chosen from the group consisting of methyl and ethyl;
R³ is chosen from the group consisting of methyl, ethyl and isopropyl;
or in which
R² and R³ form, together with the nitrogen atom to which they are bonded, a piperidyl, pyrrolidyl or 2,6-dimethylmorpholinyl radical;
R⁴ is chosen from the group consisting of Cl and Br;
R⁵ and R⁶ are chosen, independently of one another, from hydrogen, methyl groups and ethyl groups or can form, together with the carbon atom to which they are bonded, a cyclopropyl ring which can be substituted with one or two chlorine or bromine atoms or with a phenyl or halophenyl group, and
R⁷ is chosen from the group consisting of methyl, methoxy, ethoxy, trimethylsilyl, triethylsilyl, phenyl, benzyl, 4-chlorobenzyl, 4-chlorophenyl, 4-bromophenyl, 2,4-dichlorophenyl, 2-chlorophenoxy, 3-chlorophenoxy, 4-chlorophenoxy and 3-trifluoromethyl groups;
or
R⁵ and R⁶ can form, together with the carbon atom to which they are bonded, a double bond which is substituted with a 4-fluorophenyl group, and
R⁷ is hydrogen or C₁₋₄-alkyl;
and the salts thereof.

4. Thiadiazolyloxyphenylamidines of the formula (I) according to one of Claims 1 to 3, in which R⁷ is a phenyl or benzyl group which in each case is substituted in the 4-position with chlorine or bromine.

5. Thiadiazolyloxyphenylamidine according to one of Claims 1 to 4, chosen from the group consisting of N'-{4-[(3-(tert-butyl)-1,2,4-thiadiazol-5-yl)oxy]-2-chloro-5-methylphenyl}-N-ethyl-N-methylimidoformamide (Example 1); N'-{2-bromo-4-[(3-(tert-butyl)-1,2,4-thiadiazol-5-yl)oxy]-5-methylphenyl}-N-ethyl-N-methylimidoformamide (Example 2); N'-(2-chloro-4-{[3-(1-chlorocyclopropyl)-1,2,4-thiadiazol-5-yl]oxy}-5-methylphenyl)-N-ethyl-N-methylimidoformamide (Example 3); N'-(2-chloro-4-{[3-(1-chlorocyclopropyl)-1,2,4-thiadiazol-5-yl]oxy}-5-methylphenyl)-N-methyl-N-(propan-2-yl)imidoformamide (Example 4); 2-chloro-4-{[3-(1-chlorocyclopropyl)-1,2,4-thiadiazol-5-yl]oxy}-5-methyl-N-[(E)-piperidin-1-ylmethylidene]aniline (Example 5); N'-[2-chloro-4-({3-[1-(4-chlorophenyl)ethyl]-1,2,4-thiadiazol-5-yl}oxy)-5-methylphenyl]-N-ethyl-N-methylimidoformamide (Example 6); 2-chloro-4-({3-[1-(4-chlorophenyl)ethyl]-1,2,4-thiadiazol-5-yl}oxy)-5-methyl-N-[(E)-piperidin-1-ylmethylidene]aniline (Example 7); N'-[2-chloro-4-({3-[1-(4-chlorophenyl)ethyl]-1,2,4-thiadiazol-5-yl}oxy)-5-methylphenyl]-N-methyl-N-(propan-2-yl)imidoformamide (Example 8); N'-[2-chloro-4-({3-[2-(4-chlorophenyl)ethyl]-1,2,4-thiadiazol-5-yl}oxy)-5-methylphenyl]-N-ethyl-N-methylimidoformamide (Example 9); 2-chloro-4-({3-[2-(4-chlorophenyl)ethyl]-1,2,4-thiadiazol-5-yl}oxy)-5-methyl-N-[(Z)-piperidin-1-ylmethylidene]aniline (Example 10); N'-[2-chloro-4-({3-[2-(4-chlorophenyl)propan-2-yl]-1,2,4-thiadiazol-5-yl}oxy)-5-methylphenyl]-N-ethyl-N-methylimidoformamide (Example 11); 2-chloro-4-({3-[2-(4-chlorophenyl)propan-2-yl]-1,2,4-thiadiazol-5-yl}oxy)-5-methyl-N-[(Z)-piperidin-1-ylmethylidene]aniline (Example 12); N'-[2-chloro-4-({3-[2-(4-chlorophenyl)propan-2-yl]-1,2,4-thiadiazol-5-yl}oxy)-5-methylphenyl]-N-methyl-N-(propan-2-yl)imidoformamide (Example 13); N'-{4-[(3-benzyl-1,2,4-thiadiazol-5-yl)oxy]-2-chloro-5-methylphenyl}-N-ethyl-N-methyl-imidoformamide (Example 14); N'-[2-chloro-4-({3-[(E)-2-(4-fluorophenyl)ethenyl]-1,2,4-thiadiazol-5-yl}oxy)-5-methylphenyl]-N-ethyl-N-methylimidoformamide (Example 15); 2-chloro-4-({3-[(E)-2-(4-fluorophenyl)ethenyl]-1,2,4-thiadiazol-5-yl}oxy)-5-methyl-N-[(Z)-piperidin-l-ylmethylidene]aniline (Example 16); N'-[2-chloro-4-({3-[1-(4-chlorophenyl)cyclopropyl]-1,2,4-thiadiazol-5-yl}oxy)-5-methylphenyl]-N-ethyl-N-methylimidoformamide (Example 17); N'-(2-chloro-4-{[3-(4-chlorobenzyl)-1,2,4-thiadiazol-5-yl]oxy}-5-methylphenyl)-N-ethyl-N-methylimidoformamide (Example 18); N'-(2-chloro-4-{[3-(chloromethyl)-1,2,4-thiadiazol-5-yl]oxy}-5-methylphenyl)-N-ethyl-N-methylimidoformamide (Example 19); N'-[2-chloro-4-({3-[1-(4-chlorophenyl)cyclopropyl]-1,2,4-thiadiazol-5-yl}oxy)-5-methylphenyl]-N-methyl-N-(propan-2-yl)imidoformamide (Example 20); 2-chloro-4-({3-[1-(4-chlorophenyl)cyclopropyl]-1,2,4-thiadiazol-5-yl}oxy)-5-methyl-N-[(Z)-piperidin-1-ylmethylidene]aniline (Example 21); N'-[2-chloro-4-({3-[(4-chlorophenoxy)methyl]-1,2,4-thiadiazol-5-yl}oxy)-5-methylphenyl]-N-ethyl-N-methylimidoformamide (Example 22); N'-[2-chloro-4-({3-[(E)-2-(4-fluorophenyl)ethenyl]-1,2,4-thiadiazol-5-yl}oxy)-5-methylphenyl]-N-methyl-N-(propan-2-yl)imidoformamide (Example 23); N'-{4-[(3-(tert-butyl)-1,2,4-thiadiazol-5-yl)oxy]-2-chloro-5-methylphenyl}-N-methyl-N-propylimidoformamide (Example 24); N'-{4-[(3-(tert-butyl)-1,2,4-thiadiazol-5-yl)oxy]-2-chloro-5-methylphenyl}-N-methyl-N-(prop-2-en-l-yl)imidoformamide (Example 25); N'-{4-[(3-(tert-butyl)-1,2,4-thiadiazol-5-yl)oxy]-2-chloro-5-methylphenyl}-N-(cyclopropylmethyl)-N-methylimidoformamide (Example 26); N'-{4-[(3-(tert-butyl)-1,2,4-thiadiazol-5-yl)oxy]-2-chloro-5-methylphenyl}-N-(3-methoxypropyl)-N-methylimidoformamide (Example 27); N'-(2-chloro-5-methyl-4-{[3-(2-methyl-1-phenylpropan-2-yl)-1,2,4-thiadiazol-5-yl]oxy}phenyl)-N-ethyl-N-methylimido-formamide (Example 28); rel-N'-[2-chloro-5-methyl-4-({3-[(1R,2R)-2phenylcyclopropyl]-1,2,4-thiadiazol-5-yl}oxy)phenyl]-N-ethyl-N-methylimido-formamide (Example 29); rel-2-chloro-5-methyl-4-({3-[(1R,2R)-2-phenylcyclopropyl]-1,2,4-thiadiazol-5-yl}oxy)-N-[(E)-piperidin-1-yl-methylidene]aniline (Example 30); rel-N'-[2-chloro-5-methyl-4-({3-[(1R,2R)-2-phenylcyclopropyl]-1,2,4-thiadiazol-5-yl}oxy)phenyl]-N-methyl-N-(propan-2-yl)imidoformamide (Example 31); N'-(2-chloro-4-{[3-(2,2-dichloro-1-methylcyclopropyl)-1,2,4-thiadiazol-5-yl]oxy}-5-methylphenyl)-N-ethyl-N-methylimidoformamide (Example 32); N'-(4-{[3-(4-bromobenzyl)-1,2,4-thiadiazol-5-yl]oxy}-2-chloro-5-methylphenyl)-N-ethyl-N-methylimidoformamide (Example 33); N'-[2-chloro-5-methyl-4-({3-[3-(trifluoromethyl)benzyl]-1,2,4-thiadiazol-5-yl}oxy)phenyl]-N-ethyl-N-methylimidoformamide (Example 34); N'-[2-chloro-4-({3-[(3-chlorophenoxy)methyl]-1,2,4-thiadiazol-5-yl}oxy)-5-methylphenyl]-N-ethyl-N-methylimidoformamide (Example 35); N'-[2-chloro-4-({3-[(2-chlorophenoxy)methyl]-1,2,4-thiadiazol-5-yl}oxy)-5-methylphenyl]-N-ethyl-N-methylimidoformamide (Example 36); N'-(2-chloro-5-methyl-4-{[3-(phenoxymethyl)-1,2,4-thiadiazol-5-yl]oxy}phenyl)-N-ethyl-N-methylimidoformamide (Example 37); N'-(2-chloro-4-{[3-(2,4-dichlorobenzyl)-1,2,4-thiadiazol-5-yl]oxy}-5-methylphenyl)-N-ethyl-N-methylimidoformamide (Example 38); N'-(2-chloro-4-{[3-(1-chloro-2-methylpropan-2-yl)-1,2,4-thiadiazol-5-yl]oxy}-5-methylphenyl)-N-ethyl-N-methylimidoformamide (Example 39); N'-(2-chloro-4-{[3-(2,4-difluorobenzyl)-1,2,4-thiadiazol-5-yl]oxy}-5-methylphenyl)-N-ethyl-N-methylimidoformamide (Example 40).

6. Process for the preparation of the thiadiazolyloxyphenylamidines according to one of Claims 1 to 5 comprising at least one of the following stages (a) to (j):
(a) reaction of nitrobenzene derivatives of the formula (III) with a thiadiazolyl alcohol of the formula (II) according to the following reaction scheme: 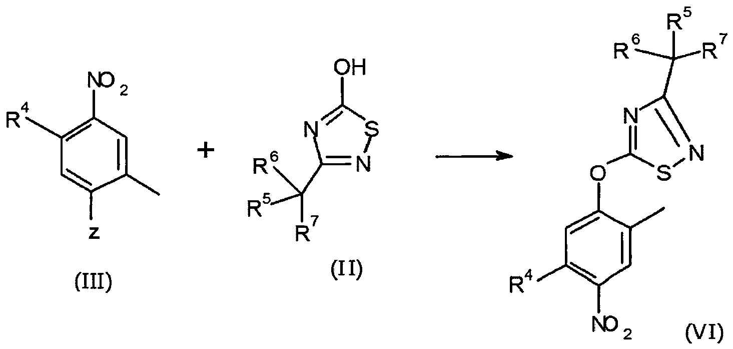
(b) reaction of nitrophenol derivatives of the formula (V) with thiadiazolyl derivatives of the formula (IV) according to the following reaction scheme: 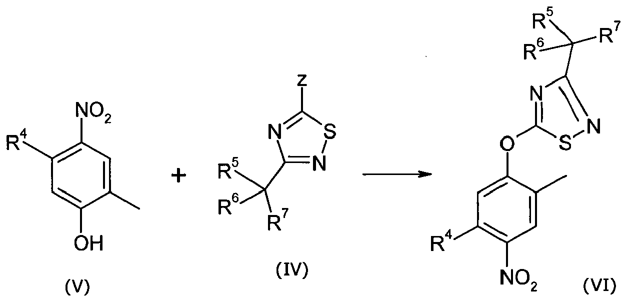
(c) reaction of anilines of the formula (VII) with a thiadiazolyl alcohol of the formula (II) according to the following reaction scheme: 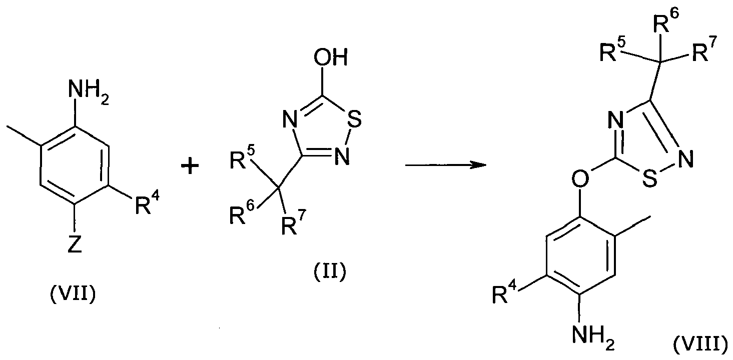
(d) reaction of aminophenols of the formula (XII) with thiadiazolyl derivatives of the formula (IV) according to the following reaction scheme: 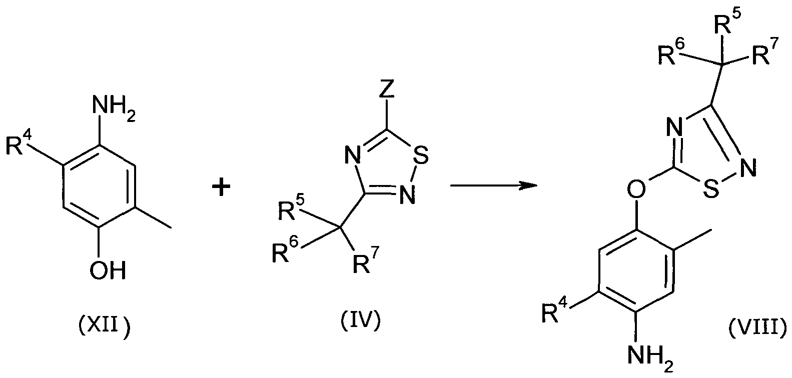
(e) reduction of the nitrophenoxy ethers of the formula (VI) to give aniline ethers of the formula (VIII) according to the following reaction scheme: 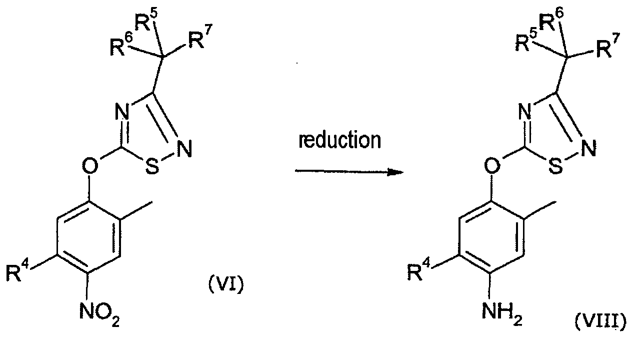
(f) reaction of the aniline ethers of the formula (VIII) with
(i) aminoacetals of the formula (XIII) or
(ii) amides of the formula (XIV) or
(iii) amines of the formula (XV) in the presence of orthoesters of the formula (XVI)
according to the following reaction scheme: 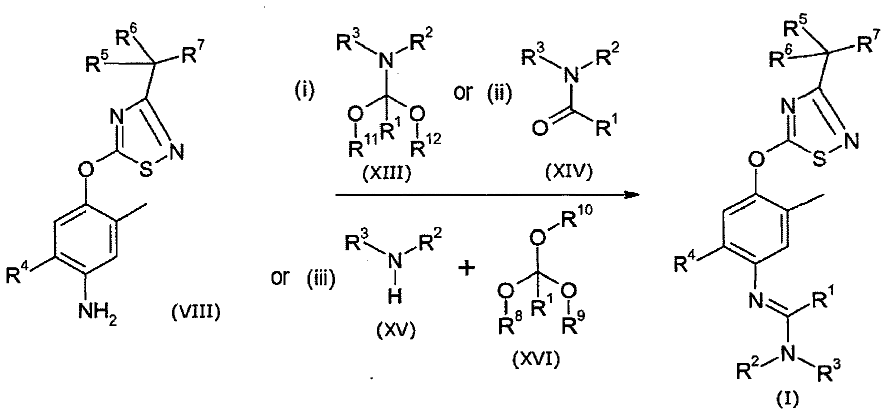
(g) reaction of the aminophenols of the formula (XII) with
(i) aminoacetals of the formula (XIII) or
(ii) amides of the formula (XIV) or
(iii) amines of the formula (XV) in the presence of orthoesters of the formula (XVI)
according to the following reaction scheme: 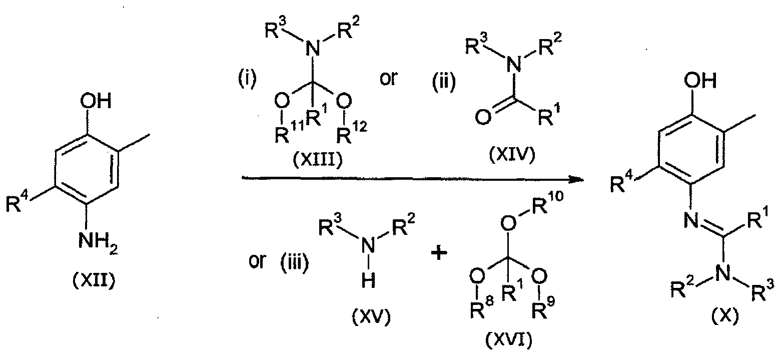
(h) reaction of the aminophenols of the formula (VII) with
(i) aminoacetals of the formula (XIII) or
(ii) amides of the formula (XIV) or
(iii) amines of the formula (XV) in the presence of orthoesters of the formula (XVI)
according to the following reaction scheme: 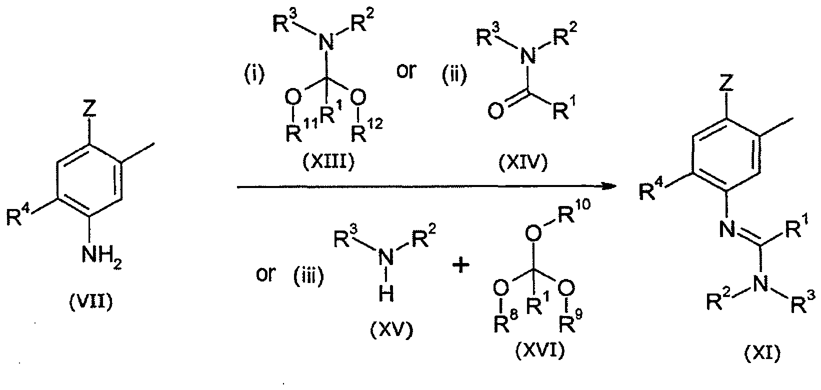
(i) reaction of amidines of the formula (XI) with a thiadiazolyl alcohol of the formula (II) according to the following reaction scheme: 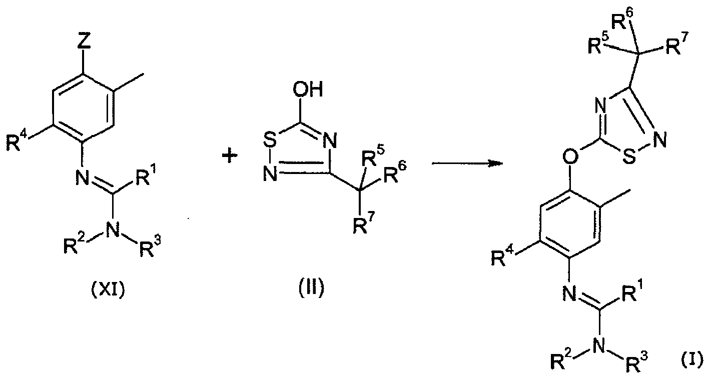
(j) reaction of amidines of the formula (XI) with thiadiazolyl derivatives of the formula (IV) according to the following reaction scheme: 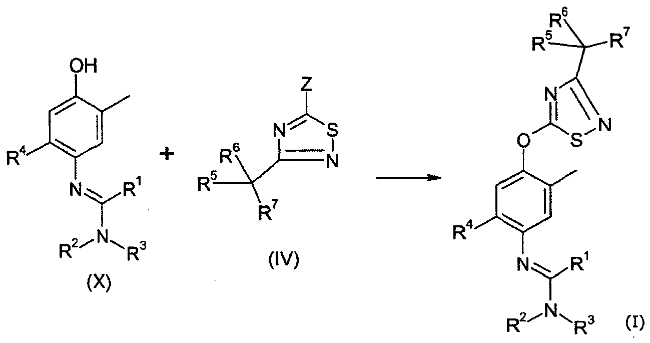 in which, in the above schemes,
Z is a leaving group;
R¹ to R⁷ have the above meanings;
and
R⁸ to R¹⁰ are chosen, independently of one another, from the group consisting of hydrogen, C₁₋₁₂-alkyl, C₂₋₁₂-alkenyl, C₂₋₁₂-alkynyl, C₅₋₁₈-aryl, C₇₋₁₉-arylalkyl and C₇₋₁₉-alkylaryl groups and in each case R⁸ with R⁹, R⁸ with R¹⁰ or R⁹ with R¹⁰ can form, together with the atoms to which they are bonded and if appropriate with additional carbon, nitrogen, oxygen or sulphur atoms, a five-, six- or seven-membered ring;
R¹¹ and R¹² are chosen, independently of one another, from the group consisting of hydrogen, C₁₋₁₂-alkyl, C₂₋₁₂-alkenyl, C₂₋₁₂-alkynyl, C₅₋₁₈-aryl and C₇₋₁₉-arylalkyl groups and can form, together with the atoms to which they are bonded, a five-, six- or seven-membered ring.

7. Thiadiazolyl derivatives of the formula (IV): 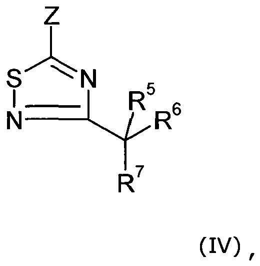 in which Z is a leaving group chosen from the group consisting of triflate, mesylate and tosylate and R⁵, R⁶, R⁷ have the meanings given in Claim 1.

8. Thiadiazolyl aminophenyl ethers of the formula (VIII): 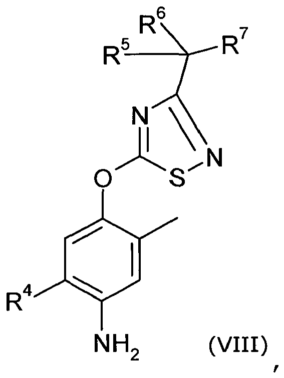 in which R⁴ to R⁷ have the meanings given in Claim 1.

9. Thiadiazolyl nitrophenyl ethers of the formula (VI): 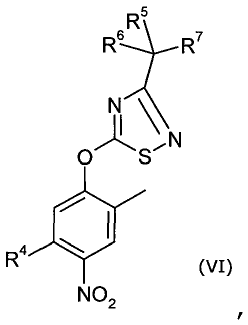 in which R⁴ to R⁷ have the meanings given in Claim 1.

10. Composition for combating undesirable microorganisms in plant protection and in material protection, comprising at least one thiadiazolyloxyphenylamidine according to one of Claims 1 to 5.

11. Use of a thiadiazolyloxyphenylamidine according to any of Claims 1 to 5 in combating undesirable microorganisms in plant protection and in material protection.

12. Method for combating undesirable microorganisms in plant protection and in material protection, **characterized in that** thiadiazolyloxyphenylamidines according to one of Claims 1 to 5 are applied to the microorganisms and/or to the habitat thereof.

13. Seed, which is treated with at least one thiadiazolyloxyphenylamidine according to one of Claims 1 to 5.

## Revendications

1. Thiadiazolyloxyphénylamidines de formule (I) 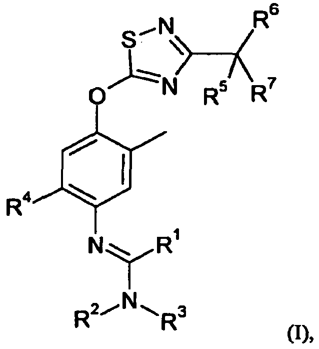 dans laquelle
R¹ est choisi parmi un atome d'hydrogène ; des groupes alkyle en C₁-C₁₂, alcényle en C₂-C₁₂, alcynyle en C₂-C₁₂ linéaires ou ramifiés ou des groupes alkyle en C₃-C₈, alcényle en C₄-C₈, alcynyle en C₄-C₈ cycliques, dans le système cyclique de tous les groupes cycliques nommés précédemment un ou plusieurs atomes de carbone pouvant être remplacés par des hétéroatomes, choisis parmi N, O, P et S, et tous les groupes mentionnés précédemment pouvant être substitués par un ou plusieurs groupes qui sont choisis parmi -R', -X, -OR', -SR', -NR'₂, -SiR'₃, -COOR', -CN et -CONR₂', R' étant un atome d'hydrogène ou un groupe alkyle en C₁-C₁₂ ; -SH ; -SR'', R'' étant un groupe alkyle en C₁-C₁₂ qui peut être substitué par un ou plusieurs groupes qui sont choisis parmi -R', -X, -OR', -SR', -NR'₂, -SiR'₃, -COOR', -CN et -CONR'₂, R' ayant la signification ci-dessus ;
R² est choisi parmi des groupes alkyle en C₁-C₁₂, alcényle en C₂-C₁₂, alcynyle en C₂-C₁₂ linéaires ou ramifiés, des groupes alkyle en C₃-C₈, alcényle en C₄-C₈, alcynyle en C₄-C₈ cycliques ou aryle en C₅-C₁₈, aralkyle en C₇-C₁₉ ou alkaryle en C₇-C₁₉, dans le système cyclique de tous les groupes cycliques nommés précédemment un ou plusieurs atomes de carbone pouvant être remplacés par des hétéroatomes, choisis parmi N, O, P et S, et tous les groupes mentionnés précédemment pouvant être substitués par un ou plusieurs groupes qui sont choisis parmi -R', -X, -OR', -SR', -NR'₂, -SiR'₃, -COOR', -CN et -CONR₂', R' ayant les significations ci-dessus ;
R³ est choisi parmi -CN, -SH, -SR", OR", -(C=O)-R", R" ayant les significations ci-dessus ; des groupes alkyle en C₁-C₁₂, alcényle en C₂-C₁₂, alcynyle en C₂-C₁₂ linéaires ou ramifiés, des groupes alkyle en C₃-C₈, alcényle en C₄-C₈, alcynyle en C₄-C₈ cycliques ou aryle en C₅-C₁₈, aralkyle en C₇-C₁₉ ou alkaryle en C₇-C₁₉, dans le système cyclique de tous les groupes cycliques nommés précédemment un ou plusieurs atomes de carbone pouvant être remplacés par des hétéroatomes, choisis parmi N, O, P et S, et tous les groupes mentionnés précédemment pouvant être substitués par un ou plusieurs groupes qui sont choisis parmi -R', -X, -OR', -SR', -NR'₂, -SiR'₃, -COOR', -CN et -CONR₂', R' ayant les significations ci-dessus ; ou dans laquelle
R² et R³,
R² et R¹ ou
R¹ et R³ peuvent former ensemble avec les atomes auxquels ils sont liés ou avec d'autres atomes, choisis parmi N, O, P et S, un cycle à quatre à sept chaînons, qui peut être substitué par des groupes R', OR', SR', NR'₂, SiR'₃, R' ayant les significations ci-dessus ;
R⁴ est choisi dans le groupe constitué par un atome d'halogène (-X) et -CN ;
R⁵ R⁶ et R⁷, sont choisis chacun indépendamment parmi un atome d'hydrogène, des groupes alkyle en C₁-C₁₂, alcényle en C₂-C₁₂, alcynyle en C₂-C₁₂ linéaires ou ramifiés, des groupes alkyle en C₃-C₈, alcényle en C₄-C₈, alcynyle en C₄-C₈ cycliques ou aryle en C₅-C₁₈, aralkyle en C₇-C₁₉ ou alkaryle en C₇-C₁₉, dans le système cyclique de tous les groupes cycliques nommés précédemment un ou plusieurs atomes de carbone pouvant être remplacés par des hétéroatomes, choisis parmi N, O, P et S, et tous les groupes mentionnés précédemment pouvant être substitués par un ou plusieurs groupes qui sont choisis parmi -R', des groupes halogéno (-X), alcoxy (-OR'), thioéther ou mercapto (-SR'), amino (-NR'₂), silyle (-SiR'₃), carboxy (-COOR'), cyano (-CN) et amido (-CONR₂'), R' ayant les significations ci-dessus ;
ou
R⁵ et R⁶, R⁶ et R⁷ ou R⁵ et R⁷ peuvent former ensemble avec l'atome de carbone auxquels ils sont liés ou avec d'autres atomes, choisis parmi N, O, P et S, un cycle à trois à sept chaînons, qui peut être substitué par des groupes X, R', OR', SR', NR'₂, SiR'₃, aryle en C₅-C₁₈, aralkyle en C₇-C₁₉ ou alkaryle en C₇-C₁₉, R' ayant les significations ci-dessus et les groupes aryle, aralkyle ou alkaryle pouvant être substitués dans le cycle par des groupes X, R', OR', SR' ;
ou
R⁵ et R⁶, R⁶ et R⁷ ou R⁵ et R⁷ peuvent former ensemble avec l'atome de carbone auxquels ils sont liés une double liaison, qui peut être substituée par -CN, -SH, -SR", OR", -(C=O)-R", R" ayant les significations ci-dessus ; des groupes alkyle en C₁-C₁₂, alcényle en C₂-C₁₂, alcynyle en C₂-C₁₂ linéaires ou ramifiés, des groupes alkyle en C₃-C₈, alcényle en C₄-C₈, alcynyle en C₄-C₈ cycliques ou aryle en C₅-C₁₈, aralkyle en C₇-C₁₉ ou alkaryle en C₇-C₁₉, dans le système cyclique de tous les groupes cycliques nommés précédemment un ou plusieurs atomes de carbone pouvant être remplacés par des hétéroatomes, choisis parmi N, O, P et S, et tous les groupes mentionnés précédemment pouvant être substitués par un ou plusieurs groupes qui sont choisis parmi -R', -X, -OR', -SR', -NR'₂, -SiR'₃, -COOR', -CN et -CONR₂', R' ayant les significations ci-dessus ;
et leurs sels, à l'exclusion des composés dans lesquels
R¹ représente H, R² et R³ représentent le groupe méthyle, R⁴ représente Cl et R⁵, R⁶, R⁷ représentent le groupe méthyle ou R¹ représente H, R² représente le groupe méthyle, R³ représente le groupe éthyle, R⁴ représente Cl et R⁵, R⁶, R⁷ représentent le groupe méthyle ou R¹ représente H, R² et R³ représentent (CH₂)₄, R⁴ représente Cl et R⁵, R⁶, R⁷ représentent le groupe méthyle ou R¹ représente H, R² et R³ représentent (CH₂)₅, R⁴ représente Cl et R⁵, R⁶, R⁷ représentent le groupe méthyle.

2. Thiadiazolyloxyphénylamidines de formule (I), selon la revendication 1, dans lesquelles
R¹ est choisi dans le groupe constitué par un atome d'hydrogène, un groupe mercapto (-SH) ou des groupes alkyle en C₁-C₈;
R² est choisi parmi des groupes alkyle en C₁-C₈ linéaires ou ramifiés ;
R³ est choisi parmi des groupes alkyle en C₁-C₈ linéaires, ramifiés et alicycliques ;
ou dans lesquelles
R² et R³ peuvent former ensemble avec l'atome d'azote auquel ils sont liés ou avec d'autres atomes, qui sont choisis parmi N et O, un cycle à cinq ou six chaînons, qui peut être substitué par un ou plusieurs groupes alkyle en C₁-C₁₂ ;
R⁴ est choisi dans le groupe constitué par F, Cl, Br et I ;
R⁵ et R⁶ sont choisis, indépendamment l'un de l'autre, parmi un atome d'hydrogène, des groupes alkyle en C₁-C₈ linéaires ;
R⁷ est choisi dans le groupe constitué par un atome d'hydrogène, des groupes alkyle en C₁-C₁₂ linéaires, ramifiés, alicycliques ou hétérocycliques, des atomes d'halogène, des groupes halogénoalkyle(C₁-C₄), des groupes phényle et benzyle, les groupes phényle ou benzyle pouvant être substitués par un ou plusieurs atomes, choisis parmi -R', -X, -OR', -SR', -NR'₂, -SiR'₃, -COOR', -CN et -CONR₂' ;
ou
R⁵ et R⁶ forment ensemble avec l'atomes de carbone auquel ils sont liés ou avec d'autres atomes, choisis parmi N, O, P et S, un cycle à trois, quatre ou cinq chaînons, qui peut être substitué par des groupes X, R' ou phényle, R' ayant les significations ci-dessus et
R⁷ est choisi parmi un atome d'hydrogène et des atomes d'halogène, des groupes phényle, phénoxy et benzyle, les groupes nommés précédemment pouvant être substitués par un, deux ou plus de deux atomes d'halogène;
ou
R⁵ et R⁶ peuvent former ensemble avec l'atome de carbone auxquels ils sont liés une double liaison, qui peut être substituée par hydrogène, des groupes alkyle en C₁-C₁₂ linéaires ou ramifiés ou des groupes alkyle en C₃-C₈ cycliques, des atomes d'halogène, des groupes halogénoalkyle(C₁-C₄), des groupes phényle et benzyle, les groupes phényle ou benzyle précités pouvant être substitués par un ou plusieurs atomes, choisis parmi -R', -X, -OR', -SR', -NR'₂, -SiR'₃, -COOR', -CN et -CONR₂' ;
R⁷ est un atome d'hydrogène ou un groupe alkyle en C₁-C₄ ;
et leurs sels.

3. Thiadiazolyloxyphénylamidines de formule (I), selon la revendication 1 ou 2, dans lesquelles
R¹ est choisi dans le groupe constitué par un atome d'hydrogène, les groupes mercapto et méthyle ;
R² est choisi dans le groupe constitué par les groupes méthyle et éthyle ;
R³ est choisi dans le groupe constitué par les groupes méthyle, éthyle et isopropyle ;
ou dans lesquelles
R² et R³ forment ensemble avec l'atome d'azote, auquel ils sont liés, un radical pipéridyle, pyrrolidyle ou 2,6-diméthylmorpholinyle ;
R⁴ est choisi dans le groupe constitué par Cl et Br ;
R⁵ et R⁶ sont choisis, indépendamment l'un de l'autre, parmi un atome d'hydrogène, les groupes méthyle et éthyle ou forment ensemble avec l'atome de carbone, auquel ils sont liés, un cycle cyclopropyle, qui peut être substitué par un ou deux atomes de chlore ou de brome ou par un groupe phényle ou halogénophényle et
R⁷ est choisi dans le groupe constitué par les groupes méthyle, méthoxy, éthoxy, triméthylsilyle, triéthylsilyle, phényle, benzyle, 4-chlorobenzyle, 4-chlorophényle, 4-bromophényle, 2,4-dichlorophényle, 2-chlorophénoxy, 3-chlorophénoxy, 4-chloro-phénoxy et 3-trifluorométhyle
ou
R⁵ et R⁶ peuvent former ensemble avec l'atome de carbone auquel ils sont liés une double liaison, qui est substitués par un groupe 4-fluorophényle et
R⁷ est un atome d'hydrogène ou un groupe alkyle en C₁-C₄ ;
et leurs sels.

4. Thiadiazolyloxyphénylamidines de formule (I), selon l'une quelconque des revendications 1 à 3, dans lesquelles R⁷ est un groupe phényle ou un groupe benzyle, qui sont substitués chacun en position 4 par chloro ou bromo.

5. Thiadiazolyloxyphénylamidines selon l'une quelconque des revendications 1 à 4, choisies dans le groupe constitué par le N'-{4-[(3-tert-butyl-1,2,4-thiadiazol-5-yl)oxy]-2-chloro-5-méthylphényl}-N-éthyl-N-méthylimidoformamide (exemple 1) ; le N'-{2-bromo-4-[(3-tert-butyl-1,2,4-thiadiazol-5-yl)oxy]-5-méthyl-phényl}-N-éthyl-N-méthylimidoformamide (exemple 2) ; le N'-(2-chloro-4-{[3-(1-chlorocyclopropyl)-1,2,4-thiadiazol-5-yl]oxy}-5-méthylphényl)-N-éthyl-N-méthylimidoformamide (exemple 3) ; le N'-(2-chloro-4-{[3-(1-chlorocyclopropyl)-1,2,4-thiadiazol-5-yl]oxy}-5-méthylphényl)-N-méthyl-N-propan-2-ylimidoformamide (exemple 4) ; la 2-chloro-4-{[3-(1-chlorocyclopropyl)-1,2,4-thiadiazol-5-yl]oxy}-5-méthyl-N-[(E)-pipéridin-1-ylméthylidène]aniline (exemple 5) ; le N'-[2-chloro-4-({3-[1-(4-chlorophényl)éthyl]-1,2,4-thiadiazol-5-yl}oxy)-5-méthylphényl]-N-éthyl-N-méthylimidoformamide (exemple 6) ; la 2-chloro-4-({3-[1-(4-chlorophényl)-éthyl]-1,2,4-thiadiazol-5-yl}oxy)-5-méthyl-N-[(E)-pipéridin-1-ylméthylidène]aniline (exemple 7) ; le N'-[2-chloro-4-({3-[1-(4-chlorophényl)éthyl]-1,2,4-thiadiazol-5-yl}oxy)-5-méthylphényl]-N-méthyl-N-propan-2-ylimidoformamide (exemple 8) ; le N'-[2-chloro-4-({3-[2-(4-chlorophényl)éthyl]-1,2,4-thiadiazol-5-yl}oxy)-5-méthylphényl]-N-éthyl-N-méthylimidoformamide (exemple 9) ; la 2-chloro-4-({3-[2-(4-chlorophényl)-éthyl]-1,2,4-thiadiazol-5-yl}oxy)-5-méthyl-N-[(Z)-pipéridin-1-ylméthylidène]aniline (exemple 10) ; le N'-[2-chloro-4-({3-[2-(4-chlorophényl)propan-2-yl]-1,2,4-thiadiazol-5-yl}oxy)-5-méthylphényl)-N-éthyl-N-méthylimidoformamide (exemple 11) ; la 2-chloro-4-({3-[2-(4-chlorophényl)propan-2-yl]-1,2,4-thiadiazol-5-yl}oxy)-5-méthyl-N-[(Z)-pipéridin-1-ylméthylidène]-aniline (exemple 12) ; le N'-[2-chloro-4-({3-[2-(4-chlorophényl)propan-2-yl]-1,2,4-thiadiazol-5-yl}oxy)-5-méthylphényl]-N-méthyl-N-propan-2-ylimidoformamide (exemple 13) ; le N'-{4-[(3-benzyl-1,2,4-thiadiazol-5-yl)oxy]-2-chloro-5-méthylphényl}-N-éthyl-N-méthylimido-formamide (exemple 14) ; le N'-[2-chloro-4-({3-[(E)-2-(4-fluorophényl)éthényl]-1,2,4-thiadiazol-5-yl}oxy)-5-méthylphényl]-N-éthyl-N-méthylimidoformamide (exemple 15) ; la 2-chloro-4-({3-[(E)-2-(4-fluoro-phényl)éthényl]-1,2,4-thiadiazol-5-yl}oxy)-5-méthyl-N-[(Z)-pipéridin-1-ylméthylidène]aniline (exemple 16) ; le N'-[2-chloro-4-({3-[1-(4-chlorophényl)cyclopropyl]-1,2,4-thiadiazol-5-yl}oxy)-5-méthylphényl]-N-éthyl-N-méthylimidoformamide (exemple 17) ; le N'-(2-chloro-4-{[3-(4-chlorobenzyl)-1,2,4-thiadiazol-5-yl]oxy}-5-méthylphényl)-N-éthyl-N-méthylimidoformamide (exemple 18) ; le N'-(2-chloro-4-{[3-(chlorométhyl)-1,2,4-thiadiazol-5-yl]oxy}-5-méthylphényl)-N-éthyl-N-méthylimidoformamide (exemple 19) ; le N'-[2-chloro-4-({3-[1-(4-chlorophényl)cyclopropyl]-1,2,4-thiadiazol-5-yl}oxy)-5-méthylphényl]-N-méthyl-N-propan-2-ylimido-formamide (exemple 20) ; la 2-chloro-4-({3-[1-(4-chlorophényl)cyclopropyl]-1,2,4-thiadiazol-5-yl}oxy)-5-méthyl-N-[(Z)-pipéridin-1-ylméthylidène]aniline (exemple 21) ; le N'-[2-chloro-4-({3-[(4-chloro-phénoxy)méthyl]-1,2,4-thiadiazol-5-yl}oxy)-5-méthyl-phényl]-N-éthyl-N-méthylimidoformamide (exemple 22) ; le N'-[2-chloro-4-({3-[(E)-2-(4-fluorophényl)éthényl]-1,2,4-thiadiazol-5-yl}oxy)-5-méthylphényl]-N-méthyl-N-propan-2-ylimidoformamide (exemple 23) ; le N'-{4-[(3-tert-butyl-1,2,4-thiadiazol-5-yl)oxy]-2-chloro-5-méthylphényl}-N-méthyl-N-propylimidoformamide (exemple 24) ; le N'-{4-[(3-tert-butyl-1,2,4-thiadiazol-5-yl)oxy]-2-chloro-5-méthylphényl}-N-méthyl-N-prop-2-én-1-ylimidoformamide (exemple 25) ; le N'-{4-[(3-tert-butyl-1,2,4-thiadiazol-5-yl)oxy]-2-chloro-5-méthylphényl}-N-(cyclopropylméthyl)-N-méthylimido-formamide (exemple 26) ; le N'-{4-[(3-tert-butyl-1,2,4-thiadiazol-5-yl)oxy]-2-chloro-5-méthylphényl}-N-(3-méthoxypropyl)-N-méthylimidoformamide (exemple 27) ; le N'-(2-chloro-5-méthyl-4-{[3-(2-méthyl-1-phénylpropan-2-yl)-1,2,4-thiadiazol-5-yl]oxy}phényl)-N-éthyl-N-méthyl-imidoformamide (exemple 28) ; le rel-N'-[2-chloro-5-méthyl-4-({3-[(1R,2R)-2-phénylcyclopropyl]-1,2,4-thiadiazol-5-yl}oxy)phényl]-N-éthyl-N-méthylimido-formamide (exemple 29) ; la rel-2-chloro-5-méthyl-4-({3-[(1R,2R)-2-phénylcyclopropyl]-1,2,4-thiadiazol-5-yl}oxy)-N-[(E)-pipéridin-1-ylméthylidène]aniline (exemple 30) ; le rel-N'-[2-chloro-5-méthyl-4-({3-[(1R,2R)-2-phénylcyclopropyl]-1,2,4-thiadiazol-5-yl}-oxy)phényl]-N-méthyl-N-propan-2-ylimidoformamide (exemple 31) ; le N'-(2-chloro-4-{[3-(2,2-dichloro-1-méthylcyclopropyl)-1,2,4-thiadiazol-5-yl]oxy}-5-méthyl-phényl)-N-éthyl-N-méthylimidoformamide (exemple 32) ; le N'-(4-{[3-(4-bromobenzyl)-1,2,4-thiadiazol-5-yl]-oxy}-2-chloro-5-méthylphényl)-N-éthyl-N-méthylimido-formamide (exemple 33) ; le N'-[2-chloro-5-méthyl-4-({3-[3-(trifluorométhyl)benzyl]-1,2,4-thiadiazol-5-yl}oxy)phényl]-N-éthyl-N-méthylimidoformamide (exemple 34) ; le N'-[2-chloro-4-({3-[(3-chloro-phénoxy)méthyl]-1,2,4-thiadiazol-5-yl}oxy)-5-méthyl-phényl]-N-éthyl-N-méthylimidoformamide (exemple 35) ; le N'-[2-chloro-4-({3-[(2-chlorophénoxy)méthyl]-1,2,4-thiadiazol-5-yl)oxy)-5-méthylphényl]-N-éthyl-N-méthyl-imidoformamide (exemple 36) ; le N'-(2-chloro-5-méthyl-4-{[3-(phénoxyméthyl)-1,2,4-thiadiazol-5-yl]oxy}-phényl)-N-éthyl-N-méthylimidoformamide (exemple 37) ; le N'-(2-chloro-4-{[3-(2,4-dichlorobenzyl)-1,2,4-thiadiazol-5-yl]oxy}-5-méthylphényl)-N-éthyl-N-méthylimidoformamide (exemple 38) ; le N'-(2-chloro-4-{[3-(1-chloro-2-méthylpropan-2-yl)-1,2,4-thiadiazol-5-yl]oxy}-5-méthylphényl)-N-éthyl-N-méthylimidoformamide (exemple 39) ; le N'-(2-chloro-4-{[3-(2,4-difluoro-benzyl)-1,2,4-thiadiazol-5-yl]oxy}-5-méthylphényl)-N-éthyl-N-méthylimidoformamide (exemple 40).

6. Procédé pour la préparation des thiadiazolyloxyphénylamidines selon l'une quelconque des revendications 1 à 5, comprenant au moins une des étapes (a) à (j) suivantes :
(a) mise en réaction de dérivés de nitrobenzène de formule (III) avec un thiadiazolyl-alcool de formule (II) selon le schéma de réaction suivant : 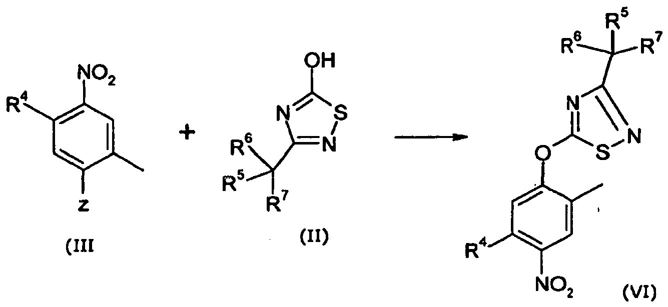
(b) mise en réaction de dérivés de nitrophénol de formule (V) avec des dérivés thiadiazolyle de formule (IV) selon le schéma de réaction suivant : 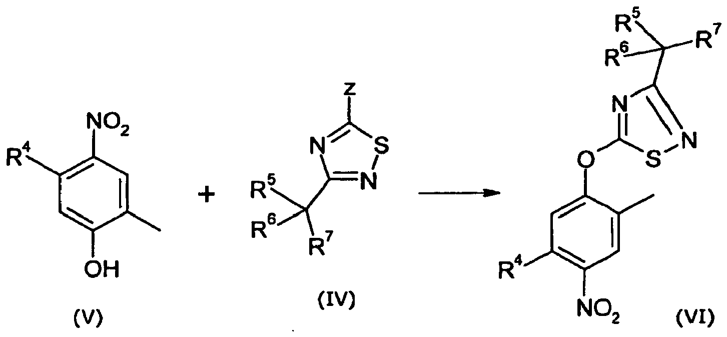
(c) mise en réaction d'anilines de formule (VII) avec un thiadiazolyl-alcool de formule (II) selon le schéma de réaction suivant : 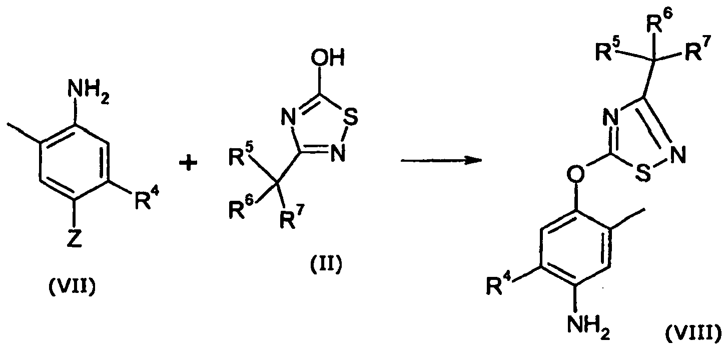
(d) mise en réaction d'aminophénols de formule (XII) avec des dérivés thiadiazolyle de formule (IV) selon le schéma de réaction suivant : 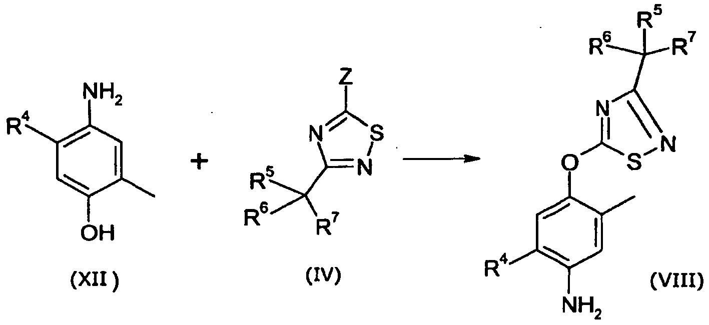
(e) réduction des nitrophénoxyéthers de formule (VI) en éthers d'aniline de formule (VIII) selon le schéma de réaction suivant : 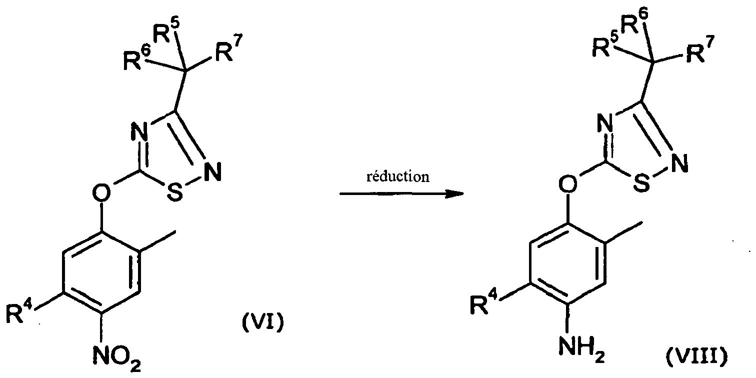
(f) mise en réaction des éthers d'aniline de formule (VIII) avec
(i) des aminoacétals de formule (XIII) ou
(ii) avec des amides de formule (XIV) ou
(iii) avec des amines de formule (XV) en présence d'orthoesters de formule (XVI)
selon le schéma de réaction suivant : 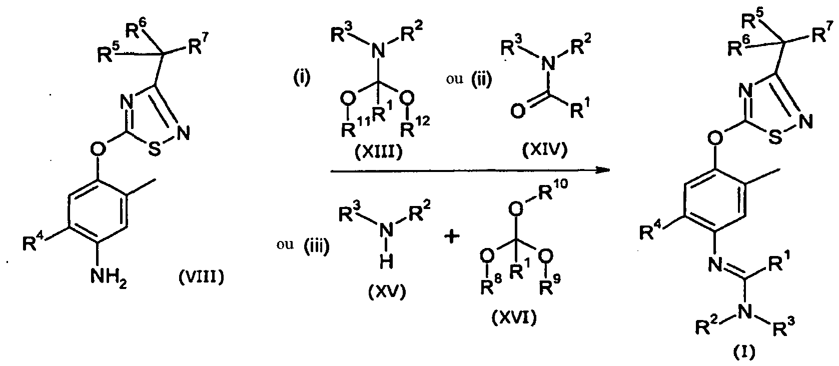
(g) mise en réaction des aminophénols de formule (XII) avec
(i) des aminoacétals de formule (XIII) ou
(ii) avec des amides de formule (XIV) ou
(iii) avec des amines de formule (XV) en présence d'orthoesters de formule (XVI)
selon le schéma de réaction suivant : 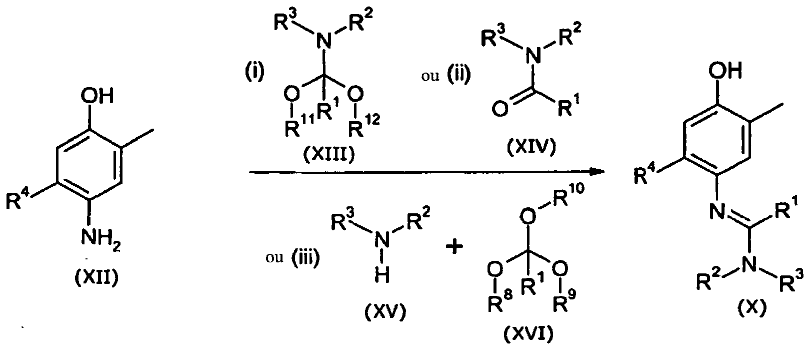
(h) mise en réaction des aminophénols de formule (VII) avec
(i) des aminoacétals de formule (XIII) ou
(ii) avec des amides de formule (XIV) ou
(iii) avec des amines de formule (XV) en présence d'orthoesters de formule (XVI)
selon le schéma de réaction suivant : 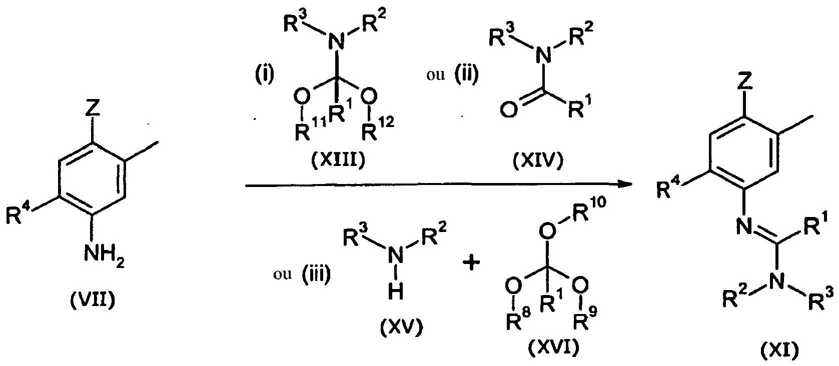
(i) mise en réaction d'amidines de formule (XI) avec un thiadiazolyl-alcool de formule (II) selon le schéma de réaction suivant : 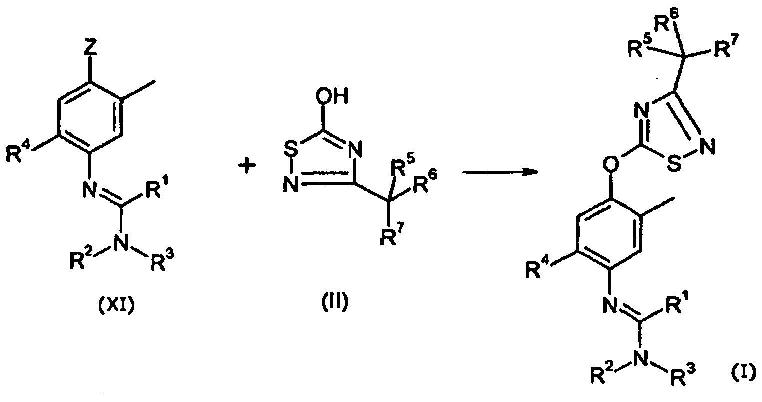
(j) mise en réaction d'amidines de formule (XI) avec des dérivés thiadiazolyle de formule (IV) selon le schéma de réaction suivant : 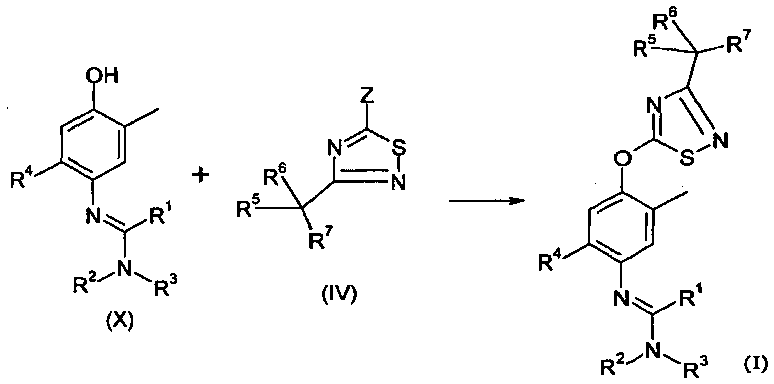 dans les schémas ci-dessus
Z étant un groupe partant ;
R¹ à R⁷ ayant les significations ci-dessus ;
et
R⁸ à R¹⁰ étant choisis chacun indépendamment dans le groupe constitué par un atome d'hydrogène, des groupes alkyle en C₁-C₁₂, alcényle en C₂-C₁₂, alcynyle en C₂-C₁₂ ou aryle en C₅-C₁₈ ou arylalkyle en C₇-C₁₉, alkylaryle en C₇-C₁₉ et respectivement R⁸ avec R⁹, R⁸ avec R¹⁰ ou R⁹ avec R¹⁰ pouvant former ensemble avec les atomes auxquels ils sont liés et éventuellement avec d'autres atomes de carbone, d'azote, d'oxygène ou de soufre un cycle à cinq, six ou sept chaînons ;
R¹¹ et R¹² étant choisis indépendamment l'un de l'autre dans le groupe constitué par un atome d'hydrogène, des groupes alkyle en C₁-C₁₂, alcényle en C₂-C₁₂, alcynyle en C₂-C₁₂ ou aryle en C₅-C₁₈ ou arylalkyle en C₇-C₁₉ et pouvant former ensemble avec les atomes auxquels ils sont liés un cycle à cinq, six ou sept chaînons.

7. Dérivés thiadiazolyle de formule (IV) 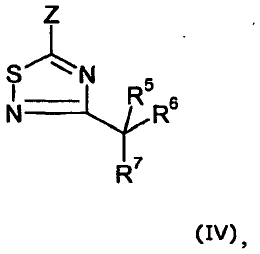 dans laquelle Z est un groupe partant, choisi dans le groupe constitué par triflate, mésylate ou tosylate et R⁵, R⁶, R⁷ ont les significations indiquées dans la revendication 1.

8. Thiadiazolylaminophényléthers de formule (VIII) 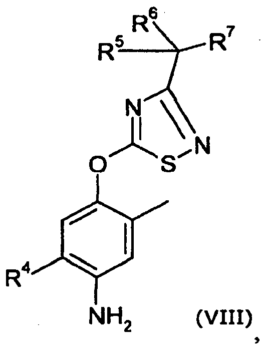 dans laquelle R⁴ à R⁷ ont les significations indiquées dans la revendication 1.

9. Thiadiazolylaminophényléthers de formule (VI) 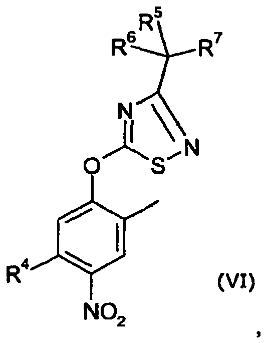 dans laquelle R⁴ à R⁷ ont les significations indiquées dans la revendication 1.

10. Agent destiné à la lutte contre des micro-organismes indésirables dans la protection des plantes et dans la protection des matériaux, comprenant au moins une thiadiazolyloxyphénylamidine selon l'une quelconque des revendications 1 à 5.

11. Utilisation d'une thiadiazolyloxyphénylamidine selon l'une quelconque des revendications 1 à 5, pour la lutte contre des micro-organismes indésirables dans la protection des plantes et dans la protection des matériaux.

12. Procédé pour la lutte contre des micro-organismes indésirables dans la protection des plantes et dans la protection des matériaux, **caractérisé en ce qu'**on applique des thiadiazolyloxyphénylamidines selon l'une quelconque des revendications 1 à 5 sur les micro-organismes et/ou dans leur habitat.

13. Semence, qui est traitée par au moins une thiadiazolyloxyphénylamidine selon l'une quelconque des revendications 1 à 5.
